# EUROPEAN PATENT SPECIFICATION

(11) **EP 3 924 055 B1**
(45) Date of publication and mention of the grant of the patent: **03.04.2024**
(21) Application number: 20708627.3
(22) Date of filing: 13.02.2020
(51) Int. Cl.: A61P 35/00, C07D 401/04, C07D 401/14, A61K 31/454

(54) **SUBSTITUTED 3-(1-OXOISOINDOLIN-2-YL)PIPERIDINE-2,6-DIONE DERIVATIVES AND USES THEREOF**
SUBSTITUIERTE 3-(1-OXOISOINDOLIN-2-YL)PIPERIDIN-2,6-DION-DERIVATE UND VERWENDUNGEN DAVON
DÉRIVÉS DE 3-(1-OXOISOINDOLINE-2-YL)PIPÉRIDINE-2,6-DIONE SUBSTITUÉS ET LEURS UTILISATIONS

(30) Priority: 15.02.2019 US 201962806142 P
(43) Date of publication of application: 22.12.2021
(73) Proprietor: Novartis AG, 4056 Basel (CH)
(72) Inventor: BECKWITH, Rohan Eric John, Cambridge, MA 02139 (US); BONAZZI, Simone, Cambridge, MA 02139 (US); CERNIJENKO, Artiom, Cambridge, MA 02139 (US); LAM, Philip, Cambridge, MA 02139 (US); THOMSEN, Noel Marie-France, Cambridge, MA 02139 (US)
(74) Representative: Reid, Stephanie Marie
(86) International application number: PCT/IB2020/051206
(87) International publication number: WO 2020/165834

(56) References cited:
- WO-A1-2018/102725
- WO-A1-2018/140809
- WO-A1-2019/038717
- WO-A1-2019/079569
- US-A1- 2018 099 940
- PETER H SCHAFER ET AL: "Cereblon modulator iberdomide induces degradation of the transcription factors Ikaros and Aiolos: immunomodulation in healthy volunteers and relevance to systemic lupus erythematosus", ANNALS OF THE RHEUMATIC DISEASES, vol. 77, no. 10, 26 June 2018 (2018-06-26) , pages 1516-1523, XP055686777, GB ISSN: 0003-4967, DOI: 10.1136/annrheumdis-2017-212916

## Description

### FIELD OF THE INVENTION

The present invention relates to substituted 3-(1-oxoisoindolin-2-yl)piperidine-2,6-dione compounds and compositions and their use for the treatment of IKAROS Family Zinc Finger 2 (IKZF2)-dependent diseases or disorders or where reduction of IKZF2 or IKZF4 protein levels can ameliorate a disease or disorder.

### BACKGROUND OF THE INVENTION

IKAROS Family Zinc Finger 2 (IKZF2) (also known as Helios) is one of the five members of the Ikaros family of transcription factors found in mammals. IKZF2 contains four zinc finger domains near the N-terminus, which are involved in DNA binding, and two zinc finger domains at the C-terminus, which are involved in protein dimerization. IKZF2 is about 50% identical with Ikaros family members, Ikaros (IKZF1), Aiolos (IKZF3), and Eos (IKZF4) with highest homology in the zinc finger regions (80%+ identity). These four Ikaros family transcription factors bind to the same DNA consensus site and can heterodimerize with each other when co-expressed in cells. The fifth Ikaros family protein, Pegasus (IKZF5), is only 25% identical to IKZF2, binds a different DNA site than other Ikaros family members and does not readily heterodimerize with the other Ikaros family proteins. IKZF2, IKZF1 and IKZF3 are expressed mainly in hematopoietic cells while IKZF4 and IKZF5 are expressed in a wide variety of tissues. (John, L.B., et al., (2011), Mol. Immunol. 48:1272-1278; Perdomo, J., et al., (2000), J. Biol. Chem. 275:38347-38354.)

IKZF2 is believed to have an important role in the function and stability of regulatory T cells (Tregs). IKZF2 is highly expressed at the mRNA and protein level by regulatory T-cell populations. Knockdown of IKZF2 by siRNA has been shown to result in downregulation of FoxP3 and to impair the ability of isolated human CD4+ CD25+ Tregs to block T-cell activation *in vitro.* Moreover, overexpression of IKZF2 in isolated murine Tregs has been shown to increase expression of Treg related markers such as CD 103 and GITR and the IKZF2 overexpressing cells showed increased suppression of responder T-cells. IKZF2 has also been found to bind the promoter of FoxP3, the defining transcription factor of the regulatory T-cell lineage, and to affect FoxP3 expression.

Knockout of IKZF2 within FoxP3-expressing Tregs in mice has been shown to cause activated Tregs to lose their inhibitory properties, to express T-effector cytokines, and to take on T-effector functions. IKZF2 knockout mutant mice develop autoimmune disease by 6-8 months of age, with increased numbers of activated CD4 and CD8 T cells, follicular helper T cells and germinal center B cells. This observed effect is believed to be cell intrinsic, as Rag2-/- mice given bone marrow from IKZF2 knockout mice, but not bone marrow from IKZF2+/+ develop autoimmune disease. Direct evidence that IKZF2 affects regulatory T-cell function has been shown in the analysis of mice in which IKZF2 was deleted only in FoxP3 expressing cells (FoxP3-YFP-Cre Heliosfl/fl). The results showed that the mice also develop autoimmune disease with similar features as observed in the whole animal IKZF2 knockout. Moreover, pathway analysis of a CHIP-SEQ experiment has also suggested that IKZF2 is affecting expression of genes in the STAT5/IL-2Rα pathway in regulatory T-cells. This effect of IKZF2 loss was shown to be more apparent after an immune challenge (viral infection or injection with sheep's blood), and it was noted that after immune stimulation, the IKZF2 negative regulatory T cells began to take on features of effector T cells. (Getnet, D., et al., Mol. Immunol. (2010), 47: 1595-1600; Bin Dhuban, K.., et al., (2015), J. Immunol. 194 :3687-96; Kim, H-J., et al., (2015), Science 350 :334-339; Nakawaga, H., et al., (2016) PNAS, 113: 6248-6253).

D1 (Schafer, P. H., et al., (2018), Ann. Rheum. Dis. 77: 15-16-1523) discloses cereblon modulator iberdomide, an orally available immunomodulatory compound under development for the treatment of systemic lupus erythematosus and relapsed/refractory multiple myeloma. D1 reports the iberomide mediated degradation of the transcription factors Ikaros and Aiolos.

Overexpression of Ikaros isoforms which lack the DNA binding regions have been shown to be associated with multiple human haematological malignancies. Recently, mutations in the IKZF2 gene, which lead to abnormal splicing variants, have been identified in adult T-cell leukemias and low hypodiploid acute lymphoblastic leukemia. It has been proposed that these isoforms, which are capable of dimerization, have a dominant negative effect on Ikaros family transcription factors which primes the development of lymphomas. IKZF2 knockout mutants that survive into adulthood do not develop lymphomas, supporting this hypothesis (Asanuma, S., et al., (2013), Cancer Sci. 104: 1097-1106; Zhang, Z., et al., (2007), Blood 109:2190-2197; Kataoka, D., et al., (2015), Nature Genetics 47: 1304-1315.)

Currently, anti-CTLA4 antibodies are used in the clinic to target Tregs in tumors. However, targeting CTLA4 often causes systemic activation of T-effector cells, resulting in excessive toxicity and limiting therapeutic utility. Up to 3/4 of patients treated with a combination of anti-PD1 and anti-CTLA4 have reported grade 3 or higher adverse events. Thus, a strong need exists to provide compounds that target Tregs in tumors without causing systemic activation of T-effector cells.

An IKZF2-specific degrader has the potential to focus the enhanced immune response to areas within or near tumors providing a potentially more tolerable and less toxic therapeutic agent for the treatment of cancer.

### SUMMARY OF THE INVENTION

The compounds of the invention have use as therapeutic agents, particularly for cancers and related diseases. In one aspect, the compounds of the invention have IKZF2 degrader activity, preferably having such activity at or below the 50 µM level, and more preferably having such activity at or below the 10 µM level. In another aspect, the compounds of the invention have degrader activity for IKZF2 that is selective over one or more of IKZF1, IKZF3, IKZF4, and/or IKZF5. In another aspect, the compounds of the invention have degrader activity for both IKZF2 and IKZF4. The compounds of the invention have usefulness in treating cancer and other diseases for which such degrader activity would be beneficial for the patient. For example, while not intending to be bound by any theory, the inventors believe that reducing levels of IKZF2 in Tregs in a tumor may allow the patient immune system to more effectively attack the disease. In summary, the present invention provides novel IKZF2 degraders useful for the treatment of cancer and other diseases.

The present invention is defined in and by the appended claims. The description below is encompassed by the invention to the extent that the subject matter is defined in the appended claims. At its most general, the present description relates to compounds for use in treating cancer.

A first aspect of the present invention relates to compounds of Formula (I) 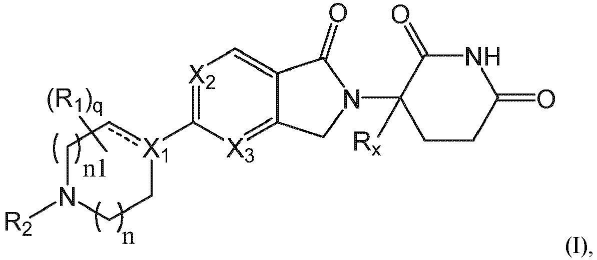 wherein:
X₁ is CR₃;
-̅-̅-̅-̅-̅-̅ is optionally a double bond when X₁ is CR₃ and R₃ is absent;
X₂ is N and X₃ is CR₁₄; or X₂ is CR₁₃ and X₃ is N; or X₂ is CR₁₅ and X₃ is CR₁₄; or X₂ is CR₁₃ and X₃ is CR₁₆;
each R₁ is independently D, (C₁-C₆)alkyl, (C₁-C₆)haloalkyl, (C₁-C₆)hydroxyalkyl, CN, or halogen, or
two R₁ together with the carbon atoms to which they are attached form (C₃-C₇)cycloalkyl or a 4- to 6-membered heterocycloalkyl ring comprising 1 to 3 heteroatoms selected from O, N, and S, or
two R₁, when on adjacent atoms, together with the atoms to which they are attached form a (C₆-C₁₀)aryl ring or a 5- or 6-membered heteroaryl ring comprising 1 to 3 heteroatoms selected from O, N, and S;
R₂ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one or more R₄; and the aryl, heteroaryl, cycloalkyl, and heterocycloalkyl are optionally substituted with one or more R₅, or
R₁ and R₂, when on adjacent atoms, together with the atoms to which they are attached form a 5- or 6-membered heterocycloalkyl ring;
R₃ is H or D, or R₃ is absent when -̅-̅-̅-̅-̅-̅ is a double bond;
each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one or more R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one or more R₁₀;
R₆ and R_{6'} are each independently H, (C₁-C₆)alkyl, or (C₆-C₁₀)aryl;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, - NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one or more R₁₁, and the aryl, heteroaryl, and heterocycloalkyl are optionally substituted with one or more substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more R₁₀, or
two R₇ together with the atoms to which they are attached form a (C₅-C₇) cycloalkyl ring or a 5- to 7-membered heterocycloalkyl ring comprising 1 to 3 heteroatoms selected from O, N, and S, optionally substituted with one or more R₁₀;
R₈ and R₉ are each independently H or (C₁-C₆)alkyl;
each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN, or
two R₁₀ together with the carbon atom to which they are attached form a =(O);
each R₁₁ is independently selected from CN, (C₁-C₆)alkoxy, (C₆-C₁₀)aryl, and 5-to 7-membered heterocycloalkyl comprising 1 to 3 heteroatoms selected from O, N, and S, wherein the aryl and heterocycloalkyl are optionally substituted with one or more substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN;
R₁₂ is (C₁-C₆)alkyl, (C₁-C₆)haloalkyl, (C₆-C₁₀)aryl, or 5- to 7-membered heterocycloalkyl comprising 1 to 3 heteroatoms selected from O, N, and S;
R₁₃ is H, halogen, -OH, or -NH₂;
R₁₄ is H, (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN;
R₁₅ is halogen, -OH, or -NH₂;
R₁₆ is (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN;
Rₓ is H or D;
p is 0, 1, or 2;
n is 0, 1, or 2;
n1 is 1 or 2, wherein n + n1 ≤ 3; and
q is 0, 1, 2, 3, or 4;
or pharmaceutically acceptable salts, hydrates, solvates, stereoisomers, and tautomers thereof.

In one aspect of the invention, the hydrogens in the compound of Formula (I) are present in their normal isotopic abundances. In a preferred aspect of the invention, the hydrogens are isotopically enriched in deuterium (D), and in a particularly preferred aspect of the invention the hydrogen at position Rₓ is enriched in D, as discussed in more detail concerning isotopes and isotopic enrichment below.

Another aspect of the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient. The pharmaceutical composition is useful in the treatment of IKZF2-dependent diseases or disorders. The pharmaceutical composition may further comprise at least one additional pharmaceutical agent.

In another aspect, the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient for use in the treatment of an IKZF2-dependent disease or disorder by reducing IKZF2 protein levels wherein reduction of IKZF2 protein levels treats the IKZF2-dependent disease or disorder. The pharmaceutical composition is useful in the treatment of IKZF2-dependent diseases or disorders. The pharmaceutical composition may further comprise at least one additional pharmaceutical agent.

Another aspect of the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient. The pharmaceutical composition is useful in the treatment of diseases or disorders affected by the reduction of IKZF2 protein levels. The pharmaceutical composition may further comprise at least one additional pharmaceutical agent.

In another aspect, the present invention relates to a pharmaceutical composition comprising a therapeutically effective amount of a compound of Formula (I), or a pharmaceutically acceptable salt, hydrate, solvate, stereoisomer, or tautomer thereof, and a pharmaceutically acceptable carrier or excipient for use in the treatment of a disease or disorder affected by the reduction of IKZF2 protein levels wherein reduction of IKZF2 protein levels treats the disease or disorder. The pharmaceutical composition may further comprise at least one additional pharmaceutical agent.

Another aspect of the present invention relates to a compound of Formula (I), or a pharmaceutically acceptable salt, hydrate, solvate, stereoisomer, or tautomer thereof, for use in the treatment of a disease or disorder that is affected by the reduction of IKZF2 protein levels.

In another aspect of the invention, the compounds according to the invention are formulated into pharmaceutical compositions comprising an effective amount, preferably a pharmaceutically effective amount, of a compound according to the invention or salt, hydrate, solvate, stereoisomer, or tautomer thereof, and a pharmaceutically acceptable excipient or carrier.

In some embodiments of the methods disclosed herein, the administration of the compound of Formula (I), or a pharmaceutically acceptable salt, hydrate, solvate, stereoisomer, or tautomer thereof, is performed orally, parentally, subcutaneously, by injection, or by infusion.

The present invention provides degraders of IKZF2 that are therapeutic agents in the treatment of diseases such as cancer and metastasis, in the treatment of diseases affected by the modulation of IKZF2 protein levels, and in the treatment IKZF2-dependent diseases or disorders.

In one embodiment, the disease or disorder that can be treated by the compounds of the present invention is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, gastrointestinal stromal tumor (GIST), prostate cancer, breast carcinoma, lymphomas, leukaemia, myeloma, bladder carcinoma, colon cancer, cutaneous melanoma, hepatocellular carcinoma, endometrial cancer, ovarian cancer, cervical cancer, lung cancer, renal cancer, glioblastoma multiform, glioma, thyroid cancer, parathyroid tumor, nasopharyngeal cancer, tongue cancer, pancreatic cancer, esophageal cancer, cholangiocarcinoma, gastric cancer, soft tissue sarcomas, rhabdomyosarcoma (RMS), synovial sarcoma, osteosarcoma, rhabdoid cancers, and Ewing's sarcoma. In another embodiment, the IKZF2-dependent disease or disorder is a cancer for which the immune response is deficient or an immunogenic cancer.

The present invention provides agents with novel mechanisms of action toward IKZF2 proteins in the treatment of various types of diseases including cancer and metastasis, in the treatment of diseases affected by the modulation of IKZF2 protein levels, and in the treatment IKZF2-dependent diseases or disorders. Ultimately the present invention provides the medical community with a novel pharmacological strategy for the treatment of diseases and disorders associated with IKZF2 proteins.

Thus, the present invention therefore provides compounds of formula (I), or pharmaceutically acceptable salts, hydrates, solvates, stereoisomers, and tautomers thereof, and also provides these compounds, pharmaceutically acceptable salts, hydrates, solvates, stereoisomers, and tautomers thereof and pharmaceutical compositions thereof, for use use in the treatment of cancer, especially in the treatment of cancers described in the claims.

Various embodiments of the invention are described herein and in the claims.

### DETAILED DESCRIPTION OF THE INVENTION

The present invention relates to compounds and compositions that are capable of modulating IKZF2 protein levels. The invention features methods of treating, preventing, or ameliorating a disease or disorder in which IKZF2 plays a role by administering to a patient in need thereof a therapeutically effective amount of a compound of Formula (I), or a pharmaceutically acceptable salt, hydrate, solvate, stereoisomer, or tautomer thereof. The methods of the present invention can be used in the treatment of a variety of IKZF2-dependent diseases and disorders by modulating IKZF2 protein levels. Modulation of IKZF2 protein levels through degradation provides a novel approach to the treatment, prevention, or amelioration of diseases including, but not limited to, cancer and metathesis, and other IKZF2-dependent diseases or disorders.

In one aspect, the compounds of the invention have use as therapeutic agents, particularly for cancers and related diseases. In one aspect, the compounds of the invention have IKZF2 degradation activity, preferably having such activity at or below the 50 µM level, and more preferably having such activity at or below the 10 µM level. In another aspect, the compounds of the invention have degrader activity for IKZF2 that is selective over one or more of IKZF1, IKZF3, IKZF4, and/or IKZF5. In another aspect, the compounds of the invention have degrader activity for both IKZF2 and IKZF4. The compounds of the invention have usefulness in treating cancer and other diseases for which such degradation activity would be beneficial for the patient. For example, while not intending to be bound by any theory, the inventors believe that reducing levels of IKZF2 in Tregs in a tumor may allow the patient immune system to more effectively attack the disease. In summary, the present invention provides novel IKZF2 degraders useful for the treatment of cancer and other diseases.

In a first aspect of the invention, the compounds of Formula (I) are described: 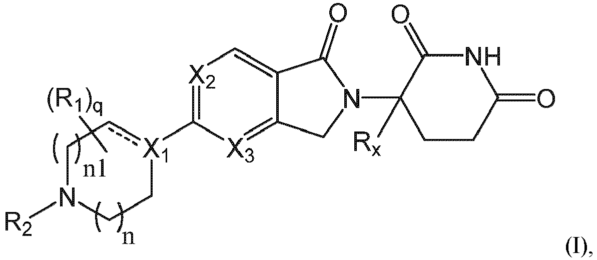 or pharmaceutically acceptable salts, hydrates, solvates, stereoisomers, and tautomers thereof, wherein R₁, R₂, Rₓ, X₁, X₂, X₃, n, n1, and q are as defined herein.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs.

### Definition of Terms and Conventions Used

Terms not specifically defined herein should be given the meanings that would be given to them by one of skill in the art in light of the invention and the context. As used in the specification and appended claims, however, unless specified to the contrary, the following terms have the meaning indicated and the following conventions are adhered to.

### A. Chemical Nomenclature, Terms, and Conventions

In the groups, radicals, or moieties defined below, the number of carbon atoms is often specified preceding the group, for example, (C₁-C₁₀)alkyl means an alkyl group or radical having 1 to 10 carbon atoms. In general, for groups comprising two or more subgroups, the last named group is the radical attachment point, for example, "alkylaryl" means a monovalent radical of the formula alkyl-aryl-, while "arylalkyl" means a monovalent radical of the formula aryl-alkyl-. Furthermore, the use of a term designating a monovalent radical where a divalent radical is appropriate shall be construed to designate the respective divalent radical and vice versa. Unless otherwise specified, conventional definitions of terms control and conventional stable atom valences are presumed and achieved in all formulas and groups. The articles "a" and "an" refer to one or more than one (e.g., to at least one) of the grammatical object of the article. By way of example, "an element" means one element or more than one element.

The term "and/or" means either "and" or "or" unless indicated otherwise.

The term "optionally substituted" means that a given chemical moiety (e.g., an alkyl group) can (but is not required to) be bonded other substituents (e.g., heteroatoms). For instance, an alkyl group that is optionally substituted can be a fully saturated alkyl chain (e.g., a pure hydrocarbon). Alternatively, the same optionally substituted alkyl group can have substituents different from hydrogen. For instance, it can, at any point along the chain be bounded to a halogen atom, a hydroxyl group, or any other substituent described herein. Thus, the term "optionally substituted" means that a given chemical moiety has the potential to contain other functional groups, but does not necessarily have any further functional groups. Suitable substituents used in the optional substitution of the described groups include, without limitation, halogen, oxo, -OH, -CN, -COOH, -CH₂CN, -O-(C₁-C₆)alkyl, (C₁-C₆)alkyl, (C₁-C₆)alkoxy, (C₁-C₆)haloalkyl, (C₁-C₆)haloalkoxy, -O-(C₂-C₆)alkenyl, -O-(C₂-C₆)alkynyl, (C₂-C₆)alkenyl, (C₂-C₆)alkynyl, -OH, - OP(O)(OH)₂, -OC(O)(C₁-C₆)alkyl, -C(O)(C₁-C₆)alkyl, -OC(O)O(C₁-C₆)alkyl, -NH₂, -NH((C₁-C₆)alkyl), - N((C₁-C₆)alkyl)₂, -NHC(O)(C₁-C₆)alkyl, -C(O)NH(C₁-C₆)alkyl, -S(O)₂(C₁-C₆)alkyl, -S(O)NH(C₁-C₆)alkyl, and S(O)N((C₁-C₆)alkyl)₂. The substituents can themselves be optionally substituted. "Optionally substituted" as used herein also refers to substituted or unsubstituted whose meaning is described below.

The term "substituted" means that the specified group or moiety bears one or more suitable substituents wherein the substituents may connect to the specified group or moiety at one or more positions. For example, an aryl substituted with a cycloalkyl may indicate that the cycloalkyl connects to one atom of the aryl with a bond or by fusing with the aryl and sharing two or more common atoms.

The term "unsubstituted" means that the specified group bears no substituents.

Unless otherwise specifically defined, "aryl" means a cyclic, aromatic hydrocarbon group having 1 to 3 aromatic rings, including monocyclic or bicyclic groups such as phenyl, biphenyl, or naphthyl. When containing two aromatic rings (bicyclic, etc.), the aromatic rings of the aryl group are optionally joined at a single point (e.g., biphenyl), or fused (e.g., naphthyl). The aryl group is optionally substituted by one or more substituents, e.g., 1 to 5 substituents, at any point of attachment. Exemplary substituents include, but are not limited to, -H, -halogen, -CN, -O-(C₁-C₆)alkyl, (C₁-C₆)alkyl, -O-(C₂-C₆)alkenyl, -O-(C₂-C₆)alkynyl, (C₂-C₆)alkenyl, (C₂-C₆)alkynyl, -OH, -OP(O)(OH)₂, -OC(O)(C₁-C₆)alkyl, -C(O)(C₁-C₆)alkyl, - OC(O)O(C₁-C₆) alkyl, NH₂, NH((C₁-C₆)alkyl), N((C₁-C₆)alkyl)₂, -S(O)₂-(C₁-C₆)alkyl, -S(O)NH(C₁-C₆)alkyl, and S(O)N((C₁-C₆)alkyl)₂. The substituents are themselves optionally substituted. Furthermore, when containing two fused rings, the aryl groups optionally have an unsaturated or partially saturated ring fused with a fully saturated ring. Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, and the like.

Unless otherwise specifically defined, "heteroaryl" means a monovalent monocyclic aromatic radical of 5 to 24 ring atoms or a polycyclic aromatic radical, containing one or more ring heteroatoms selected from N, O, or S, the remaining ring atoms being C. Heteroaryl as herein defined also means a bicyclic heteroaromatic group wherein the heteroatom is selected from N, O, or S. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, dihydrobenzoxanyl, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydropyrrolo[1,2-a]pyrimidinyl, 3,4-dihydro-2H-1Δ²-pyrrolo[2,1-b]pyrimidine, dibenzo[b,d]thiophene, pyridin-2-one, furo[3,2-c]pyridinyl, furo[2,3-c]pyridinyl, 1H-pyrido[3,4-b][1,4]thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo[1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo[1,5-b][1,2]oxazinyl, 4,5,6,7-tetrahydropyrazolo[1,5-a]pyridinyl, thiazolo[5,4 d]thiazolyl, imidazo[2,1-b][1,3,4]thiadiazolyl, thieno[2,3-b]pyrrolyl, 3H-indolyl, and derivatives thereof. Furthermore, when containing two fused rings the aryl groups herein defined may have an unsaturated or partially saturated ring fused with a fully saturated ring. Exemplary ring systems of these heteroaryl groups include indolinyl, indolinonyl, dihydrobenzothiophenyl, dihydrobenzofuran, chromanyl, thiochromanyl, tetrahydroquinolinyl, dihydrobenzothiazine,3,4-dihydro-1H-isoquinolinyl, 2,3-dihydrobenzofuran, indolinyl, indolyl, and dihydrobenzoxanyl.

Halogen or "halo" mean fluorine, chlorine, bromine, or iodine.

"Alkyl" means a straight or branched chain saturated hydrocarbon containing 1-12 carbon atoms. Examples of a (C₁-C₆)alkyl group include, but are not limited to, methyl, ethyl, propyl, butyl, pentyl, hexyl, isopropyl, isobutyl, *sec*-butyl, *tert*-butyl, isopentyl, neopentyl, and isohexyl.

"Alkoxy" means a straight or branched chain saturated hydrocarbon containing 1-12 carbon atoms containing a terminal "O" in the chain, e.g., -O(alkyl). Examples of alkoxy groups include, without limitation, methoxy, ethoxy, propoxy, butoxy, t-butoxy, or pentoxy groups.

"Alkenyl" means a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, *n*-butenyl, iso-butenyl, pentenyl, or hexenyl. An alkenyl group can be unsubstituted or substituted and may be straight or branched.

"Alkynyl" means a straight or branched chain unsaturated hydrocarbon containing 2-12 carbon atoms. The "alkynyl" group contains at least one triple bond in the chain. Examples of alkenyl groups include ethynyl, propargyl, *n*-butynyl, iso-butynyl, pentynyl, or hexynyl. An alkynyl group can be unsubstituted or substituted.

"Alkylene" or "alkylenyl" means a divalent alkyl radical. Any of the above mentioned monovalent alkyl groups may be an alkylene by abstraction of a second hydrogen atom from the alkyl. As herein defined, alkylene may also be a (C₁-C₆)alkylene. An alkylene may further be a (C₁-C₄)alkylene. Typical alkylene groups include, but are not limited to, -CH₂-, -CH(CH₃)-, -C(CH₃)₂-, -CH₂CH₂-, -CH₂CH(CH₃)-, - CH₂C(CH₃)₂-, -CH₂CH₂CH₂-, -CH₂CH₂CH₂CH-, and the like.

"Cycloalkyl" or "carbocyclyl" means a monocyclic or polycyclic saturated or partially unsaturated non-aromatic carbon ring containing 3-18 carbon atoms. Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl and derivatives thereof. A (C₃-C₈)cycloalkyl is a cycloalkyl group containing between 3 and 8 carbon atoms. A cycloalkyl group can be fused (e.g., decalin) or bridged (e.g., norbornane).

"Heterocyclyl" or "heterocycloalkyl" means a saturated or partially saturated monocyclic or polycyclic ring containing carbon and at least one heteroatom selected from oxygen, nitrogen, or sulfur (O, N, or S) and wherein there is not delocalized n electrons (aromaticity) shared among the ring carbon or heteroatoms. The heterocycloalkyl ring structure may be substituted by one or more substituents. The substituents can themselves be optionally substituted. Examples of heterocyclyl rings include, but are not limited to, oxetanyl, azetadinyl, tetrahydrofuranyl, tetrahydropyranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, oxazolidinonyl, 1,4-dioxanyl, dihydrofuranyl, 1,3-dioxolanyl, imidazolidinyl, imidazolinyl, dithiolanyl, and homotropanyl.

"Hydroxyalkyl" means an alkyl group substituted with one or more -OH groups. Examples of hydroxyalkyl groups include HO-CH₂-, HO-CH₂CH₂-, and CH₂-CH(OH)-.

"Haloalkyl" means an alkyl group substituted with one or more halogens. Examples of haloalkyl groups include, but are not limited to, trifluoromethyl, difluoromethyl, pentafluoroethyl, trichloromethyl, etc.

"Haloalkoxy" means an alkoxy group substituted with one or more halogens. Examples of haloalkyl groups include, but are not limited to, trifluoromethoxy, difluoromethoxy, pentafluoroethoxy, trichloromethoxy, etc.

"Cyano" means a substituent having a carbon atom joined to a nitrogen atom by a triple bond, e.g., C≡N.

"Amino" means a substituent containing at least one nitrogen atom (e.g., NH₂).

"Pomalidomide" or 4-amino-2-(2,6-dioxopiperidin-3-yl)isoindoline-1,3-dione has the following structure:

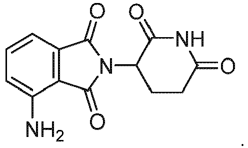

### B. Salt, and Solvate Terms and Conventions

"Salt" means an ionic form of the parent compound or the product of the reaction between the parent compound with a suitable acid or base to make the acid salt or base salt of the parent compound. Salts of the compounds of the present invention can be synthesized from the parent compounds which contain a basic or acidic moiety by conventional chemical methods. Generally, the salts are prepared by reacting the free base or acid parent compound with stoichiometric amounts or with an excess of the desired salt-forming inorganic or organic acid or base in a suitable solvent or various combinations of solvents.

"Pharmaceutically acceptable salt" means a salt of a compound of the invention which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. As the compounds of the present invention are useful in both free base and salt form, in practice, the use of the salt form amounts to use of the base form. Lists of suitable salts are found in, e.g., S.M. Birge et al., J. Pharm. Sci., 1977, 66, pp. 1-19.

"Pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid, sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, pyruvic acid, salicylic acid, stearic acid, succinic acid, sulfanilic acid, tartaric acid, p-toluenesulfonic acid, undecanoic acid, and the like.

"Pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, polyamine resins, and the like. Particularly preferred organic nontoxic bases are isopropylamine, diethylamine, ethanolamine, trimethylamine, dicyclohexylamine, choline, and caffeine.

"Solvate" means a complex of variable stoichiometry formed by a solute, for example, a compound of Formula (I)) and solvent, for example, water, ethanol, or acetic acid. This physical association may involve varying degrees of ionic and covalent bonding, including hydrogen bonding. In certain instances, the solvate will be capable of isolation, for example, when one or more solvent molecules are incorporated in the crystal lattice of the crystalline solid. In general, such solvents selected for the purpose of the invention do not interfere with the biological activity of the solute. Solvates encompasses both solution-phase and isolatable solvates. Representative solvates include hydrates, ethanolates, methanolates, and the like.

"Hydrate" means a solvate wherein the solvent molecule(s) is/are water.

The compounds of the present invention as discussed below include the free base or acid thereof, their salts, and solvates and may include oxidized sulfur atoms or quaternized nitrogen atoms in their structure, although not explicitly stated or shown, particularly the pharmaceutically acceptable forms thereof. Such forms, particularly the pharmaceutically acceptable forms, are intended to be embraced by the appended claims.

### C. Isomer Terms and Conventions

"Isomers" means compounds having the same number and kind of atoms, and hence the same molecular weight, but differing with respect to the arrangement or configuration of the atoms in space. The term includes stereoisomers and geometric isomers.

"Stereoisomer" or "optical isomer" mean a stable isomer that has at least one chiral atom or restricted rotation giving rise to perpendicular dissymmetric planes (e.g., certain biphenyls, allenes, and spiro compounds) and can rotate plane-polarized light. Because asymmetric centers and other chemical structure exist in the compounds of the invention which may give rise to stereoisomerism, the invention contemplates stereoisomers and mixtures thereof. The compounds of the invention and their salts include asymmetric carbon atoms and may therefore exist as single stereoisomers, racemates, and as mixtures of enantiomers and diastereomers. Typically, such compounds will be prepared as a racemic mixture. If desired, however, such compounds can be prepared or isolated as pure stereoisomers, *i.e.,* as individual enantiomers or diastereomers, or as stereoisomer-enriched mixtures. As discussed in more detail below, individual stereoisomers of compounds are prepared by synthesis from optically active starting materials containing the desired chiral centers or by preparation of mixtures of enantiomeric products followed by separation or resolution, such as conversion to a mixture of diastereomers followed by separation or recrystallization, chromatographic techniques, use of chiral resolving agents, or direct separation of the enantiomers on chiral chromatographic columns. Starting compounds of particular stereochemistry are either commercially available or are made by the methods described below and resolved by techniques well-known in the art.

"Enantiomers" means a pair of stereoisomers that are non-superimposable mirror images of each other.

"Diastereoisomers" or "diastereomers" mean optical isomers which are not mirror images of each other.

"Racemic mixture" or "racemate" mean a mixture containing equal parts of individual enantiomers.

"Non-racemic mixture" means a mixture containing unequal parts of individual enantiomers.

"Geometrical isomer" means a stable isomer, which results from restricted freedom of rotation about double bonds (e.g., cis-2-butene and trans-2-butene) or in a cyclic structure (e.g., cis-1,3-dichlorocyclobutane and trans-1,3-dichlorocyclobutane). Because carbon-carbon double (olefinic) bonds, C=N double bonds, cyclic structures, and the like may be present in the compounds of the invention, the invention contemplates each of the various stable geometric isomers and mixtures thereof resulting from the arrangement of substituents around these double bonds and in these cyclic structures. The substituents and the isomers are designated using the cis/trans convention or using the E or Z system, wherein the term "E" means higher order substituents on opposite sides of the double bond, and the term "Z" means higher order substituents on the same side of the double bond. A thorough discussion of E and Z isomerism is provided in J. March, Advanced Organic Chemistry: Reactions, Mechanisms, and Structure, 4th ed., John Wiley & Sons, 1992. Several of the following examples represent single E isomers, single Z isomers, and mixtures of E/Z isomers. Determination of the E and Z isomers can be done by analytical methods such as x-ray crystallography, ¹H NMR, and ¹³C NMR.

Some of the compounds of the invention can exist in more than one tautomeric form. As mentioned above, the compounds of the invention include all such tautomers.

It is well-known in the art that the biological and pharmacological activity of a compound is sensitive to the stereochemistry of the compound. Thus, for example, enantiomers often exhibit strikingly different biological activity including differences in pharmacokinetic properties, including metabolism, protein binding, and the like, and pharmacological properties, including the type of activity displayed, the degree of activity, toxicity, and the like. Thus, one skilled in the art will appreciate that one enantiomer may be more active or may exhibit beneficial effects when enriched relative to the other enantiomer or when separated from the other enantiomer. Additionally, one skilled in the art would know how to separate, enrich, or selectively prepare the enantiomers of the compounds of the invention from this invention and the knowledge of the prior art.

Thus, although the racemic form of drug may be used, it is often less effective than administering an equal amount of enantiomerically pure drug; indeed, in some cases, one enantiomer may be pharmacologically inactive and would merely serve as a simple diluent. For example, although ibuprofen had been previously administered as a racemate, it has been shown that only the S-isomer of ibuprofen is effective as an anti-inflammatory agent (in the case of ibuprofen, however, although the R-isomer is inactive, it is converted in vivo to the S-isomer, thus, the rapidity of action of the racemic form of the drug is less than that of the pure S-isomer). Furthermore, the pharmacological activities of enantiomers may have distinct biological activity. For example, S-penicillamine is a therapeutic agent for chronic arthritis, while R-penicillamine is toxic. Indeed, some purified enantiomers have advantages over the racemates, as it has been reported that purified individual isomers have faster transdermal penetration rates compared to the racemic mixture. See U.S. Pat. Nos. 5,114,946 and 4,818,541.

Thus, if one enantiomer is pharmacologically more active, less toxic, or has a preferred disposition in the body than the other enantiomer, it would be therapeutically more beneficial to administer that enantiomer preferentially. In this way, the patient undergoing treatment would be exposed to a lower total dose of the drug and to a lower dose of an enantiomer that is possibly toxic or an inhibitor of the other enantiomer.

Preparation of pure enantiomers or mixtures of desired enantiomeric excess (ee) or enantiomeric purity are accomplished by one or more of the many methods of (a) separation or resolution of enantiomers, or (b) enantioselective synthesis known to those of skill in the art, or a combination thereof. These resolution methods generally rely on chiral recognition and include, for example, chromatography using chiral stationary phases, enantioselective host-guest complexation, resolution or synthesis using chiral auxiliaries, enantioselective synthesis, enzymatic and nonenzymatic kinetic resolution, or spontaneous enantioselective crystallization. Such methods are disclosed generally in Chiral Separation Techniques: A Practical Approach (2nd Ed.), G. Subramanian (ed.), Wiley-VCH, 2000; T.E. Beesley and R.P.W. Scott, Chiral Chromatography, John Wiley & Sons, 1999; and Satinder Ahuja, Chiral Separations by Chromatography, Am. Chem. Soc., 2000. Furthermore, there are equally well-known methods for the quantitation of enantiomeric excess or purity, for example, GC, HPLC, CE, or NMR, and assignment of absolute configuration and conformation, for example, CD ORD, X-ray crystallography, or NMR.

In general, all tautomeric forms and isomeric forms and mixtures, whether individual geometric isomers or stereoisomers or racemic or non-racemic mixtures, of a chemical structure or compound is intended, unless the specific stereochemistry or isomeric form is specifically indicated in the compound name or structure.

### D. Pharmaceutical Administration and Treatment Terms and Conventions

A "patient" or "subject" is a mammal, e.g., a human, mouse, rat, guinea pig, dog, cat, horse, cow, pig, or nonhuman primate, such as a monkey, chimpanzee, baboon or, rhesus. In certain embodiments, the subject is a primate. In yet other embodiments, the subject is a human.

An "effective amount" or "therapeutically effective amount" when used in connection with a compound means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein.

The terms "pharmaceutically effective amount" or "therapeutically effective amount" means an amount of a compound according to the invention which, when administered to a patient in need thereof, is sufficient to effect treatment for disease-states, conditions, or disorders for which the compounds have utility. Such an amount would be sufficient to elicit the biological or medical response of a tissue, system, or patient that is sought by a researcher or clinician. The amount of a compound of according to the invention which constitutes a therapeutically effective amount will vary depending on such factors as the compound and its biological activity, the composition used for administration, the time of administration, the route of administration, the rate of excretion of the compound, the duration of treatment, the type of disease-state or disorder being treated and its severity, drugs used in combination with or coincidentally with the compounds of the invention, and the age, body weight, general health, sex, and diet of the patient. Such a therapeutically effective amount can be determined routinely by one of ordinary skill in the art having regard to their own knowledge, the prior art, and this invention.

As used herein, the term "pharmaceutical composition" refers to a compound of the invention, or a pharmaceutically acceptable salt, hydrate, solvate, stereoisomer, or tautomer thereof, together with at least one pharmaceutically acceptable carrier, in a form suitable for oral or parenteral administration.

"Carrier" encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

A subject is "in need of' a treatment if such subject would benefit biologically, medically, or in quality of life from such treatment (preferably, a human).

As used herein, the term "inhibit", "inhibition", or "inhibiting" refers to the reduction or suppression of a given condition, symptom, or disorder, or disease, or a significant decrease in the baseline activity of a biological activity or process.

As used herein, the term "treat", "treating", or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing", or "prevention" of any disease or disorder refers to the prophylactic treatment of the disease or disorder; or delaying the onset or progression of the disease or disorder.

"Pharmaceutically acceptable" means that the substance or composition must be compatible chemically and/or toxicologically, with the other ingredients comprising a formulation, and/or the mammal being treated therewith.

"Disorder" means, and is used interchangeably with, the terms disease, condition, or illness, unless otherwise indicated.

"Administer", "administering", or "administration" means to either directly administering a disclosed compound or pharmaceutically acceptable salt of the disclosed compound or a composition to a subject, which can form an equivalent amount of active compound within the subject's body.

"Compounds of the present invention", "compounds of the invention", and equivalent expressions (unless specifically identified otherwise) refer to compounds of Formulae (I), (Ia), (Ib), (Ic), (Id), (Ie), (If), (Ig), (Ih), (Ii), (Ij), (Ik), and (Il), as herein described including the tautomers, salts particularly the pharmaceutically acceptable salts, and the solvates and hydrates thereof, where the context so permits thereof, as well as all stereoisomers (including diastereoisomers and enantiomers), rotamers, tautomers, and isotopically labelled compounds (including deuterium substitutions), as well as inherently formed moieties (e.g., polymorphs, solvates and/or hydrates). For purposes of this invention, solvates and hydrates are generally considered compositions. In general and preferably, the compounds of the invention and the formulas designating the compounds of the invention are understood to only include the stable compounds thereof and exclude unstable compounds, even if an unstable compound might be considered to be literally embraced by the compound formula. Similarly, reference to intermediates, whether or not they themselves are claimed, is meant to embrace their salts and solvates, where the context so permits. For the sake of clarity, particular instances when the context so permits are sometimes indicated in the text, but these instances are purely illustrative and it is not intended to exclude other instances when the context so permits.

"Stable compound" or "stable structure" means a compound that is sufficiently robust to survive isolation to a useful degree of purity from a reaction mixture, and formulation into an efficacious therapeutic or diagnostic agent. For example, a compound, which would have a "dangling valency" or is a carbanion is not a compound contemplated by the invention.

In a specific embodiment, the term "about" or "approximately" means within 20%, preferably within 10%, and more preferably within 5% of a given value or range.

The yield of each of the reactions described herein is expressed as a percentage of the theoretical yield. "Cancer" means any cancer caused by the proliferation of malignant neoplastic cells, such as tumors, neoplasms, carcinomas, sarcomas, leukemias, lymphomas, and the like. For example, cancers include, but are not limited to, mesothelioma, leukemias, and lymphomas such as cutaneous T-cell lymphomas (CTCL), noncutaneous peripheral T-cell lymphomas, lymphomas associated with human T-cell lymphotrophic virus (HTLV) such as adult T-cell leukemia/lymphoma (ATLL), B-cell lymphoma, acute nonlymphocytic leukemias, chronic lymphocytic leukemia, chronic myelogenous leukemia, acute myelogenous leukemia, lymphomas, and multiple myeloma, non-Hodgkin lymphoma, acute lymphatic leukemia (ALL), chronic lymphatic leukemia (CLL), Hodgkin's lymphoma, Burkitt lymphoma, adult T-cell leukemia lymphoma, acute-myeloid leukemia (AML), chronic myeloid leukemia (CML), or hepatocellular carcinoma. Further examples include myelodisplastic syndrome, childhood solid tumors such as brain tumors, neuroblastoma, retinoblastoma, Wilms' tumor, bone tumors, and soft-tissue sarcomas, common solid tumors of adults such as head and neck cancers (e.g., oral, laryngeal, and nasopharyngeal), esophageal cancer, genitourinary cancers (e.g., prostate, bladder, renal, uterine, ovarian, testicular), lung cancer (e.g., small-cell and non-small cell), breast cancer, pancreatic cancer, melanoma, and other skin cancers, stomach cancer, brain tumors, tumors related to Gorlin's syndrome (e.g., medulloblastoma, meningioma, etc.), and liver cancer. Additional exemplary forms of cancer which may be treated by the subject compounds include, but are not limited to, cancer of skeletal or smooth muscle, stomach cancer, cancer of the small intestine, rectum carcinoma, cancer of the salivary gland, endometrial cancer, adrenal cancer, anal cancer, rectal cancer, parathyroid cancer, and pituitary cancer.

Additional cancers that the compounds described herein may be useful in preventing, treating, and studying are, for example, colon carcinoma, familiary adenomatous polyposis carcinoma, and hereditary non-polyposis colorectal cancer, or melanoma. Further, cancers include, but are not limited to, labial carcinoma, larynx carcinoma, hypopharynx carcinoma, tongue carcinoma, salivary gland carcinoma, gastric carcinoma, adenocarcinoma, thyroid cancer (medullary and papillary thyroid carcinoma), renal carcinoma, kidney parenchyma carcinoma, cervix carcinoma, uterine corpus carcinoma, endometrium carcinoma, chorion carcinoma, testis carcinoma, urinary carcinoma, melanoma, brain tumors such as glioblastoma, astrocytoma, meningioma, medulloblastoma and peripheral neuroectodermal tumors, gall bladder carcinoma, bronchial carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing's sarcoma, and plasmocytoma.

"Simultaneously" or "simultaneous" when referring to a method of treating or a therapeutic use means with a combination of a compound of Formula (I), or a pharmaceutically acceptable salt, hydrate, solvate, stereoisomer, or tautomer thereof, and one or more second agent(s) means administration of the compound and the one or more second agent(s) by the same route and at the same time.

"Separately" or "separate" when referring to a method of treating or a therapeutic use means with a combination of a compound of Formula (I), or a pharmaceutically acceptable salt, hydrate, solvate, stereoisomer, or tautomer thereof, and one or more second agent(s) means administration of the compound and the one or more second agent(s) by different routes and at approximately the same time.

By therapeutic administration "over a period of time" means, when referring to a method of treating or a therapeutic use with a combination of a compound of Formula (I), or a pharmaceutically acceptable salt, hydrate, solvate, stereoisomer, or tautomer thereof, and one or more second agent(s), administration of the compound and the one or more second agent(s) by the same or different routes and at different times. In some embodiments, the administration of the compound or the one or more second agent(s) occurs before the administration of the other begins. In this way, it is possible to administer a one of the active ingredients (i.e., a compound of the Formula (I), or a pharmaceutically acceptable salt, hydrate, solvate, stereoisomer, or tautomer thereof, or one or more second agent(s)) for several months before administering the other active ingredient or ingredients. In this case, no simultaneous administration occurs. Another therapeutic administration over a period of time consists of the administration over time of the two or more active ingredients of the combination using different frequencies of administration for each of the active ingredients, whereby at certain time points in time simultaneous administration of all of the active ingredients takes place whereas at other time points in time only a part of the active ingredients of the combination may be administered (e.g., for example, a compound of formula (I), or a pharmaceutically acceptable salt, hydrate, solvate, stereoisomer, or tautomer thereof, and the one or more second agents the therapeutic administration over a period of time could be such that a compound of Formula (I), or a pharmaceutically acceptable salt, hydrate, solvate, stereoisomer, or tautomer thereof, is administered once a day and the one or more second agent(s) is administered once every four weeks.)

"IKZF2-dependent disease or disorder" means any disease or disorder which is directly or indirectly affected by the modulation of IKZF2 protein levels.

"IKZF4-dependent disease or disorder" means any disease or disorder which is directly or indirectly affected by the modulation of IKZF4 protein levels.

### D. Specific Embodiments and Methods for Testing Compounds of Formula (I)

The present invention relates to compounds or pharmaceutically acceptable salts, hydrates, solvates, stereoisomers, or tautomers thereof, capable of modulating IKZF2 protein levels, which are useful for the treatment of diseases and disorders associated with modulation of IKZF2 protein levels. The invention further relates to compounds, or pharmaceutically acceptable salts, hydrates, solvates, stereoisomers, or tautomers thereof, which are useful for reducing or decreasing IKZF2 protein levels.

In one embodiment, the compounds of Formula (I) have the structure of Formula (Ia): 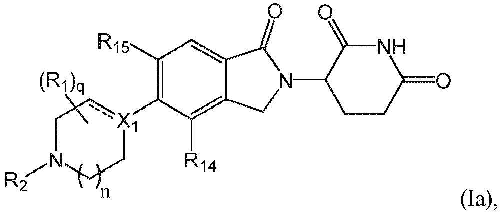 or pharmaceutically acceptable salts, hydrates, solvates, stereoisomers, and tautomers thereof.

In another embodiment, the compounds of Formula (I) have the structure of Formula (Ib): 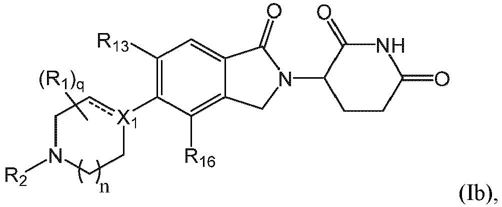 or pharmaceutically acceptable salts, hydrates, solvates, stereoisomers, and tautomers thereof.

In another embodiment, the compounds of Formula (I) have the structure of Formula (Ic): 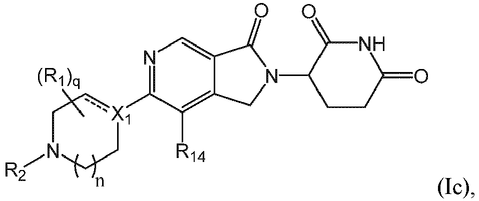 or pharmaceutically acceptable salts, hydrates, solvates, stereoisomers, and tautomers thereof.

In another embodiment, the compounds of Formula (I) have the structure of Formula (Id): 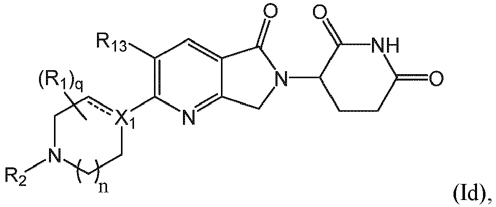 or pharmaceutically acceptable salts, hydrates, solvates, stereoisomers, and tautomers thereof.

In another embodiment, the compounds of Formula (I) have the structure of Formula (Ie): 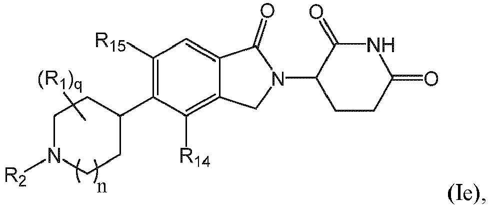 or pharmaceutically acceptable salts, hydrates, solvates, stereoisomers, and tautomers thereof.

In another embodiment, the compounds of Formula (I) have the structure of Formula (If): 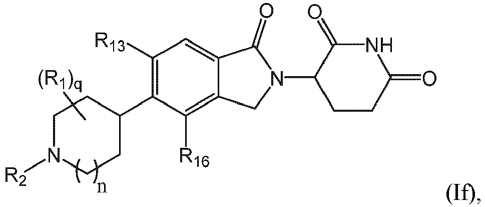 or pharmaceutically acceptable salts, hydrates, solvates, stereoisomers, and tautomers thereof.

In another embodiment, the compounds of Formula (I) have the structure of Formula (Ig): 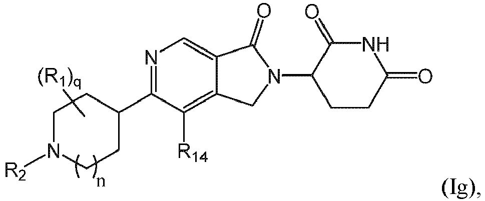 or pharmaceutically acceptable salts, hydrates, solvates, stereoisomers, and tautomers thereof.

In another embodiment, the compounds of Formula (I) have the structure of Formula (Ih): 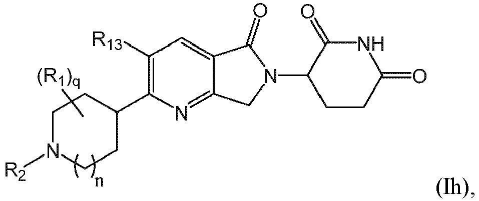 or pharmaceutically acceptable salts, hydrates, solvates, stereoisomers, and tautomers thereof.

In another embodiment, the compounds of Formula (I) have the structure of Formula (Ii): 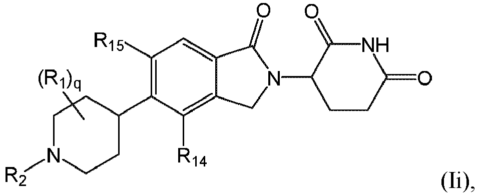 or pharmaceutically acceptable salts, hydrates, solvates, stereoisomers, and tautomers thereof.

In another embodiment, the compounds of Formula (I) have the structure of Formula (Ij): 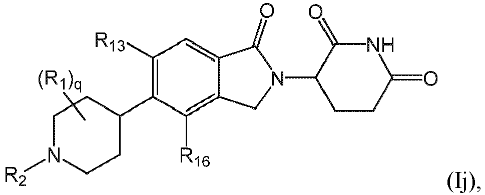 or pharmaceutically acceptable salts, hydrates, solvates, stereoisomers, and tautomers thereof.

In another embodiment, the compounds of Formula (I) have the structure of Formula (Ik): 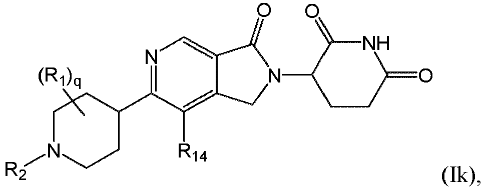 or pharmaceutically acceptable salts, hydrates, solvates, stereoisomers, and tautomers thereof.

In another embodiment, the compounds of Formula (I) have the structure of Formula (Il): 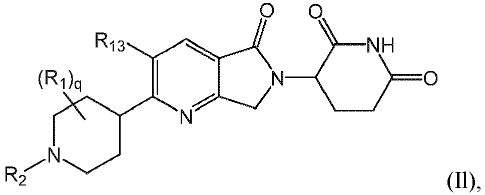 or pharmaceutically acceptable salts, hydrates, solvates, stereoisomers, and tautomers thereof.

In some embodiments of the formulae above (e.g., Formula (I), Formula (Ia), Formula (Ib) Formula (Ic), or Formula (Id) Formula (Ie), Formula (If), Formula (Ig), or Formula (Ih) Formula (Ii), Formula (Ij), Formula (Ik), and/or Formula (Il)), wherein:
R₂ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one to four R₄; and the aryl, heteroaryl, cycloalkyl, and heterocycloalkyl are optionally substituted with one to four R₅, or
R₁ and R₂, when on adjacent atoms, together with the atoms to which they are attached form a 5- or 6- membered heterocycloalkyl ring;
each R₄ is independently selected from -C(O)OR₆, -C(O)NR₆R_{6'}, -NR₆C(O)R_{6'}, halogen, -OH, - NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or
two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one to four R₁₀;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, - O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or
two R₇ together with the atoms to which they are attached form a (C₅-C₇) cycloalkyl ring or a 5-to 7-membered heterocycloalkyl ring comprising 1 to 3 heteroatoms selected from O, N, and S, optionally substituted with one to four R₁₀;
each R₁₁ is independently selected from CN, (C₁-C₆)alkoxy, (C₆-C₁₀)aryl, and 5-to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN;
or a pharmaceutically acceptable salt, hydrate, solvate, stereoisomer, or tautomer thereof.

In some embodiments of the formulae above, Rₓ is D. In another embodiment, Rₓ is H.

In some embodiments of the formulae above, X₁ is CR₃.

In some embodiments of the formulae above, X₂ is N and X₃ is CR₁₄. In another embodiment, X₂ is CR₁₃ and X₃ is N. In another embodiment, X₂ is CR₁₅ and X₃ is CR₁₄. In another embodiment, X₂ is CR₁₃ and X₃ is CR₁₆. In another embodiment, X₂ is N and X₃ is CH. In another embodiment, X₂ is CH and X₃ is N. In another embodiment, X₂ is CH and X₃ is CR₁₆. In another embodiment, X₂ is CR₁₅ and X₃ is CH.

In some embodiments of the formulae above, each R₁ is independently (C₁-C₆)haloalkyl, (C₁-C₆)hydroxyalkyl, CN, or halogen. In another embodiment, each R₁ is independently (C₁-C₆)alkyl, (C₁-C₆)haloalkyl, CN, or halogen. In yet another embodiment, each R₁ is independently (C₁-C₆)alkyl, (C₁-C₆)hydroxyalkyl, CN, or halogen. In another embodiment, each R₁ is independently (C₁-C₆)alkyl, (C₁-C₆)haloalkyl, CN, or halogen. In yet another embodiment, each R₁ is independently (C₁-C₆)alkyl or (C₁-C₆)haloalkyl.

In another embodiment, each R₁ is independently (C₁-C₆)haloalkyl, (C₁-C₆)hydroxyalkyl, or halogen. In another embodiment, each R₁ is independently (C₁-C₆)alkyl, (C₁-C₆)haloalkyl, or halogen. In yet another embodiment, each R₁ is independently (C₁-C₆)alkyl, (C₁-C₆)hydroxyalkyl, or halogen. In another embodiment, each R₁ is independently (C₁-C₆)alkyl, (C₁-C₆)haloalkyl, or halogen. In yet another embodiment, each R₁ is independently (C₁-C₆)alkyl or (C₁-C₆)haloalkyl. In another embodiment, each R₁ is independently (C₁-C₆)alkyl or halogen. In yet another embodiment, each R₁ is independently (C₁-C₆)haloalkyl or halogen. In another embodiment, each R₁ is independently D or (C₁-C₆)alkyl. In another embodiment, each R₁ is independently (C₁-C₆)alkyl.

In some embodiments of the formulae above, two R₁ together with the carbon atoms to which they are attached form a (C₃-C₇)cycloalkyl or a 4- to 6- membered heterocycloalkyl ring comprising 1 to 3 heteroatoms selected from O, N, and S. In another embodiment, two R₁ together with the carbon atoms to which they are attached form a (C₃-C₇)cycloalkyl or a 5- or 6- membered heterocycloalkyl ring comprising 1 to 3 heteroatoms selected from O, N, and S. In yet another embodiment, two R₁ together with the carbon atoms to which they are attached form a (C₃-C₇)cycloalkyl or a 4- or 5- membered heterocycloalkyl ring comprising 1 to 3 heteroatoms selected from O, N, and S. In another embodiment, two R₁ together with the carbon atoms to which they are attached form a (C₄-C₇)cycloalkyl or a 4- to 6- membered heterocycloalkyl ring comprising 1 to 3 heteroatoms selected from O, N, and S. In yet another embodiment, two R₁ together with the carbon atoms to which they are attached form a (C₄-C₆)cycloalkyl or a 4- to 6- membered heterocycloalkyl ring comprising 1 to 3 heteroatoms selected from O, N, and S.

In another embodiment, two R₁ together with the carbon atoms to which they are attached form a (C₃-C₇)cycloalkyl. In yet another embodiment, two R₁ together with the carbon atoms to which they are attached form a (C₃-C₆)cycloalkyl. In another embodiment, two R₁ together with the carbon atoms to which they are attached form a (C₄-C₇)cycloalkyl. In yet another embodiment, two R₁ together with the carbon atoms to which they are attached form a (C₅-C₇)cycloalkyl. In another embodiment, two R₁ together with the carbon atoms to which they are attached form a (C₆-C₇)cycloalkyl. In yet another embodiment, two R₁ together with the carbon atoms to which they are attached form a (C₅-C₆)cycloalkyl. In another embodiment, two R₁ together with the carbon atoms to which they are attached form a (C₄-C₆)cycloalkyl. In yet another embodiment, two R₁ together with the carbon atoms to which they are attached form a (C₃-C₆)cycloalkyl. In another embodiment, two R₁ together with the carbon atoms to which they are attached form a (C₃-C₅)cycloalkyl. In yet another embodiment, two R₁ together with the carbon atoms to which they are attached form a 4- to 6- membered heterocycloalkyl ring comprising 1 to 3 heteroatoms selected from O, N, and S. In another embodiment, two R₁ together with the carbon atoms to which they are attached form a 5- or 6-membered heterocycloalkyl ring comprising 1 to 3 heteroatoms selected from O, N, and S. In yet another embodiment, two R₁ together with the carbon atoms to which they are attached form a 4- or 5- membered heterocycloalkyl ring comprising 1 to 3 heteroatoms selected from O, N, and S.

In some embodiments of the formulae above, two R₁, when on adjacent atoms, together with the atoms to which they are attached form a phenyl ring or a 5- or 6-membered heteroaryl ring comprising 1 to 3 heteroatoms selected from O, N, and S. In another embodiment, two R₁, when on adjacent atoms, together with the atoms to which they are attached form a phenyl ring. In another embodiment, two R₁, when on adjacent atoms, together with the atoms to which they are attached form a phenyl ring. In yet another embodiment, two R₁, when on adjacent atoms, together with the atoms to which they are attached form a 5- or 6-membered heteroaryl ring comprising 1 to 3 heteroatoms selected from O, N, and S. In another embodiment, two R₁, when on adjacent atoms, together with the atoms to which they are attached form a 5-membered heteroaryl ring comprising 1 to 3 heteroatoms selected from O, N, and S. In yet another embodiment, two R₁, when on adjacent atoms, together with the atoms to which they are attached form a 6-membered heteroaryl ring comprising 1 to 3 heteroatoms selected from O, N, and S.

In some embodiments of the formulae above, R₂ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one to four R₄; and the aryl, heteroaryl, cycloalkyl, and heterocycloalkyl are optionally substituted with one to four R₅. In another embodiment, R₂ is (C₁-C₄)alkyl, (C₆-C₁₀)aryl, (C₃-C₈)cycloalkyl, or 5- to 7-membered heterocycloalkyl comprising 1 to 3 heteroatoms selected from O, N, and S, wherein the alkyl is optionally substituted with one to three R₄; and wherein the aryl, cycloalkyl, and heterocycloalkyl are optionally substituted with one to three R₅. In another embodiment, R₂ is (C₁-C₄)alkyl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one to three R₄; and wherein the heteroaryl, cycloalkyl, and heterocycloalkyl are optionally substituted with one to three R₅. In another embodiment, R₂ is (C₁-C₄)alkyl, (C₆-C₁₀)aryl, 5- or 6-membered heteroaryl comprising 1 to 3 heteroatoms selected from O, N, and S, or (C₃-C₈)cycloalkyl, wherein the alkyl is optionally substituted with one to three R₄; and wherein the aryl, heteroaryl, and cycloalkyl, are optionally substituted with one to three R₅. In another embodiment, R₂ is (C₁-C₄)alkyl, (C₆-C₁₀)aryl, 5- or 6-membered heteroaryl comprising 1 to 3 heteroatoms selected from O, N, and S, or 5- to 7-membered heterocycloalkyl comprising 1 to 3 heteroatoms selected from O, N, and S, wherein the alkyl is optionally substituted with one to three R₄; and wherein the aryl, heteroaryl, and heterocycloalkyl are optionally substituted with one to three R₅.

In another embodiment, R₂ is (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the aryl, heteroaryl, cycloalkyl, and heterocycloalkyl are optionally substituted with one to three R₅. In another embodiment, R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, R₂ is phenyl, (C₃-C₈)cycloalkyl, or 5- to 7-membered heterocycloalkyl comprising 1 to 3 heteroatoms selected from O, N, and S, wherein the phenyl, cycloalkyl, and heterocycloalkyl are optionally substituted with one to three R₅. In another embodiment, R₂ is (C₁-C₃)alkyl optionally substituted with one to three R₄. In yet another embodiment, R₂ is (C₁-C₃)alkyl substituted with one to three R₄.

In another embodiment, R₂ is (C₃-C₈)cycloalkyl or 5- to 7-membered heterocycloalkyl comprising 1 to 3 heteroatoms selected from O, N, and S, wherein the cycloalkyl and heterocycloalkyl are optionally substituted with one to three R₅. In yet another embodiment, R₂ is (C₆-C₁₀)aryl or 5- or 6-membered heteroaryl comprising 1 to 3 heteroatoms selected from O, N, and S, wherein the aryl and heteroaryl are optionally substituted with one to three R₅. In another embodiment, R₂ is (C₃-C₈)cycloalkyl or (C₆-C₁₀)aryl, wherein the cycloalkyl and aryl are optionally substituted with one to three R₅. In yet another embodiment, R₂ is 5- or 6-membered heteroaryl comprising 1 to 3 heteroatoms selected from O, N, and S, or 5- to 7-membered heterocycloalkyl comprising 1 to 3 heteroatoms selected from O, N, and S, wherein the heteroaryl and heterocycloalkyl are optionally substituted with one to three R₅. In another embodiment, R₂ is (C₆-C₁₀)aryl optionally substituted with one to three R₅. In yet another embodiment, R₂ is 5- or 6-membered heteroaryl comprising 1 to 3 heteroatoms selected from O, N, and S, optionally substituted with one to three R₅. In another embodiment, R₂ is (C₃-C₈)cycloalkyl optionally substituted with one to three R₅. In yet another embodiment, R₂ is 5- to 7-membered heterocycloalkyl comprising 1 to 3 heteroatoms selected from O, N, and S, optionally substituted with one to three R₅.

In some embodiments of the formulae above, R₁ and R₂, when on adjacent atoms, together with the atoms to which they are attached form a 5-membered heterocycloalkyl ring. In another embodiment, R₁ and R₂, when on adjacent atoms, together with the atoms to which they are attached form a 6-membered heterocycloalkyl ring.

In some embodiments of the formulae above, R₃ is D. In another embodiment, R₃ is H. In another embodiment, R₃ is absent when *-̅-̅-̅-̅-̅-̅* is a double bond.

In some embodiments of the formulae above, each R₄ is independently selected from -C(O)OR₆, - C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from -C(O)OR₆, -C(O)NR₆R_{6'}, - NR₆C(O)R_{6'}, halogen, -OH, -NH₂, or CN. In another embodiment, each R₄ is independently selected from -C(O)OR₆, -C(O)NR₆R_{6'}, -NR₆C(O)R_{6'}, halogen, or -OH. In another embodiment, each R₄ is independently selected from halogen, -OH, (C₆-C₁₀)aryl, 5- or 6-membered heteroaryl comprising 1 to 4 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl ring comprising 1 to 3 heteroatoms selected from O, N, and S, wherein the aryl, heteroaryl, cycloalkyl, and heterocycloalkyl groups are optionally substituted with one to four R₇.

In another embodiment, each R₄ is independently selected from -C(O)OR₆, -C(O)NR₆R_{6'}, and - NR₆C(O)R_{6'}. In another embodiment, each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to four R₇. In yet another embodiment, each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to four R₇. In another embodiment, each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In another embodiment, each R₄ is independently selected from (C₆-C₁₀)aryl and 5- or 6-membered heteroaryl comprising 1 to 3 heteroatoms selected from O, N, and S, wherein the aryl and heteroaryl are optionally substituted with one to three R₇. In yet another embodiment, each R₄ is independently selected from (C₆-C₁₀)aryl and 5- or 6-membered heteroaryl comprising 1 to 3 heteroatoms selected from O, N, and S, wherein the aryl and heteroaryl are substituted with one to three R₇.

In another embodiment, each R₄ is independently selected from (C₃-C₈)cycloalkyl and 5- to 7-membered heterocycloalkyl comprising 1 to 3 heteroatoms selected from O, N, and S, wherein the cycloalkyl and heterocycloalkyl groups are optionally substituted with one to three R₇. In another embodiment, each R₄ is independently selected from (C₃-C₈)cycloalkyl and 5- to 7-membered heterocycloalkyl comprising 1 to 3 heteroatoms selected from O, N, and S, wherein the cycloalkyl and heterocycloalkyl groups are substituted with one to three R₇.

In another embodiment, each R₄ is independently (C₆-C₁₀)aryl optionally substituted with one to three R₇. In yet another embodiment, each R₄ is independently 5- or 6-membered heteroaryl comprising 1 to 3 heteroatoms selected from O, N, and S, optionally substituted with one to three R₇.

In another embodiment, each R₄ is (C₃-C₈)cycloalkyl optionally substituted with one to three R₇. In another embodiment, each R₄ is independently 5- to 7-membered heterocycloalkyl comprising 1 to 3 heteroatoms selected from O, N, and S, optionally substituted with one to three R₇.

In some embodiments of the formulae above, each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S. In another embodiment, each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C₁-C₆)hydroxyalkyl, halogen, -OH, -NH₂, and CN. In yet another embodiment, each R₅ is independently selected from (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S.

In another embodiment, each R₅ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S.

In another embodiment, each R₅ is independently selected from (C₁-C₆)alkyl, (C₁-C₆)alkoxy, (C₁-C₆)haloalkyl, and (C₁-C₆)haloalkoxy. In yet another embodiment, each R₅ is independently selected from (C₁-C₆)hydroxyalkyl, halogen, -OH, -NH₂, and CN. In another embodiment, each R₅ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, and CN.

In some embodiments of the formulae above, two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one to four R₁₀. In another embodiment, two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to three R₁₀, or two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one three R₁₀.

In another embodiment, two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to three R₁₀. In yet another embodiment, two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one three R₁₀.

In another embodiment, two R₅, when on adjacent atoms, together with the atoms to which they are attached form a (C₆-C₁₀)aryl ring optionally substituted with one to three R₁₀. In another embodiment, two R₅, when on adjacent atoms, together with the atoms to which they are attached form a phenyl ring optionally substituted with one to three R₁₀. In yet another embodiment, two R₅, when on adjacent atoms, together with the atoms to which they are attached form a 5- or 6-membered heteroaryl ring comprising 1 to 3 heteroatoms selected from O, N, and S, optionally substituted with one to three R₁₀.

In another embodiment, two R₅, when on adjacent atoms, together with the atoms to which they are attached form a (C₅-C₇)cycloalkyl ring optionally substituted with one three R₁₀. In another embodiment, two R₅, when on adjacent atoms, together with the atoms to which they are attached form a (C₆-C₇)cycloalkyl ring optionally substituted with one three R₁₀. In another embodiment, two R₅, when on adjacent atoms, together with the atoms to which they are attached form a (C₅-C₆)cycloalkyl ring optionally substituted with one three R₁₀. In another embodiment, two R₅, when on adjacent atoms, together with the atoms to which they are attached form a (C₅)cycloalkyl ring optionally substituted with one three R₁₀. In another embodiment, two R₅, when on adjacent atoms, together with the atoms to which they are attached form a (C₆)cycloalkyl ring optionally substituted with one three R₁₀. In another embodiment, two R₅, when on adjacent atoms, together with the atoms to which they are attached form a (C₇)cycloalkyl ring optionally substituted with one three R₁₀.

In another embodiment, two R₅, when on adjacent atoms, together with the atoms to which they are attached form a 5- to 7-membered heterocycloalkyl ring comprising 1 to 3 heteroatoms selected from O, N, and S optionally substituted with one three R₁₀. In another embodiment, two R₅, when on adjacent atoms, together with the atoms to which they are attached form a 5- or 6-membered heterocycloalkyl ring comprising 1 to 3 heteroatoms selected from O, N, and S optionally substituted with one three R₁₀. In another embodiment, two R₅, when on adjacent atoms, together with the atoms to which they are attached form a 6- or 7-membered heterocycloalkyl ring comprising 1 to 3 heteroatoms selected from O, N, and S optionally substituted with one three R₁₀. In another embodiment, two R₅, when on adjacent atoms, together with the atoms to which they are attached form a 5-membered heterocycloalkyl ring comprising 1 to 3 heteroatoms selected from O, N, and S optionally substituted with one three R₁₀. In another embodiment, two R₅, when on adjacent atoms, together with the atoms to which they are attached form a 6-membered heterocycloalkyl ring comprising 1 to 3 heteroatoms selected from O, N, and S optionally substituted with one three R₁₀. In another embodiment, two R₅, when on adjacent atoms, together with the atoms to which they are attached form a 7-membered heterocycloalkyl ring comprising 1 to 3 heteroatoms selected from O, N, and S optionally substituted with one three R₁₀.

In some embodiments of the formulae above, R₆ is H or (C₁-C₃)alkyl. In another embodiment, R₆ is H or (C₆-C₁₀)aryl. In yet another embodiment, R₆ is (C₁-C₃)alkyl or (C₆-C₁₀)aryl. In another embodiment, R₆ is H, methyl, ethyl, n-propyl, or isopropyl. In another embodiment, R₆ is H, methyl or ethyl. In yet another embodiment, R₆ is H or methyl. In another embodiment, R₆ is H.

In some embodiments of the formulae above, R_{6'} is H or (C₁-C₃)alkyl. In another embodiment, R_{6'} is H or (C₆-C₁₀)aryl. In yet another embodiment, R_{6'} is (C₁-C₃)alkyl or (C₆-C₁₀)aryl. In another embodiment, R_{6'} is H, methyl, ethyl, n-propyl, or isopropyl. In another embodiment, R_{6'} is H, methyl or ethyl. In yet another embodiment, R_{6'} is H or methyl. In another embodiment, R_{6'} is H.

In some embodiments of the formulae above, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)Rs, - (CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy. In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)Rs, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one to four R₁₁, and the aryl, heteroaryl, and heterocycloalkyl are optionally substituted with one to four substituent each independently selected from halogen, (C₁-C₆)alkyl, (C₁-C₆)haloalkyl, and (C₁-C₆)alkoxy.

In another embodiment, each R₇ is independently selected from -(CH₂)₀₋₃C(O)OR₈, -NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, -O(CH₂)₀₋₃-5- or 6-membered heteroaryl comprising 1 to 3 heteroatoms selected from O, N, and S, bicyclic 9- or 10-membered heteroaryl comprising 1 to 3 heteroatoms selected from O, N, and S, wherein the aryl and heteroaryl and heterocycloalkyl are optionally substituted with one to four substituents each independently selected from halogen, (C₁-C₆)alkyl, (C₁-C₆)haloalkyl, and (C₁-C₆)alkoxy.

In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -C(O)OR₈, -C(O)NR₈R₉, - NR₈C(O)R₉, (C₁-C₆)hydroxyalkyl, halogen, -OH, -NH₂, CN, (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S. In another embodiment, each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, and CN.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, and CN. In yet another embodiment, each R₇ is independently selected from (C₁-C₆)alkyl, (C₁-C₆)alkoxy, (C₁-C₆)haloalkyl, (C₁-C₆)haloalkoxy. In another embodiment, each R₇ is independently selected from -C(O)Rs, -C(O)OR₈, -C(O)NR₈R₉, -NR₈C(O)R₉, (C₁-C₆)hydroxyalkyl, halogen, -OH, -NH₂, and CN. In another embodiment, each R₇ is independently selected from (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S.

In another embodiment, each R₇ is independently selected from (C₁-C₆)alkyl, (C₁-C₆)alkoxy, (C₁-C₆)haloalkyl, (C₁-C₆)haloalkoxy, -C(O)R₈, -C(O)OR₈, -C(O)NR₈R₉, -NR₈C(O)R₉, (C₁-C₆)hydroxyalkyl, halogen, -OH, -NH₂, CN, (C₆-C₁₀)aryl, 5- or 6-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S. In yet another embodiment, each R₇ is independently selected from (C₁-C₆)alkyl, (C₁-C₆)alkoxy, halogen, -OH, CN, and (C₆-C₁₀)aryl.

In some embodiments of the formulae above, two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀. In another embodiment, two R₇, when on adjacent atoms, together with the atoms to which they are attached form a (C₆-C₁₀)aryl ring optionally substituted with one to four R₁₀. In another embodiment, two R₇, when on adjacent atoms, together with the atoms to which they are attached form a 5- or 6-membered heteroaryl ring comprising 1 to 3 heteroatoms selected from O, N, and S, optionally substituted with one to four R₁₀. In another embodiment, two R₇ together with the atoms to which they are attached form a (C₅-C₇) cycloalkyl ring optionally substituted with one to four R₁₀. In another embodiment, two R₇ together with the atoms to which they are attached form a 5- to 7-membered heterocycloalkyl ring comprising 1 to 3 heteroatoms selected from O, N, and S, optionally substituted with one to four R₁₀.

In another embodiment, two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one to four R₁₀, or two R₇, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S, optionally substituted with one to four R₁₀.

In another embodiment, two R₇, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S, optionally substituted with one to four R₁₀. In another embodiment, two R₇, when on adjacent atoms, together with the atoms to which they are attached form a (C₅-C₇)cycloalkyl ring optionally substituted with one to four R₁₀.In another embodiment, two R₇, when on adjacent atoms, together with the atoms to which they are attached form a 5- to 7-membered heterocycloalkyl ring comprising 1 to 3 heteroatoms selected from O, N, and S, optionally substituted with one to four R₁₀.

In some embodiments of the formulae above, R₈ is H or (C₁-C₃)alkyl. In another embodiment, R₈ is H, methyl, ethyl, *n*-propyl, or isopropyl. In another embodiment, R₈ is H, methyl or ethyl. In yet another embodiment, R₈ is H or methyl. In another embodiment, R₈ is H

In some embodiments of the formulae above, R₉ is H or (C₁-C₃)alkyl. In another embodiment, R₉ is H, methyl, ethyl, *n*-propyl, or isopropyl. In another embodiment, R₉ is H, methyl or ethyl. In yet another embodiment, R₉ is H or methyl. In another embodiment, R₉ is H.

In some embodiments of the formulae above, each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, and halogen. In another embodiment, each R₁₀ is independently selected from -OH, -NH₂, and CN. In yet another embodiment, each R₁₀ is independently selected from (C₁-C₆)alkyl, (C₁-C₆)alkoxy, (C₁-C₆)haloalkyl, (C₁-C₆)haloalkoxy, and halogen. In another embodiment, each R₁₀ is independently selected from (C₁-C₆)alkyl, (C₁-C₆)haloalkyl, and halogen. In yet another embodiment, each R₁₀ is independently selected from (C₁-C₆)alkyl and halogen.

In some embodiments of the formulae above, two R₁₀ together with the carbon atom to which they are attached form a =(O).

In some embodiments of the formulae above, each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN. In another embodiment, each R₁₁ is independently selected from CN, (C₁-C₆)alkoxy, (C₆-C₁₀)aryl, and 5-to 7-membered heterocycloalkyl comprising 1 to 3 heteroatoms selected from O, N, and S, wherein the aryl and heterocycloalkyl are optionally substituted with one to three substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN. In yet another embodiment, each R₁₁ is independently selected from CN, (C₁-C₆)alkoxy, and (C₆-C₁₀)aryl, wherein the aryl is optionally substituted with one to three substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN.

In another embodiment, each R₁₁ is independently selected from CN, (C₁-C₆)alkoxy, and 5- to 7-membered heterocycloalkyl comprising 1 to 3 heteroatoms selected from O, N, and S, wherein the heterocycloalkyl is optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN. In another embodiment, each R₁₁ is independently selected from CN and (C₁-C₆)alkoxy. In yet another embodiment, each R₁₁ is independently selected from (C₆-C₁₀)aryl and 5- to 7-membered heterocycloalkyl comprising 1 to 3 heteroatoms selected from O, N, and S, wherein the aryl and heterocycloalkyl are optionally substituted with one to four substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, - NH₂, and CN.

In some embodiments of the formulae above, R₁₂ is (C₁-C₆)alkyl, (C₁-C₆)haloalkyl, (C₆-C₁₀)aryl, or 5- or 6-membered heterocycloalkyl comprising 1 to 3 heteroatoms selected from O, N, and S. In another embodiment, R₁₂ is (C₁-C₆)alkyl, (C₁-C₆)haloalkyl, phenyl, or 5- or 6-membered heterocycloalkyl comprising 1 to 3 heteroatoms selected from O, N, and S. In another embodiment, R₁₂ is (C₁-C₄)alkyl, (C₁-C₄)haloalkyl, phenyl, or 5- or 6-membered heterocycloalkyl comprising 1 to 3 heteroatoms selected from O, N, and S.

In some embodiments of the formulae above, R₁₃ is halogen, -OH, or -NH₂. In another embodiment, R₁₃ is H, halogen, or -NH₂. In another embodiment, R₁₃ is H, F, Cl, or -NH₂. In another embodiment, R₁₃ is H, F, Cl, -OH, or-NH₂. In another embodiment, R₁₃ is H, F, or -NH₂. In another embodiment, R₁₃ is F or -NH₂.

In some embodiments of the formulae above, R₁₄ is (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN. In another embodiment, R₁₄ is H, (C₁-C₃)alkyl, (C₁-C₃)alkoxy, (C₁-C₃)haloalkyl, (C₁-C₃)haloalkoxy, (C₁-C₃)hydroxyalkyl, F, Cl, -OH, -NH₂, -NO₂, or CN. In yet another embodiment, R₁₄ is H, (C₁-C₃)alkyl, (C₁-C₃)alkoxy, (C₁-C₃)haloalkyl, (C₁-C₃)hydroxyalkyl, halogen, -OH, -NH₂, -NO₂, or CN. In another embodiment, R₁₄ is H, (C₁-C₃)alkyl, (C₁-C₃)alkoxy, (C₁-C₃)haloalkyl, (C₁-C₃)hydroxyalkyl, F, Cl, -OH,-NH₂, -NO₂, or CN. In another embodiment, R₁₄ is (C₁-C₃)alkyl, (C₁-C₃)alkoxy, (C₁-C₃)haloalkyl, halogen, -OH, -NH₂, -NO₂, or CN. In yet another embodiment, R₁₄ is (C₁-C₃)alkyl, (C₁-C₃)alkoxy, (C₁-C₃)haloalkyl, F, Cl, -OH, -NH₂, -NO₂, or CN. In another embodiment, R₁₄ is H, (C₁-C₃)alkyl, (C₁-C₃)alkoxy, (C₁-C₃)haloalkyl, halogen, -OH, -NH₂, -NO₂, or CN. In yet another embodiment, R₁₄ is H, (C₁-C₃)alkyl, (C₁-C₃)alkoxy, (C₁-C₃)haloalkyl, F, Cl, -OH, -NH₂, -NO₂, or CN.

In some embodiments of the formulae above, R₁₅ is halogen, -OH, or -NH₂. In another embodiment, R₁₅ is F, Cl, or -NH₂. In another embodiment, R₁₅ is F, Cl, -OH, or -NH₂. In another embodiment, R₁₅ is F or -NH₂.

In some embodiments of the formulae above, R₁₆ is (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN. In another embodiment, R₁₆ is (C₁-C₃)alkyl, (C₁-C₃)alkoxy, (C₁-C₃)haloalkyl, (C₁-C₃)hydroxyalkyl, halogen, -OH, - NH₂, -NO₂, or CN. In another embodiment, R₁₆ is H, (C₁-C₃)alkyl, (C₁-C₃)alkoxy, (C₁-C₃)haloalkyl, (C₁-C₃)hydroxyalkyl, F, Cl, -OH, -NH₂, -NO₂, or CN. In another embodiment, R₁₆ is (C₁-C₃)alkyl, (C₁-C₃)alkoxy, (C₁-C₃)haloalkyl, halogen, -OH, -NH₂, -NO₂, or CN. In yet another embodiment, R₁₆ is (C₁-C₃)alkyl, (C₁-C₃)alkoxy, (C₁-C₃)haloalkyl, F, Cl, -OH, -NH₂, -NO₂, or CN.

In some embodiments of the formulae above, p is 0 or 1. In another embodiment, p is 1 or 2. In yet another embodiment, p is 0 or 2. In another embodiment, p is 0. In yet another embodiment, p is 1. In another embodiment, p is 2.

In some embodiments of the formulae above, n is 0 or 1. In another embodiment, n is 1 or 2. In yet another embodiment, n is 0 or 2. In another embodiment, n is 0. In yet another embodiment, n is 1. In another embodiment, n is 2.

In some embodiments of the formulae above, n + n1 ≤ 3.

In some embodiments of the formulae above, n1 is 1. In another embodiment, n1 is 2.

In some embodiments of the formulae above, n is 0 and n1 is 1. In another embodiment, n is 1 and n1 is 2. In another embodiment, n is 2 and n1 is 1. In another embodiment, n is 1 and n1 is 1.

In some embodiments of the formulae above, q is 0, 1, 2, or 3. In another embodiment, q is 1, 2, 3, or 4. In yet another embodiment, q is 0, 1, or 2. In another embodiment, q is 1, 2, or 3. In yet another embodiment, q is 2, 3, or 4. In another embodiment, q is 0 or 1. In yet another embodiment, q is 1 or 2. In another embodiment, q is 2 or 3. In yet another embodiment, q is 3 or 4. In another embodiment, q is 0. In yet another embodiment, q is 1. In another embodiment, q is 2. In yet another embodiment, q is 3. In another embodiment, q is 4.

In some embodiments of the formulae above, X₁ is CH and n is 1. In another embodiment, X₁ is CH, n is 1, and q is 0.

In some embodiments of the formulae above, X₁ is CH, X₂ is N, and n is 1. In another embodiment, X₁ is CH, X₂ is N, n is 1, and q is 0.

In some embodiments of the formulae above, X₁ is CH, X₃ is N, and n is 1. In another embodiment, X₁ is CH, X₃ is N, n is 1, and q is 0.

In some embodiments of the formulae above, X₁ is CH, X₂ is N, and n is 1. In another embodiment, X₁ is CH, X₂ is N, n is 1, and q is 0, 1, or 2.

In some embodiments of the formulae above, X₁ is CH, X₃ is N, and n is 1. In another embodiment, X₁ is CH, X₃ is N, n is 1, and q is 0, 1, or 2.

In some embodiments of the formulae above, X₁ is CH, n is 1, and q is 0 or 1. In another embodiment, X₁ is CH, n is 1, q is 0 or 1, and R₁ is (C₁-C₆)alkyl. In another embodiment, X₁ is CH, n is 1, q is 0 or 1, R₁ is (C₁-C₆)alkyl, and R₂ is (C₁-C₆)alkyl optionally substituted with one to three R₄. In another embodiment, X₁ is CH, n is 1, q is 0 or 1, R₁ is (C₁-C₆)alkyl, and R₂ is (C₁-C₆)alkyl substituted with one to three R₄.

In another embodiment, X₁ is CH, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₂ is N, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₃ is N, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₂ is CR₁₃, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₃ is CR₁₄, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₂ is CR₁₅, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₃ is CR₁₆, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₂ is CR₁₃, X₃ is CR₁₆, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In another embodiment, X₁ is CH, X₂ is CR₁₄, X₃ is CR₁₅, n is 1, q is 0, and R₂ is (C₁-C₆)alkyl optionally substituted with one to three R₄. In another embodiment, X₁ is CH, n is 1, q is 0, and R₂ is (C₁-C₆)alkyl substituted with one to three R₄.

In some embodiments of the formulae above, X₁ is CH, n is 1, q is 0 or 1, R₁ is (C₁-C₆)alkyl, R₂ is (C₁-C₆)alkyl optionally substituted with one to three R₄, and each R₄ is independently selected from - C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0 or 1, R₁ is (C₁-C₆)alkyl, R₂ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0 or 1, R₁ is (C₁-C₆)alkyl, R₂ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0 or 1, R₁ is (C₁-C₆)alkyl, R₂ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, and R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, X₁ is CH, n is 1, q is 0, and R₂ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, X₁ is CH, n is 1, q is 0, and R₂ is (C₆-C₁₀)aryl optionally substituted with one to three R₅. In another embodiment, X₁ is CH, n is 1, q is 0, and R₂ is 5- or 6-membered heteroaryl comprising 1 to 3 heteroatoms selected from O, N, and S optionally substituted with one to three R₅. In yet another embodiment, X₁ is CH, n is 1, q is 0, and R₂ is (C₃-C₈)cycloalkyl optionally substituted with one to three R₅. In another embodiment, X₁ is CH, n is 1, q is 0, and R₂ is 5- to 7-membered heterocycloalkyl comprising 1 to 3 heteroatoms selected from O, N, and S, optionally substituted with one to three R₅.

In some embodiments of the formulae above, X₁ is CH, n is 1, q is 0 or 1, R₁ is (C₁-C₆)alkyl, and R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, X₁ is CH, n is 1, q is 0 or 1, R₁ is (C₁-C₆)alkyl, and R₂ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, X₁ is CH, n is 1, q is 0 or 1, R₁ is (C₁-C₆)alkyl, and R₂ is (C₆-C₁₀)aryl optionally substituted with one to three R₅. In another embodiment, X₁ is CH, n is 1, q is 0, and R₂ is 5- or 6-membered heteroaryl comprising 1 to 3 heteroatoms selected from O, N, and S optionally substituted with one to three R₅. In yet another embodiment, X₁ is CH, n is 1, q is 0 or 1, R₁ is (C₁-C₆)alkyl, and R₂ is (C₃-C₈)cycloalkyl optionally substituted with one to three R₅. In another embodiment, X₁ is CH, n is 1, q is 0 or 1, R₁ is (C₁-C₆)alkyl, and R₂ is 5- to 7-membered heterocycloalkyl comprising 1 to 3 heteroatoms selected from O, N, and S, optionally substituted with one to three R₅.

In some embodiments of the formulae above, X₁ is CH, n is 1, q is 0, and R₂ is (C₁-C₆)alkyl optionally substituted with one to three R₄. In another embodiment X₁ is CH, n is 1, q is 0, and R₂ is (C₁-C₆)alkyl substituted with one to three R₄.

In some embodiments of the formulae above, X₁ is CH, n is 1, q is 0, R₂ is (C₁-C₆)alkyl optionally substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl optionally substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl substituted with one to three R₄, and each R₄ is independently selected from halogen, -OH, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl optionally substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl substituted with one to three R₄, and each R₄ is independently selected from halogen, -OH, phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl optionally substituted with one to three R₄, and each R₄ is independently selected from phenyl, 5- or 6-membered heteroaryl comprising 1 to 3 heteroatoms selected from O, N, and S, (C₃-C₈)cycloalkyl, and 5-to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, n1 is 1, q is 0, R₂ is (C₁-C₆)alkyl substituted with one to three R₄, and each R₄ is independently selected from phenyl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 1, q is 0, R₂ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the aryl and heteroaryl groups are optionally substituted with one to three R₇.

In some embodiments of the formulae above, X₁ is CH, n is 1, q is 0, R₂ is (C₁-C₆)alkyl substituted with one to three R₄, and each R₄ is independently selected from phenyl and 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl and heteroaryl groups are optionally substituted with one to three R₇.

In some embodiments of the formulae above, X₁ is CH, n is 1, n1 is 1, q is 0, R₂ is (C₁-C₆)alkyl optionally substituted with one to three R₄, and each R₄ is independently selected from phenyl and 5- or 6-membered heteroaryl comprising 1 to 3 heteroatoms selected from O, N, and S, wherein the aryl and heteroaryl groups are optionally substituted with one to three R₇.

In some embodiments of the formulae above, X₁ is CH, n is 1, n1 is 1, q is 0, R₂ is (C₁-C₆)alkyl substituted with one to three R₄, and each R₄ is independently selected from phenyl and 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl and heteroaryl groups are optionally substituted with one to three R₇.

In some embodiments of the formulae above, X₁ is CH, n is 1, q is 0, R₂ is (C₁-C₆)alkyl optionally substituted with one to three R₄, and each R₄ is phenyl optionally substituted with one to three R₇.

In some embodiments of the formulae above, X₁ is CH, n is 1, q is 0, R₂ is (C₁-C₆)alkyl substituted with one to three R₄, and each R₄ is phenyl optionally substituted with one to three R₇.

In some embodiments of the formulae above, X₁ is CH, n is 1, n1 is 1, q is 0, R₂ is (C₁-C₆)alkyl optionally substituted with one to three R₄, and each R₄ is phenyl optionally substituted with one to three R7.

In some embodiments of the formulae above, X₁ is CH, n is 1, n1 is 1, q is 0, R₂ is (C₁-C₆)alkyl substituted with one to three R₄, and each R₄ is phenyl optionally substituted with one to three R₇.

In some embodiments of the formulae above, X₁ is CH and n is 2. In another embodiment, X₁ is CH, n is 2, and q is 0. In yet another embodiment, X₁ is CH, n is 2, and q is 0 or 1. In another embodiment, X₁ is CH, n is 2, q is 0 or 1, and R₁ is (C₁-C₆)alkyl.

In some embodiments of the formulae above, X₁ is CH, n is 2, q is 0 or 1, R₁ is (C₁-C₆)alkyl, and R₂ is (C₁-C₆)alkyl optionally substituted with one to three R₄. In another embodiment, X₁ is CH, n is 2, q is 0 or 1, R₁ is (C₁-C₆)alkyl, and R₂ is (C₁-C₆)alkyl substituted with one to three R₄.

In some embodiments of the formulae above, X₁ is CH, n is 2, q is 0, and R₂ is (C₁-C₆)alkyl optionally substituted with one to three R₄. In another embodiment, X₁ is CH, n is 2, q is 0, and R₂ is (C₁-C₆)alkyl substituted with one to three R₄.

In some embodiments of the formulae above, X₁ is CH, n is 2, q is 0 or 1, R₁ is (C₁-C₆)alkyl, R₂ is (C₁-C₆)alkyl optionally substituted with one to three R₄, and each R₄ is independently selected from - C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 2, q is 0 or 1, R₁ is (C₁-C₆)alkyl, R₂ is (C₁-C₆)alkyl substituted with one to three R₄, and each R₄ is independently selected from -C(O)OR₆, (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 2, q is 0 or 1, R₁ is (C₁-C₆)alkyl, R₂ is (C₁-C₆)alkyl optionally substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 2, q is 0 or 1, R₁ is (C₁-C₆)alkyl, R₂ is (C₁-C₆)alkyl substituted with one to three R₄, and each R₄ is independently selected from (C₆-C₁₀)aryl, 5- or 6-membered heteroaryl comprising 1 to 3 heteroatoms selected from O, N, and S, (C₃-C₈)cycloalkyl, and 5- to 7-membered heterocycloalkyl comprising 1 to 3 heteroatoms selected from O, N, and S, wherein the aryl, heteroaryl, cycloalkyl, and heterocycloalkyl groups are optionally substituted with one to three R₇.

In some embodiments of the formulae above, X₁ is CH, n is 2, q is 0, and R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, X₁ is CH, n is 2, q is 0, and R₂ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, X₁ is CH, n is 2, q is 0, and R₂ is (C₆-C₁₀)aryl optionally substituted with one to three R₅. In another embodiment, X₁ is CH, n is 2, q is 0, and R₂ is 5- or 6-membered heteroaryl comprising 1 to 3 heteroatoms selected from O, N, and S optionally substituted with one to three R₅. In yet another embodiment, X₁ is CH, n is 2, q is 0, and R₂ is (C₃-C₈)cycloalkyl optionally substituted with one to three R₅. In another embodiment, X₁ is CH, n is 2, q is 0, and R₂ is 5- to 7-membered heterocycloalkyl comprising 1 to 3 heteroatoms selected from O, N, and S, optionally substituted with one to three R₅.

In some embodiments of the formulae above, X₁ is CH, n is 2, q is 0 or 1, R₁ is (C₁-C₆)alkyl, and R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, cycloalkyl, and heterocycloalkyl are optionally substituted with one to three R₅. In yet another embodiment, X₁ is CH, n is 2, q is 0 or 1, R₁ is (C₁-C₆)alkyl, and R₂ is (C₆-C₁₀)aryl, (C₃-C₈)cycloalkyl, or 5- to 7-membered heterocycloalkyl comprising 1 to 3 heteroatoms selected from O, N, and S.

In some embodiments of the formulae above, X₁ is CH, n is 2, q is 0 or 1, R₁ is (C₁-C₆)alkyl, and R₂ is (C₆-C₁₀)aryl optionally substituted with one to three R₅. In another embodiment, X₁ is CH, n is 2, q is 0, and R₂ is 5- or 6-membered heteroaryl comprising 1 to 3 heteroatoms selected from O, N, and S optionally substituted with one to three R₅. In yet another embodiment, X₁ is CH, n is 2, q is 0 or 1, R₁ is (C₁-C₆)alkyl, and R₂ is (C₃-C₈)cycloalkyl optionally substituted with one to three R₅. In another embodiment, X₁ is CH, n is 2, q is 0 or 1, R₁ is (C₁-C₆)alkyl, and R₂ is 5- to 7-membered heterocycloalkyl comprising 1 to 3 heteroatoms selected from O, N, and S, optionally substituted with one to three R₅.

In some embodiments of the formulae above, X₁ is CH, n is 1, n1 is 1, and R₂ is (C₁-C₆)alkyl optionally substituted with one to three R₄. In another embodiment, X₁ is CH, n is 1, n1 is 1, and R₂ is (C₁-C₆)alkyl substituted with one to three R₄. In another embodiment, X₁ is CH, n is 1, n1 is 1, q is 0, and R₂ is (C₁-C₆)alkyl substituted with one to three R₄. In another embodiment, X₁ is CH, n is 1, n1 is 1, q is 0, and R₂ is (C₁-C₆)alkyl substituted with one to three R₄. In another embodiment, X₁ is CH, X₂ is N and X₃ is R₁₄, n is 1, n1 is 1, q is 0, and R₂ is (C₁-C₆)alkyl substituted with one to three R₄.

In some embodiments of the formulae above, X₁ is CH, X₂ is N and X₃ is R₁₄, n is 1, n1 is 1, q is 0, and R₂ is (C₁-C₆)alkyl substituted with one to three R₄. In another embodiment, X₁ is CH, X₂ is CR₁₃ and X₃ is N is R₁₄, n is 1, n1 is 1, q is 0, and R₂ is (C₁-C₆)alkyl substituted with one to three R₄. In another embodiment, X₁ is CH, X₂ is CR₁₃ and X₃ is N, n is 1, n1 is 1, q is 0, and R₂ is (C₁-C₆)alkyl substituted with one to three R₄. In another embodiment, X₁ is CH, X₂ is CR₁₅ and X₃ is CR₁₄, n is 1, n1 is 1, q is 0, and R₂ is (C₁-C₆)alkyl substituted with one to three R₄. In another embodiment, X₁ is CH, X₂ is CR₁₅ and X₃ is CR₁₄, n is 1, n1 is 1, q is 0, and R₂ is (C₁-C₆)alkyl substituted with one to three R₄. In another embodiment, X₁ is CH, X₂ is CR₁₃ and X₃ is CR₁₆, n is 1, n1 is 1, q is 0, and R₂ is (C₁-C₆)alkyl substituted with one to three R₄. In another embodiment, X₁ is CH, X₂ is CR₁₃ and X₃ is CR₁₆, n is 1, n1 is 1, q is 0, and R₂ is (C₁-C₆)alkyl substituted with one to three R₄.

In some embodiments of the formulae above, 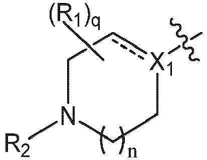 is

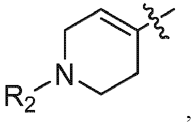

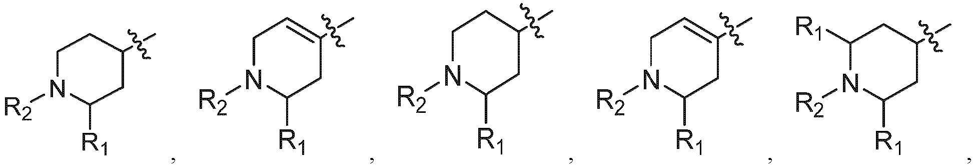

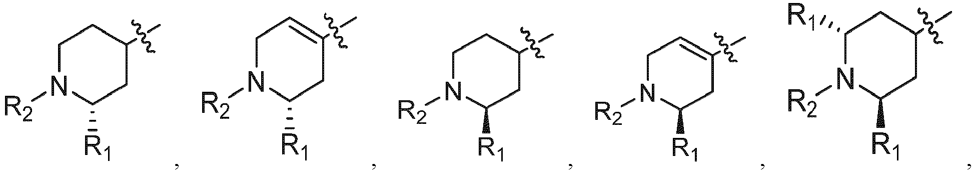

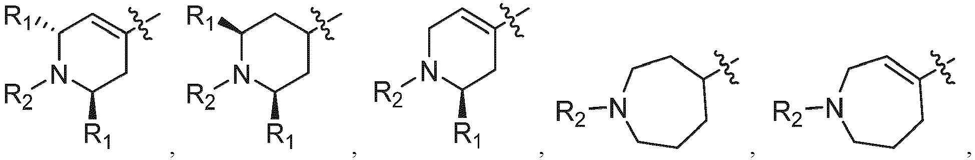

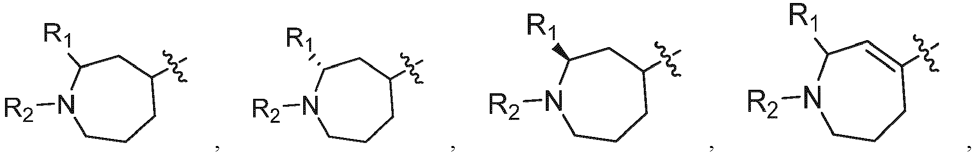

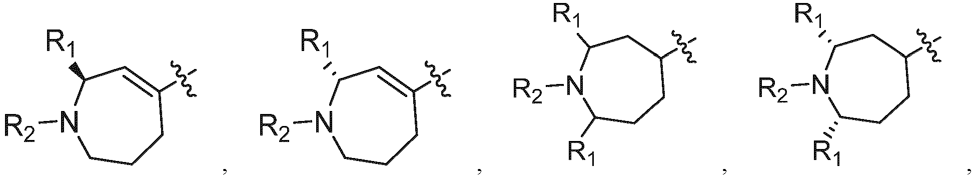

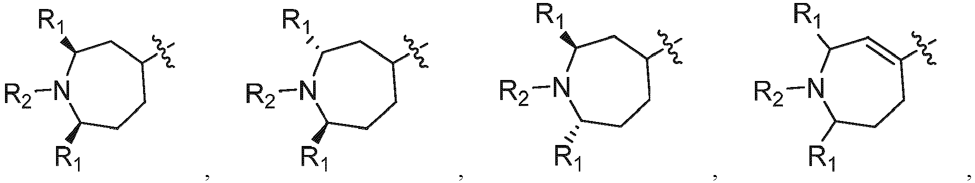

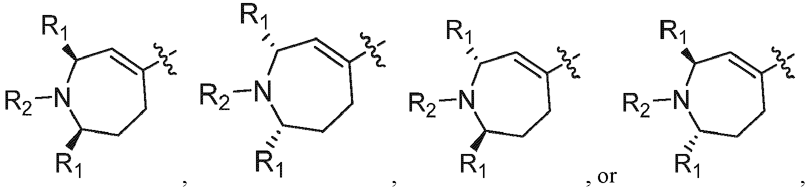

In some embodiments of the formulae above, 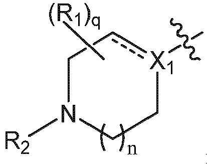 is

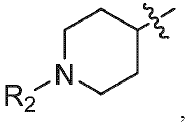

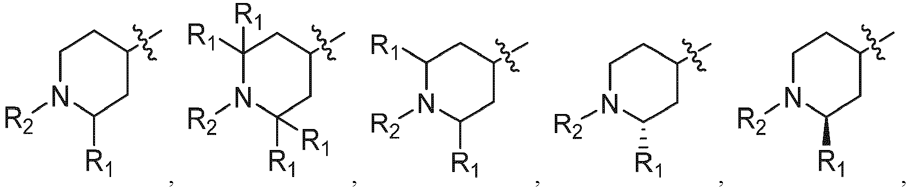

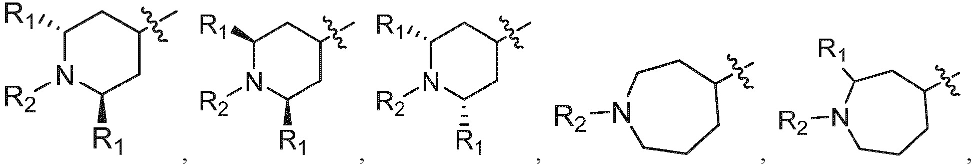

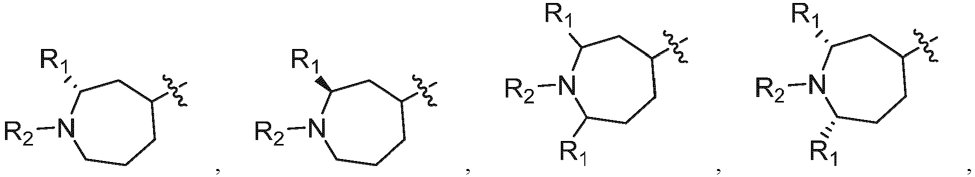

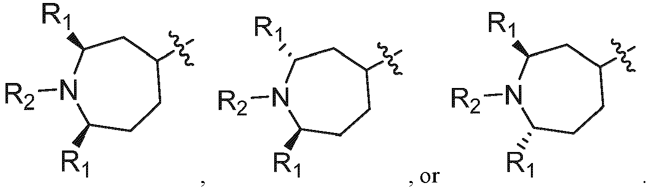

In some embodiments of the formulae above, 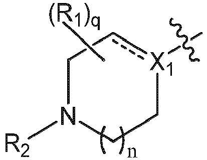 is

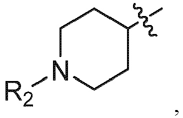

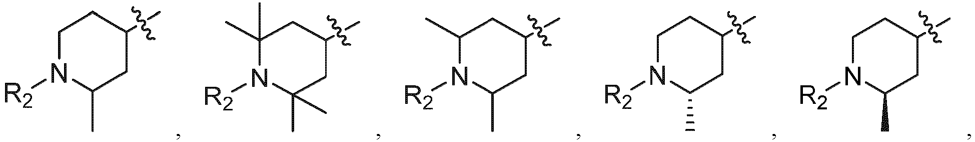

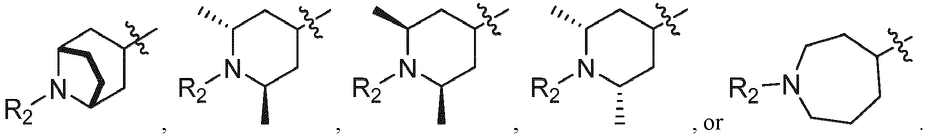

In another embodiment of the invention, the compounds of the present invention are enantiomers. In some embodiments the compounds are the (S)-enantiomer. In other embodiments the compounds are the (R)-enantiomer. In yet other embodiments, the compounds of the present invention may be (+) or (-) enantiomers.

It should be understood that all isomeric forms are included within the present invention, including mixtures thereof. If the compound contains a double bond, the substituent may be in the E or Z configuration. If the compound contains a disubstituted cycloalkyl, the cycloalkyl substituent may have a cis- or trans configuration. All tautomeric forms are also intended to be included.

Compounds of the invention, and pharmaceutically acceptable salts, hydrates, solvates, and stereoisomers thereof may exist in their tautomeric form (for example, as an amide or imino ether). All such tautomeric forms are contemplated herein as part of the present invention.

The compounds of the invention may contain asymmetric or chiral centers and, therefore, exist in different stereoisomeric forms. It is intended that all stereoisomeric forms of the compounds of the invention as well as mixtures thereof, including racemic mixtures, form part of the present invention. In addition, the present invention embraces all geometric and positional isomers. For example, if a compound of the invention incorporates a double bond or a fused ring, both the cis- and trans-forms, as well as mixtures, are embraced within the scope of the invention. Each compound herein disclosed includes all the enantiomers that conform to the general structure of the compound. The compounds may be in a racemic or enantiomerically pure form, or any other form in terms of stereochemistry. The assay results may reflect the data collected for the racemic form, the enantiomerically pure form, or any other form in terms of stereochemistry.

Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of the invention may be atropisomers (e.g., substituted biaryls) and are considered as part of this invention. Enantiomers can also be separated by use of a chiral HPLC column.

It is also possible that the compounds of the invention may exist in different tautomeric forms, and all such forms are embraced within the scope of the invention and chemical structures and names. Also, for example, all keto-enol and imine-enamine forms of the compounds are included in the invention.

All stereoisomers (for example, geometric isomers, optical isomers, and the like) of the present compounds (including those of the salts, and solvates of the compounds), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). (For example, if a compound of Formula (I) incorporates a double bond or a fused ring, both the cis- and trans-forms, as well as mixtures, are embraced within the scope of the invention. Also, for example, all keto-enol and imine-enamine forms of the compounds are included in the invention.) Individual stereoisomers of the compounds of the invention may, for example, be substantially free of other isomers, or is admixed, for example, as racemates or with all other, or other selected, stereoisomers.

The chiral centers of the compounds of the invention can have the S or R configuration as defined by the IUPAC 1974 Recommendations. In certain embodiments, each asymmetric atom has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess in the (R)- or (S)- configuration. Substituents at atoms with unsaturated double bonds may, if possible, be present in cis-(Z)- or trans-(E)- form.

The use of the terms "salt", "solvate", and the like, is intended to equally apply to the salt, and solvate of enantiomers, stereoisomers, rotamers, tautomers, positional isomers, or racemates of the inventive compounds.

The compounds of the invention may form salts which are also within the scope of this invention. Reference to a compound of the Formula herein is generally understood to include reference to salts thereof, unless otherwise indicated.

The compounds and intermediates may be isolated and used as the compound *per se.* Any formula given herein is also intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds of the invention include isotopes of hydrogen, carbon, nitrogen, oxygen, phosphorous, fluorine, and, such as ²H, ³H, ¹¹C, ¹³C, ¹⁴C, ¹⁵N, ¹⁸F, ³¹P, ³²P, respectively. The invention includes various isotopically labeled compounds as defined herein, for example those into which radioactive isotopes, such as ³H, ¹³C, and ¹⁴C, are present. Such isotopically labelled compounds are useful in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F, ¹¹C or labeled compound may be particularly desirable for PET or SPECT studies.

Further, substitution with heavier isotopes, particularly deuterium (i.e., ²H or D) may afford certain therapeutic advantages resulting from greater metabolic stability, for example, increased *in vivo* half-life, reduced dosage requirements, reduced CYP450 inhibition (competitive or time dependent) or an improvement in therapeutic index. For example, substitution with deuterium may modulate undesirable side effects of the undeuterated compound, such as competitive CYP450 inhibition, time dependent CYP450 inactivation, etc. It is understood that deuterium in this context is regarded as a substituent in compounds of the present invention. The concentration of such a heavier isotope, specifically deuterium, may be defined by the isotopic enrichment factor. The term "isotopic enrichment factor" as used herein means the ratio between the isotopic abundance and the natural abundance of a specified isotope. If a substituent in a compound of this invention is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

Isotopically-labeled compounds of the present invention can generally be prepared by conventional techniques known to those skilled in the art or by carrying out the procedures disclosed in the schemes or in the examples and preparations described below using an appropriate isotopically-labeled reagent in place of the non-isotopically labeled reagent.

Pharmaceutically acceptable solvates in accordance with the invention include those wherein the solvent of crystallization may be isotopically substituted, e.g., D₂O, d₆-acetone, d₆-DMSO.

The present invention relates to compounds which are modulators of IKZF2 protein levels. In one embodiment, the compounds of the present invention decrease IKZF2 protein levels. In yet one embodiment, the compounds of the present invention reduce IKZF2 protein levels. In another embodiment, the compounds of the present invention are degraders of IKZF2.

The present invention relates to compounds, which are modulators of IKZF2 and IKZF4 protein levels. In one embodiment, the compounds of the present invention decrease IKZF2 and IKZF4 protein levels. In yet one embodiment, the compounds of the present invention reduce IKZF2 and IKZF4 protein levels. In another embodiment, the compounds of the present invention are degraders of IKZF2.

In some embodiments, the compounds of the invention are selective over other proteins. As used herein "selective modulator", "selective degrader", or "selective compound" means, for example, a compound of the invention, that effectively modulates, decreases, or reduces the levels of a specific protein or degrades a specific protein to a greater extent than any other protein. A "selective modulator", "selective degrader", or "selective compound" can be identified, for example, by comparing the ability of a compound to modulate, decrease, or reduce the levels of or to degrade a specific protein to its ability to modulate, decrease, or reduce the levels of or to degrade other proteins. In some embodiments, the selectivity can be identified by measuring the AC₅₀, EC₅₀, or IC₅₀ of the compounds.

In some embodiments, the compounds of the present invention are selective IKZF2 modulators. As used herein "selective IKZF2 modulator", "selective IKZF2 degrader", or "selective IKZF2 compound" refers to a compound of the invention, for example, that effectively modulates, decrease, or reduces the levels of IKZF2 protein or degrades IKZF2 protein to a greater extent than any other protein, particularly any protein (transcription factor) from the Ikaros protein family (e.g., IKZF1, IKZF3, IKZF4, and IKZF5).

A "selective IKZF2 modulator", "selective IKZF2 degrader", or "selective IKZF2 compound" can be identified, for example, by comparing the ability of a compound to modulate IKZF2 protein levels to its ability to modulate levels of other members of the Ikaros protein family or other proteins. For example, a substance may be assayed for its ability to modulate IKZF2 protein levels, as well as IKZF1, IKZF3, IKZF4, IKZF5, and other proteins. In some embodiments, the selectivity can be identified by measuring the EC₅₀ of the compounds. In some embodiments, the selectivity can be identified by measuring the AC₅₀ of the compounds. In some embodiments, a selective IKZF2 degrader is identified by comparing the ability of a compound to degrade IKZF2 to its ability to degrade other members of the Ikaros protein family or other proteins.

In certain embodiments, the compounds of the invention are IKZF2 degraders that exhibit at least 2-fold, 3-fold, 5-fold, 10-fold, 25-fold, 50-fold or 100-fold selectivity for the degradation of IKZF2 over other proteins (e.g., IKZF1, IKZF3, IKZF4, and IKZF5). In various embodiments, the compounds of the invention exhibit up to 1000-fold selectivity for the degradation of IKZF2 over other proteins.

In certain embodiments, the compounds of the invention exhibit at least 2-fold, 3-fold, 5-fold, 10-fold, 25-fold, 50-fold or 100-fold selectivity for the degradation of IKZF2 over the other members of the Ikaros protein family (e.g., IKZF1, IKZF3, IKZF4, and IKZF5). In various embodiments, the compounds of the invention exhibit up to 1000-fold selectivity for the degradation of IKZF2 over the other members of the Ikaros protein family (e.g., IKZF1, IKZF3, IKZF4, and IKZF5).

In certain embodiments, the compounds of the invention exhibit at least 2-fold, 3-fold, 5-fold, 10-fold, 25-fold, 50-fold or 100-fold selectivity for the degradation of IKZF2 over IKZF1. In various embodiments, the compounds of the invention exhibit up to 1000-fold selectivity for the degradation of IKZF2 over IKZF1.

In certain embodiments, the compounds of the invention exhibit at least 2-fold, 3-fold, 5-fold, 10-fold, 25-fold, 50-fold or 100-fold selectivity for the degradation of IKZF2 over IKZF3. In various embodiments, the compounds of the invention exhibit up to 1000-fold selectivity for the degradation of IKZF2 over IKZF3.

In certain embodiments, the compounds of the invention exhibit at least 2-fold, 3-fold, 5-fold, 10-fold, 25-fold, 50-fold or 100-fold selectivity for the degradation of IKZF2 over IKZF4. In various embodiments, the compounds of the invention exhibit up to 1000-fold selectivity for the degradation of IKZF2 over IKZF4.

In certain embodiments, the compounds of the invention exhibit at least 2-fold, 3-fold, 5-fold, 10-fold, 25-fold, 50-fold or 100-fold selectivity for the degradation of IKZF2 over IKZF5. In various embodiments, the compounds of the invention exhibit up to 1000-fold selectivity for the degradation of IKZF2 over IKZF5.

In certain embodiments, the compounds of the invention exhibit at least 2-fold, 3-fold, 5-fold, 10-fold, 25-fold, 50-fold or 100-fold selectivity for the degradation of IKZF2 and IKZF4 over the other members of the Ikaros protein family (e.g., IKZF1, IKZF3, and IKZF5). In various embodiments, the compounds of the invention exhibit up to 1000-fold selectivity for the degradation of IKZF2 and IKZF4 over the other members of the Ikaros protein family (e.g., IKZF1, IKZF3, and IKZF5).

In certain embodiments, the compounds of the invention exhibit at least 2-fold, 3-fold, 5-fold, 10-fold, 25-fold, 50-fold or 100-fold selectivity for the degradation of IKZF2 and IKZF4 over IKZF1. In various embodiments, the compounds of the invention exhibit up to 1000-fold selectivity for the degradation of IKZF2 and IKZF4 over IKZF1.

In certain embodiments, the compounds of the invention exhibit at least 2-fold, 3-fold, 5-fold, 10-fold, 25-fold, 50-fold or 100-fold selectivity for the degradation of IKZF2 and IKZF4 over IKZF3. In various embodiments, the compounds of the invention exhibit up to 1000-fold selectivity for the degradation of IKZF2 and IKZF4 over IKZF3.

In certain embodiments, the compounds of the invention exhibit at least 2-fold, 3-fold, 5-fold, 10-fold, 25-fold, 50-fold or 100-fold selectivity for the degradation of IKZF2 and IKZF4 over IKZF5. In various embodiments, the compounds of the invention exhibit up to 1000-fold selectivity for the degradation of IKZF2 and IKZF4 over IKZF5.

In some embodiments, the degradation of IKZF2 is measured by AC₅₀.

Potency of can be determined by AC₅₀ value. A compound with a lower AC₅₀ value, as determined under substantially similar degradation conditions, is a more potent degrader relative to a compound with a higher AC₅₀ value. In some embodiments, the substantially similar conditions comprise determining degradation of protein levels in cells expressing the specific protein, or a fragment of any thereof.

The invention is directed to compounds as described herein and pharmaceutically acceptable salts, hydrates, solvates, stereoisomers, or tautomers thereof, and pharmaceutical compositions comprising one or more compounds as described herein, or pharmaceutically acceptable salts, hydrates, solvates, stereoisomers, or tautomers thereof.

### E. Methods of Synthesizing Compounds of Formula (I)

The compounds of the present invention may be made by a variety of methods, including standard chemistry. Suitable synthetic routes are depicted in the Schemes given below.

The compounds of the present invention may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T.W. Greene and P.G.M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of their execution, shall be consistent with the preparation of Compounds of Formula (I).

Those skilled in the art will recognize if a stereocenter exists in the compounds of the present invention. Accordingly, the present invention includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E.L. Eliel, S.H. Wilen, and L.N. Mander (Wiley-Interscience, 1994).

The compounds described herein may be made from commercially available starting materials or synthesized using known organic, inorganic, and/or enzymatic processes.

### Preparation of Compounds

The compounds of the present invention can be prepared in a number of ways well known to those skilled in the art of organic synthesis. By way of example, compounds of the present invention can be synthesized using the methods described below, together with synthetic methods known in the art of synthetic organic chemistry, or variations thereon as appreciated by those skilled in the art. Preferred methods include but are not limited to those methods described below.

Compounds of the present invention can be synthesized by following the steps outlined in General Schemes **I, II** and **III** which comprise different sequences of assembling intermediates **1-a, 1-b, 1-c, 1-d, 1-e, 1-f, 1-g, 2-a, 2-b, 2-c, 2-d, 2-e, 2-f, 3-a, 3-b, 3-c,** and **3-d.** Starting materials are either commercially available or made by known procedures in the reported literature or as illustrated. 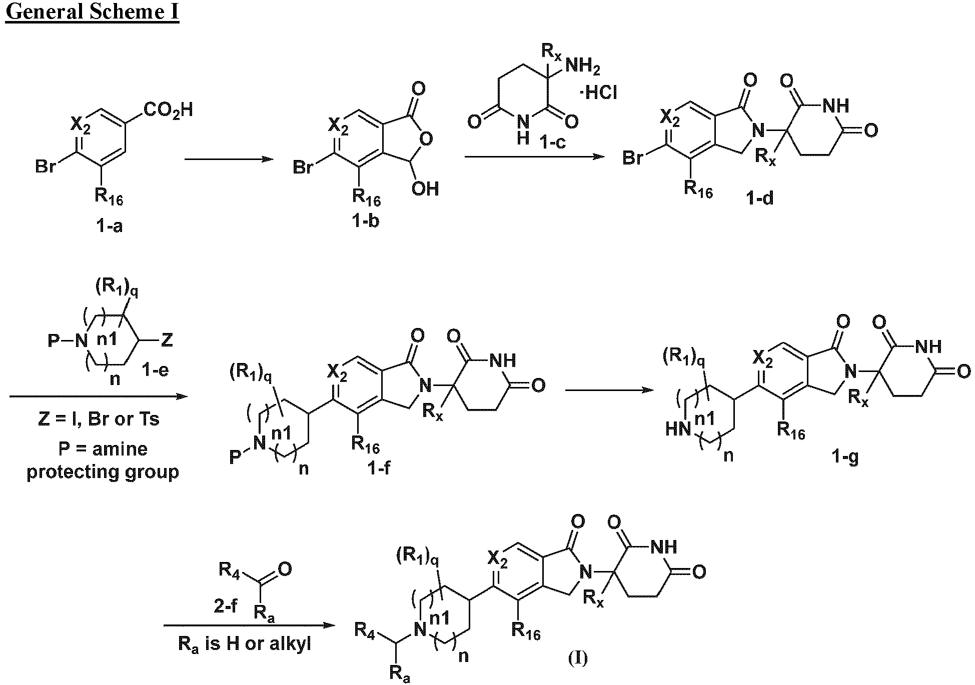 wherein R₁, R₄, R₁₆, Rₓ, X₂, n, n1, and q are as defined in Formula (I).

The general way of preparing Compounds of Formula (I) wherein X₁ is CH, X₃ is CR₁₆, R₂ is a substituted alkyl (optionally substituted with one or more R₄), and -̅ -̅ -̅ -̅ -̅ -̅ is a single bond by using intermediates **1-a, 1-b, 1-c, 1-d, 1-e, 1-f, 1-g** and **2-f** is outlined in General Scheme I. Alkylation of **1-a** with dimethylformamide (DMF) in the presence of a base (e.g., LiTMP, LDA, TMPMgCl•LiCl etc.), in a solvent (e.g., tetrahydrofuran (THF), etc.), and optionally at low temperature provides **1-b.** Reaction of **1-b** and **1-c** in the presence of a reducing agent (e.g., sodium triacetoxyborohydride (NaB(OAc)₃H), sodium cyanoborohydride (NaBH₃CN), etc.) and in a solvent (e.g., DMF) provides **1-d.** Coupling of **1-d** with iodide, bromide or tosylate **1-e** using a catalyst (e.g., NiBr₂•(DME)), ligand (picolinamide hydrochloride salt, 4,4'-di-tert-butyl-2,2'-dipyridyl, pyridine-2,6-bis(carboximidamide) dihydrochloride, 4-methoxypicolinimidamide hydrochloride,etc.), potassium iodide (KI) and manganese or zinc powder in a solvent (e.g., dimethylacetamide (DMA)) optionally at elevated temperature provides **1-f.** Removal of the amine protecting group (e.g., *tert*-butyloxycarbonyl (Boc)) on intermediate **1-f** can be accomplished using a strong acid such as trifluoroacetic acid (TFA) or hydrochloric acid (HCl) in a solvent (e.g., tetrahydrofuran (THF), 1,2-dichloroethane, dioxane or dichloromethane (DCM)) optionally at elevated temperature to provide **I-g**. Reductive amination of **1-g** with aldehyde or ketone **2-f** provides the desired compounds of Formula (I) where X₁ is CH, X₃ is CR₁₆, R₂ is a substituted alkyl, and -̅ -̅ -̅ -̅ -̅ -̅ is a single bond. 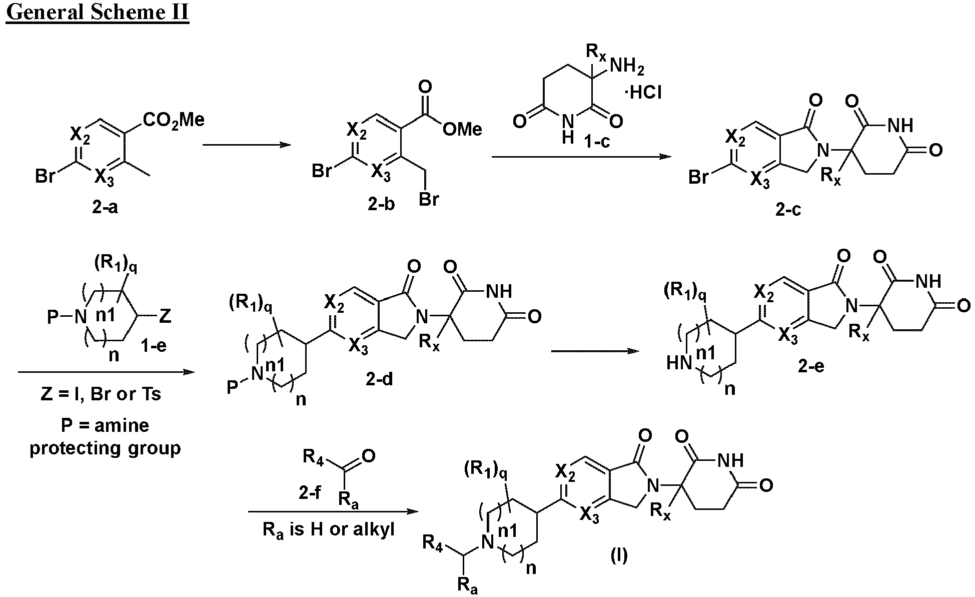 wherein R₁, R₄, Rₓ, X₂, X₃, n, n1, and q are as defined in Formula (I).

The general way of preparing Compounds of Formula (I) wherein X₁ is CH, R₂ is a substituted alkyl (optionally substituted with one or more R₄), and -̅-̅-̅-̅-̅-̅ is a single bond by using intermediates **1-c, 1-e, 2-a, 2-b, 2-c, 2-d, 2-e,** and **2-f** is outlined in General Scheme II. Bromination of **2-a** using a brominating agent (e.g., N-bromosuccinimide (NBS) or Bromine (Br₂)) and radical initiator (e.g., Azobisisobutyronitrile (AIBN)) in a solvent (e.g., 1,2-dichloroethane (DCE)) and optionally at elevated temperature yields **2-b.** Cyclization with 3-aminopiperidine-2,6-dione 1-c or its HCl or CF₃CO₂H salt using a base (e.g., *i*-Pr₂NEt) in a solvent (e.g. DMF) and optionally at elevated temperature provides **2-c.** Coupling of **2-c** with iodide, bromide or tosylate **1-e** using a catalyst (e.g., NiBr₂•(DME)), ligand (picolinamide hydrochloride salt, 4,4'-di-tert-butyl-2,2'-dipyridyl, pyridine-2,6-bis(carboximidamide) dihydrochloride, 4-methoxypicolinimidamide hydrochloride,etc.), potassium iodide (KI) and manganese or zinc powder in a solvent (e.g., dimethylacetamide (DMA)) optionally at elevated temperature provides **2-d.** Removal of the amine protecting group (e.g., *tert*-butyloxycarbonyl (Boc)) on intermediate **2-d** can be accomplished using a strong acid such as trifluoroacetic acid (TFA) or hydrochloric acid (HCl) in a solvent (e.g., tetrahydrofuran (THF), 1,2,-dichloroethane, dioxane or dichloromethane (DCM)) optionally at elevated temperature to provide **2-e.** Reductive amination **of 2-e** with aldehyde or ketone **2-f** provides a compound of Formula (I) where X₁ is CH, R₂ is a substituted alkyl (optionally substituted with one or more R₄), and -̅ -̅ -̅ -̅ -̅ -̅ is a single bond. 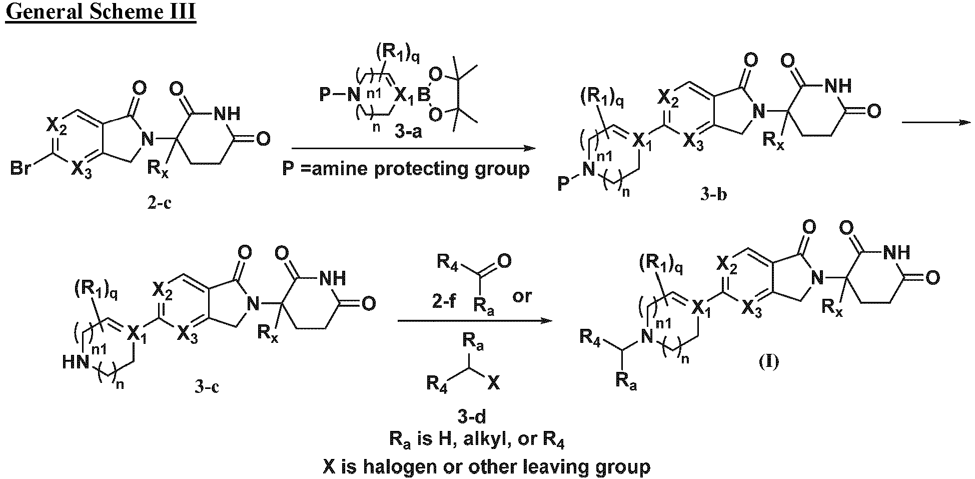 wherein R₁, R₄, Rₓ, X₁, X₂, X₃, n, n1, and q are as defined in Formula (I).

The general way of preparing Compounds of Formula (I) wherein R₂ is a substituted alkyl (optionally substituted with one or more R₄), -̅-̅-̅-̅-̅-̅ is a double bond, X₁ is CR₃, and R₃ is absent, by using intermediates **2-c, 2-f, 3-a, 3-b, 3-c,** and **3-d** is outlined in General Scheme III. Coupling of **2-c** with boronic ester **3-a** using a catalyst (e.g., Pd(dppf)Cl₂•DCM), and a base (e.g., cesium carbonate (Cs₂CO₃)), in a solvent (e.g., *N,N*-dimethylformamide (DMF)) at elevated temperature yields **3-b.** Removal of the amine protecting group (e.g., *tert*-butyloxycarbonyl (Boc)) on intermediate **3-b** can be accomplished using a strong acid such as trifluoroacetic acid (TFA) or hydrochloric acid (HCl) in a solvent (e.g., tetrahydrofuran (THF), 1,2,-dichloroethane, dioxane or dichloromethane (DCM)) optionally at elevated temperature to provide **3-c.** Reductive amination of **3-c** with aldehyde or ketone **2-f** provides a compound of Formula (I) where -̅ -̅ -̅ -̅ -̅ -̅ is a double bond, X₁ is CR₃, R₃ is absent, R₂ is a substituted alkyl. Alternatively, Compounds of Formula (I) where -̅-̅-̅-̅-̅-̅ is a double bond, X₁ is CR₃, R₃ is absent, and R₂ is a substituted alkyl can be obtained by alkylation of **3-c** with an alkyl halide **3-d** in the presence of a base (e.g., NEt₃, Cs₂CO₃, etc.), in a solvent (e.g., DCM, DMF, etc.), and optionally at elevated temperature.

A mixture of enantiomers, diastereomers, and cis/trans isomers resulting from the process described above can be separated into their single components by chiral salt technique, chromatography using normal phase, reverse phase or chiral column, depending on the nature of the separation.

Any resulting racemates of compounds of the present invention or of intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-O,O'-p-toluoyl tartaric acid, mandelic acid, malic acid, or camphor- 10-sulfonic acid. Racemic compounds of the present invention or racemic intermediates can also be resolved by chiral chromatography, e.g., high pressure liquid chromatography (HPLC) using a chiral adsorbent.

Any resulting mixtures of stereoisomers can be separated on the basis of the physicochemical differences of the constituents, into the pure or substantially pure geometric or optical isomers, diastereomers, racemates, for example, by chromatography and/or fractional crystallization.

It should be understood that in the description and formula shown above, the various groups R₁, R₄, R₁₆, Rₓ, X₁, X₂, X₃, n, n1, and q and other variables are as defined above, except where otherwise indicated. Furthermore, for synthetic purposes, the compounds of General Schemes **I, II,** and **III** are merely representative with elected radicals to illustrate the general synthetic methodology of the Compounds of Formula (I) as defined herein.

### F. Methods of Using Compounds of Formula (I)

Another aspect of the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in treating a disease associated with the modulation of, the reduction of, or a decrease in IKZF2 protein levels. In some embodiments, IKZF2 levels are modulated, reduced, or decreased through degradation of the IKZF2 protein. In some embodiments, IKZF2 protein levels are modulated, reduced, or decreased through degradation of the IKZF2 protein mediated by an E3 ligase.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the inhibition of IKZF2 activity through degradation of IKZF2. In some embodiments, IKZF2 protein degradation is mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the manufacture of a medicament for inhibiting IKZF2 activity through degradation of IKZF2. In some embodiments, IKZF2 protein degradation is mediated by an E3 ligase.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the inhibition of IKZF2 and IKZF4 activity through degradation of IKZF2 and IKZF4. In some embodiments, IKZF2 and IKZF4 protein degradation is mediated by an E3 ligase.

Another aspect of the disclosure relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof, for use in treating, preventing, inhibiting, or eliminating a disease or disorder associated with modulation of, reduction of, or a decrease in IKZF4 protein levels. In some embodiments, IKZF4 protein levels are modulated, reduced, or decreased through degradation of the IKZF4 proteins. In some embodiments, IKZF4 protein levels are modulated, reduced, or decreased through degradation of the IKZF4 protein mediated by an E3 ligase.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in treating a disease associated with the modulation of, the reduction of, or a decrease in IKZF2 and IKZF4 protein levels. In some embodiments, IKZF2 and IKZF4 protein levels are modulated, reduced, or decreased through degradation of the IKZF2 and IKZF4 proteins. In other embodiments, IKZF2 and IKZF4 protein levels are modulated, reduced, or decreased through degradation of the IKZF2 and IKZF4 proteins mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2-dependent disease or disorder by reducing or decreasing IKZF2 protein levels, wherein reduction or decrease of IKZF2 protein levels treats the IKZF2-dependent disease or disorder.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2 and IKZF4-dependent disease or disorder by reducing or decreasing IKZF2 and IKZF4 protein levels wherein the reduction of or decrease in IKZF2 and IKZF4 protein levels treats the IKZF2 and IKZF4-dependent disease or disorder.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of cancer.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2-dependent cancer.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2-dependent and IKZF4-dependent cancer.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of a cancer affected by the modulation of, the reduction of, or a decrease in IKZF2 protein levels.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of a cancer affected by the modulation of, the reduction of, or a decrease in IKZF2 and IKZF4 protein levels.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the degradation IKZF2. In some embodiments, IKZF2 protein degradation is mediated by an E3 ligase.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the modulation IKZF2 protein levels through degradation of IKZF2. In some embodiments, IKZF2 protein degradation is mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in treating an IKZF2-dependent disease or disorder in a patient in need thereof, by modulating IKZF2 protein levels through the degradation of IKZF2. In some embodiments, IKZF2 protein degradation is mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in reducing the proliferation of a cell by IKZF 2 protein levels. In some embodiments, IKZF2 protein levels are reduced through degradation of the IKZF2 protein. In some embodiments, IKZF2 protein levels are reduced through degradation of the IKZF2 protein mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the degradation IKZF2 and IKZF4. In some embodiments, IKZF2 and IKZF4 protein degradation is mediated by an E3 ligase.

In another aspect, the present invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the modulation of IKZF2 and IKZF4 protein levels through degradation of IKZF2 and IKZF4. In some embodiments, IKZF2 and IKZF4 protein degradation is mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in treating an IKZF2-dependent and IKZF4-dependent disease or disorder in a patient in need thereof by modulating IKZF2 and IKZF4 protein levels through the degradation of IKZF2 and IKZF4. In some embodiments, IKZF2 and IKZF4 protein degradation is mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in reducing the proliferation of a cell by reducing IKZF2 and IKZF4 protein levels. In some embodiments, IKZF2 and IKZF4 protein levels are reduced through degradation of the IKZF2 and IKZF4 proteins. In other embodiments, IKZF2 and IKZF4 protein levels are reduced through degradation of the IKZF2 and IKZF4 proteins mediated by an E3 ligase.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2-dependent disease or disorder.

Another aspect of the invention relates to a compound of Formula (I), or a pharmaceutically acceptable salt, hydrate, solvate, stereoisomer, or tautomer thereof, or a composition comprising a compound of Formula (I), or a pharmaceutically acceptable salt, hydrate, solvate, stereoisomer, or tautomer thereof, for use in the treatment of an IKZF2-dependent and IKZF4-dependent disease or disorder.

In another aspect, the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the reduction of IKZF2 protein levels.

Another aspect of the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the reduction of IKZF2 and IKZF4 protein levels.

In another aspect, the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the reduction of IKZF2 protein levels, wherein reduction of IKZF2 protein levels treats or ameliorates the disease or disorder.

Another aspect of the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the reduction of IKZF2 and IKZF4 protein levels, wherein reduction of IKZF2 and IKZF4 protein levels treats or ameliorates the disease or disorder.

In another aspect, the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the treatment of a disease or disorder by reducing IKZF2 protein levels, wherein reduction of IKZF2 protein levels treats or ameliorates the disease or disorder.

Another aspect of the present invention relates to a compound of Formula (I), or a pharmaceutically acceptable salt, hydrate, solvate, stereoisomer, or tautomer thereof or a composition comprising a compound of Formula (I), or a pharmaceutically acceptable salt, hydrate, solvate, stereoisomer, or tautomer thereof for use in the treatment of a disease or disorder by reducing IKZF2 and IKZF4 protein levels, wherein reduction of IKZF2 and IKZF4 protein levels treats or ameliorates the disease or disorder.

The compounds of the present invention can be used for the treatment, of a disease or disorder selected from liposarcoma, neuroblastoma, glioblastoma, bladder cancer, adrenocortical cancer, multiple myeloma, colorectal cancer, non-small cell lung cancer, Human Papilloma Virus-associated cervical, oropharyngeal, penis, anal, thyroid, or vaginal cancer or Epstein-Barr Virus-associated nasopharyngeal carcinoma, gastric cancer, rectal cancer, thyroid cancer, Hodgkin lymphoma or diffuse large B-cell lymphoma. the cancer is selected from prostate cancer, breast carcinoma, lymphomas, leukaemia, myeloma, bladder carcinoma, colon cancer, cutaneous melanoma, hepatocellular carcinoma, endometrial cancer, ovarian cancer, cervical cancer, lung cancer, renal cancer, glioblastoma multiform, glioma, thyroid cancer, parathyroid tumor, nasopharyngeal cancer, tongue cancer, pancreatic cancer, esophageal cancer, cholangiocarcinoma, gastric cancer, soft tissue sarcomas, rhabdomyosarcoma (RMS), synovial sarcoma, osteosarcoma, rhabdoid cancers, cancer for which the immune response is deficient, an immunogenic cancer, and Ewing's sarcoma. In one embodiment, the IKZF2-dependent disease or disorder is a disease or disorder is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, and gastrointestinal stromal tumor (GIST). In another embodiment, the cancer is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, acute myelogenous leukemia, and gastrointestinal stromal tumor (GIST). In another embodiment, the IKZF2-dependent disease or disorder is a disease or disorder is selected from non-small cell lung cancer (NSCLC), melanoma, triple-negative breast cancer (TNBC), nasopharyngeal cancer (NPC), and microsatellite stable colorectal cancer (mssCRC).

The disclosed compounds of the invention can be administered in effective amounts to treat or prevent a disorder and/or prevent the development thereof in subjects.

### G. Administration, Pharmaceutical Compositions, and Dosing of Compounds of the Invention

Administration of the disclosed compounds can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, and all using forms well known to those skilled in the pharmaceutical arts.

Illustrative pharmaceutical compositions are tablets and gelatin capsules comprising a compound of the invention and a pharmaceutically acceptable carrier, such as a) a diluent, e.g., purified water, triglyceride oils, such as hydrogenated or partially hydrogenated vegetable oil, or mixtures thereof, com oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride, and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes, and/or polyvinylpyrrolidone, if desired; d) a disintegrant, e.g., starches, agar, methyl cellulose, bentonite, xanthan gum, algic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions; using polyalkylene glycols such as propylene glycol, as the carrier.

The disclosed compounds can also be administered in the form of liposome delivery systems, such as small unilamellar vesicles, large unilamellar vesicles, and multilamellar vesicles. Liposomes can be formed from a variety of phospholipids, containing cholesterol, stearylamine or phosphatidylcholines.

In some embodiments, a film of lipid components is hydrated with an aqueous solution of drug to a form lipid layer encapsulating the drug, as described in U.S. Pat. No. 5,262,564.

Disclosed compounds can also be delivered by the use of monoclonal antibodies as individual carriers to which the disclosed compounds are coupled. The disclosed compounds can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the disclosed compounds can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates, and cross-linked or amphipathic block copolymers of hydrogels. In one embodiment, disclosed compounds are not covalently bound to a polymer, e.g., a polycarboxylic acid polymer, or a polyacrylate.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Another aspect of the invention is directed to pharmaceutical compositions comprising a compound of Formula (I), and a pharmaceutically acceptable carrier. The pharmaceutical acceptable carrier may further include an excipient, diluent, or surfactant.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

In one embodiment, the invention provides a kit comprising two or more separate pharmaceutical compositions, at least one of which contains a compound of the present invention. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

The dosage regimen utilizing the disclosed compound is selected in accordance with a variety of factors including type, species, age, weight, sex, and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about 0.5 mg to about 5000 mg of the disclosed compound as needed to treat the condition. Compositions for *in vivo* or *in vitro* use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

### H. Combination Therapy

The compounds of the invention can be administered in therapeutically effective amounts in a combinational therapy with one or more therapeutic agents (pharmaceutical combinations) or modalities, e.g., non-drug therapies. For example, synergistic effects can occur with other cancer agents. Where the compounds of the invention are administered in conjunction with other therapies, dosages of the co-administered compounds will of course vary depending on the type of co-drug employed, on the specific drug employed, on the condition being treated and so forth.

The compounds can be administered simultaneously (as a single preparation or separate preparation), sequentially, separately, or over a period of time to the other drug therapy or treatment modality. In general, a combination therapy envisions administration of two or more drugs during a single cycle or course of therapy. A therapeutic agent is, for example, a chemical compound, peptide, antibody, antibody fragment or nucleic acid, which is therapeutically active or enhances the therapeutic activity when administered to a patient in combination with a compound of the present invention.

In one aspect, a compound of Formula (I), or a pharmaceutically acceptable salt, hydrate, solvate, stereoisomer, or tautomer thereof, of the present invention can be combined with other therapeutic agents, such as other anti-cancer agents, anti-allergic agents, anti-nausea agents (or anti-emetics), pain relievers, cytoprotective agents, and combinations thereof.

In some embodiments, the compounds of Formula (I), or a pharmaceutically acceptable salt, hydrate, solvate, stereoisomer, or tautomer thereof of the present invention are administered in combination with one or more second agent(s) selected from a PD-1 inhibitor, a PD-L1 inhibitor, a LAG-3 inhibitor, a cytokine, an A2A antagonist, a GITR agonist, a TIM-3 inhibitor, a STING agonist, and a TLR7 agonist, to treat a disease, e.g., cancer.

In other embodiments, the compounds of Formula (I), or a pharmaceutically acceptable salt, hydrate, solvate, stereoisomer, or tautomer thereof, of the present invention are used in combination with one or more other anti-HER2 antibodies, e.g., trastuzumab, pertuzumab, margetuximab, or HT-19 described above, or with other anti-HER2 conjugates, e.g., ado-trastuzumab emtansine (also known as Kadcyla^{®}, or T-DM1).

In other embodiments, the compounds of Formula (I), or a pharmaceutically acceptable salt, hydrate, solvate, stereoisomer, or tautomer thereof, of the present invention are used in combination with one or more tyrosine kinase inhibitors, including but not limited to, EGFR inhibitors, Her3 inhibitors, IGFR inhibitors, and Met inhibitors, for treating a disease, e.g., cancer.

In another embodiment, the compounds of Formula (I) of the present invention are used in combination with one or more proliferation signaling pathway inhibitors, including but not limited to, MEK inhibitors, BRAF inhibitors, PI3K/Akt inhibitors, SHP2 inhibitors, and also mTOR inhibitors, and CDK inhibitors, for treating a disease, e.g., cancer.

In yet another embodiment, the compounds of Formula (I), or a pharmaceutically acceptable salt, hydrate, solvate, stereoisomer, or tautomer thereof, of the present invention are used in combination with one or more pro-apoptotics, including but not limited to, IAP inhibitors, BCL2 inhibitors, MCL1 inhibitors, TRAIL agents, CHK inhibitors, for treating a disease, e.g., cancer.

In a further embodiment, the compounds of Formula (I), or a pharmaceutically acceptable salt, hydrate, solvate, stereoisomer, or tautomer thereof, of the present invention are used in combination with one or more immunomodulators (*e.g.,* one or more of an activator of a costimulatory molecule or an inhibitor of an immune checkpoint molecule), for treating a disease, e.g., cancer.

### EXAMPLES

The invention is further illustrated by the following examples and synthesis schemes, which are not to be construed as limiting this invention in scope to the specific procedures herein described. It is to be understood that the examples are provided to illustrate certain embodiments and that no limitation to the scope of the invention is intended thereby.

Compounds of the present invention may be prepared by methods known in the art of organic synthesis. In all of the methods it is understood that protecting groups for sensitive or reactive groups may be employed where necessary in accordance with general principles of chemistry. Protecting groups are manipulated according to standard methods of organic synthesis (T.W. Green and P.G.M. Wuts (1999) Protective Groups in Organic Synthesis, 3rd edition, John Wiley & Sons). These groups are removed at a convenient stage of the compound synthesis using methods that are readily apparent to those skilled in the art.

### Analytical Methods, Materials, and Instrumentation

Unless otherwise noted, reagents and solvents were used as received from commercial suppliers. Proton nuclear magnetic resonance (NMR) spectra were obtained on either Bruker Avance spectrometer or Varian Oxford 400 MHz spectrometer unless otherwise noted. Spectra are given in ppm (δ) and coupling constants, *J*, are reported in Hertz. Tetramethylsilane (TMS) was used as an internal standard. Chemical shifts are reported in ppm relative to dimethyl sulfoxide (δ 2.50), methanol (δ 3.31), chloroform (δ 7.26) or other solvent as indicated in NMR spectral data. A small amount of the dry sample (2-5 mg) is dissolved in an appropriate deuterated solvent (1 mL). The chemical names were generated using ChemBioDraw Ultra v12 from Cambridge Soft.

Mass spectra (ESI-MS) were collected using a Waters System (Acquity UPLC and a Micromass ZQ mass spectrometer) or Agilent-1260 Infinity (6120 Quadrupole); all masses reported are the m/z of the protonated parent ions unless recorded otherwise. The sample was dissolved in a suitable solvent such as MeCN, DMSO, or MeOH and was injected directly into the column using an automated sample handler. The analysis is performed on Waters Acquity UPLC system (Column: Waters Acquity UPLC BEH C18 1.7µm, 2.1 x 30mm; Flow rate: 1 mL/min; 55°C (column temperature); Solvent A: 0.05% formic acid in water, Solvent B: 0.04% formic acid in MeOH; gradient 95% Solvent A from 0 to 0.10 min; 95% Solvent A to 20% Solvent A from 0.10 to 0.50 min; 20% Solvent A to 5% Solvent A from 0.50 to 0.60 min; hold at 5% Solvent A from 0.6 min to 0.8 min; 5% Solvent A to 95% Solvent A from 0.80 to 0.90 min; and hold 95% Solvent A from 0.90 to 1.15 min.
*Abbreviations used in the following examples and elsewhere herein are:*
- AC₅₀: half maximal active concentration
- AcOH: Acetic acid
- AIBN: azobisisobutyronitrile
- aq.: Aqueous
- BuLi: n-butyllithium
- br: broad
- d: doublet
- dd: doublet of doublets
- ddd: doublet of doublet of doublets
- ddq: doublet of doublet of quartets
- ddt: doublet of doublet of triplets
- dq: doublet of quartets
- dt: doublet of triplets
- dtd: doublet of triplet of doublets
- CDI: carbonyldiimidazole
- Cs₂CO₃: cesium carbonate
- DCE: 1,2-dichloroethane
- DCM: dichloromethane
- DIPEA: N,N-Diisopropylethylamine
- DMA: *N,N*-dimethylacetamide
- DMAP: 4-dimethylaminopyridine
- DME: 1,2-Dimethoxyethane
- DMF: *N,N*-dimethylformamide
- DMP: Dess-Martin periodinane or 1,1,1-Tris(acetyloxy)-1,1-dihydro-1,2-benziodoxol-3-(1H)-one
- DMSO: dimethylsulfoxide
- EC₅₀: half maximal effective concentration
- EtOH: ethanol
- Et₂O: diethyl ether
- EtOAc: ethyl acetate
- HCl: hydrogen chloride
- hept: heptet
- HPLC: high performance liquid chromatography
- h or hr: hour
- HRMS: high resolution mass spectrometry
- g: gram
- IC₅₀: half maximal inhibitory concentration
- K₂CO₃: potassium carbonate
- KI: potassium iodide
- K₃PO₄: tripotassium phosphate
- LCMS: liquid chromatography mass spectrometry
- m: multiplet
- MeCN: acetonitrile
- MeOH: methanol
- mg: milligram
- MHz: megahertz
- min: minutes
- mL: milliliter
- mmol: millimole
- M: molar
- MS: mass spectrometry
- MsCl: methanesulfonyl chloride
- NaB(OAc)₃H: sodium triacetoxyborohydride
- NaHCOs: sodium bicarbonate
- Na₂SO₄: sodium sulfate
- NBS: *N*-bromosuccinimide
- NiBr₂·DME: nickel (II) bromide ethylene glycol dimethyl ether complex
- NMI: n-methylimidazole
- NMP: N-Methyl-2-pyrrolidone
- NMR: Nuclear magnetic resonance
- PdCl₂(dppf)•DCM: [1,1'-Bis(diphenylphosphino)ferrocene]dichloropalladium(II), complex with dichloromethane
- Pd/C: palladium on carbon
- q: quartet
- qd: quartet of doublets
- quint: quintet
- quintd: quintet of doublets
- rt: room temperature
- Rt: retention time
- s: singlet
- sat.: saturated
- t: triplet
- TEA or Et₃N: triethylamine
- td: triplet of doublets
- tdd: triplet of doublet of doublets
- THF: tetrahydrofuran
- TMP: 2,2,6,6-tetramethylpiperidine
- Ts: tosyl
- tt: triplet of triplets
- ttd: triplet of triplet of doublets
- TLC: thin-layer chromatography
- UPLC: ultra-Performance Liquid Chromatography
- XPhos Pd G2: chloro(2-dicyclohexylphosphino-2',4',6'-triisopropyl-1,1'-biphenyl)[2-(2' -amino-1,1' -biphenyl)]palladium(II)
- v/v/v: volume/volume/volume (volume ratio)
- µW: microwave

### Example 1: 3-(4-fluoro-1-oxo-5-(piperidin-4-yl)isoindolin-2-yl)piperidine-2,6-dione HCl salt (INT-A)

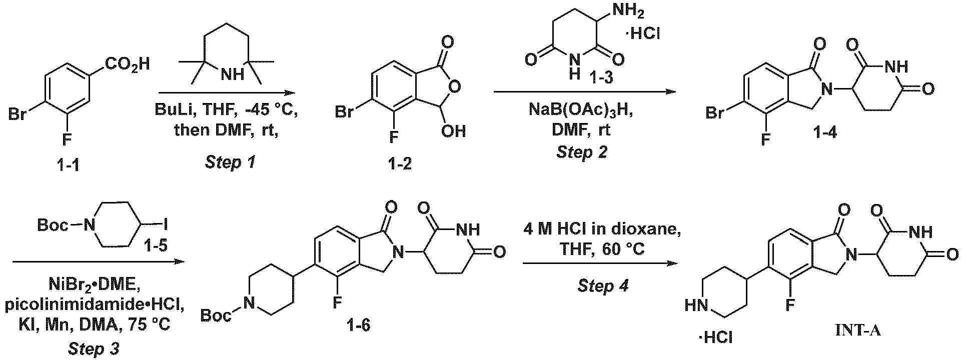

### Step 1. 5-bromo-4-fluoro-3-hydroxyisobenzofuran-1(3H)-one (1-2):

To a stirred solution of TMP (57.0 mL, 57.0 mmol) in THF (40 mL) under an atmosphere of nitrogen was added BuLi (2.7 M in heptane, 20.3 mL, 54.7 mmol) dropwise at 0 °C and the resulting mixture was stirred for 30 min at 0 °C. The reaction mixture was then cooled to about -45 °C (using dry ice/MeCN bath) and 4-bromo-3-fluorobenzoic acid (4.99 g, 22.8 mmol), dissolved in THF (15 mL), was added dropwise and stirring was continued at -45 °C for 5 h. DMF (2.65 mL, 34.2 mmol) was then added dropwise and the reaction mixture was allowed to warm to rt and stirred overnight. The reaction mixture was quenched with aq. 3M HCl (40 mL) at 0 °C and extracted with DCM (x3). The combined organic phases were dried over Na₂SO₄, filtered, and concentrated to dryness. The crude product was purified via silica gel chromatography eluting with 0 to 100% EtOAc in heptane to afford **1-2** (2.91 g, 11.40 mmol, 50% yield) as a pale brown solid. MS [M+H]⁺ = 247.0. ¹H NMR (400 MHz, Acetonitrile-*d₃*) δ 7.90 (dd, *J* = 8.0, 5.8 Hz, 1H), 7.56 (d, *J* = 8.0 Hz, 1H), 6.72 (s, 1H), 5.92 (br s, 1H).

### Step 2. 3-(5-bromo-4-fluoro-1-oxoisoindolin-2-yl)piperidine-2,6-dione (1-4):

To a stirred solution of **1-2** (2.90 g, 11.7 mmol) in DMF (20 mL) was added 3-aminopiperidine-2,6-dione HCl salt (**1-3,** 2.90 g, 17.6 mmol) and NaB(OAc)₃H (6.22 g, 29.3 mmol) and the resulting mixture was stirred for 2 days at rt. The reaction mixture was diluted with H₂O (50 mL) and cooled to 0 °C with water/ice bath which resulted in the formation of precipitate. The resulting mixture was filtered and the dark blue solid was washed with Et₂O (x3). The obtained solid was dried in a vacuum oven to afford **1-4** (1.89 g, 5.31 mmol, 45 % yield) as a grey solid. MS [M+H]⁺ = 341.1. ¹H NMR (400 MHz, DMSO-*d₆*) δ 11.02 (s, 1H), 7.88 (dd, *J* = 8.0, 6.0 Hz, 1H), 7.55 (d, *J* = 8.0 Hz, 1H), 5.12 (dd, *J* = 13.3, 5.1 Hz, 1H), 4.62 (d, *J* = 17.6 Hz, 1H), 4.45 (d, *J* = 17.6 Hz, 1H), 2.99 - 2.85 (m, 1H), 2.66 - 2.55 (m, 1H), 2.47 - 2.36 (m, 1H), 2.05 - 1.96 (m, 1H).

### Step 3. tert-butyl 4-(2-(2,6-dioxopiperidin-3-yl)-4-fluoro-1-oxoisoindolin-5-yl)piperidine-1-carboxylate (1-6):

To a stirred suspension of NiBr₂•(DME) (13.57 mg, 0.044 mmol), picolinamide HCl salt (6.93 mg, 0.044 mmol), KI (438 mg, 2.64 mmol) and manganese powder (241 mg, 4.40 mmol) in DMA (1 mL) under an atmosphere of nitrogen were added **1-4** (300 mg, 0.879 mmol) and tert-butyl 4-iodopiperidine-1-carboxylate (**1-5**, 410 mg, 1.319 mmol), dissolved in DMA (2 mL). The resulting mixture was then stirred vigorously at 75 °C for 6 hours under an atmosphere of nitrogen. The reaction mixture was filtered and was washed with a minimal amount of MeCN. The obtained filtrate was concentrated to dryness. The crude product was purified via silica gel chromatography eluting with 0 to 50% EtOAc:EtOH (v/v = 3:1) in DCM to afford **1-6** (117 mg, 0.191 mmol, 22% yield) as a white powder. MS [M-*t*Bu+H]⁺= 390.3.

### Step 4. 3-(4-fluoro-1-oxo-5-(piperidin-4-yl)isoindolin-2-yl)piperidine-2,6-dione HCl salt (INT-A):

To a stirred solution of **1-6** (117 mg, 0.192 mmol) in THF (3 mL) was added 4 M hydrogen chloride in dioxane (1.5 mL, 6.00 mmol) and the resulting mixture was stirred for 4 hours at 60 °C. Formation of a precipitate was observed. The reaction mixture was diluted with Et₂O (6 mL) and filtered. The precipitate was washed with Et₂O (x4) and then dried on a high vacuum to afford **INT-A** (64 mg, 0.16 mmol, 85% yield) was obtained as a white solid. MS [M+H]⁺ = 346.1. ¹H NMR (400 MHz, Deuterium Oxide) δ 7.65 (d, *J* = 7.9 Hz, 1H), 7.55 (dd, *J* = 7.9, 6.2 Hz, 1H), 5.19 (dd, *J* = 13.3, 5.3 Hz, 1H), 4.69 (d, *J* = 17.6 Hz, 1H), 4.59 (d, *J* = 17.6 Hz, 1H), 3.61 - 3.54 (m, 2H), 3.38 (tt, *J* = 12.2, 3.8 Hz, 1H), 3.20 (td, *J* = 13.0, 3.2 Hz, 2H), 3.01 - 2.84 (m, 2H), 2.56 (qd, *J* = 12.9, 5.3 Hz, 1H), 2.33 - 2.25 (m, 1H), 2.18 - 2.11 (m, 2H), 2.10 - 1.97 (m, 2H).

### Example 2: 3-(5-(1-benzylpiperidin-4-yl)-4-fluoro-1-oxoisoindolin-2-yl)piperidine-2,6-dione (I-3)

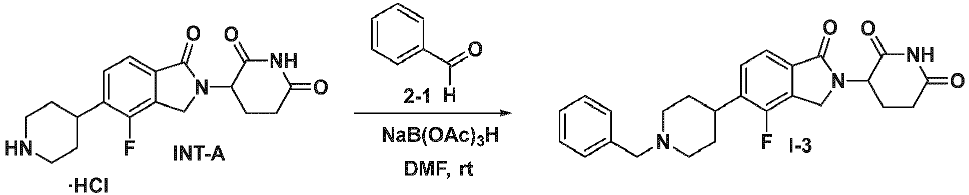

To a stirred solution of **INT-A** (60.0 mg, 0.157 mmol) in DMF (1 mL) were added NaB(OAc)₃H (66.6 mg, 0.314 mmol) and benzaldehyde (**2-1**, 0.032 mL, 0.31 mmol) under an atmosphere of nitrogen. The resulting mixture was stirred for 5 h at room temperature. The reaction mixture was concentrated to dryness and the crude product was purified via silica gel chromatography eluting with 0 to 100% EtOAc:EtOH:Et₃N (v/v/v= 75:25:1) in DCM to afford **I-3** (25.5 mg, 0.058 mmol, 37% yield) as a white solid. MS [M+H]⁺ = 436.3. ¹H NMR (400 MHz, Methylene Chloride-*d₂*) δ 8.27 (s, 1H), 7.55 (d, *J* = 7.8 Hz, 1H), 7.43 - 7.38 (m, 1H), 7.34 - 7.28 (m, 4H), 7.27 - 7.21 (m, 1H), 5.11 (dd, *J* = 13.4,5.2 Hz, 1H), 4.42 (d, *J* = 16.3 Hz, 1H), 4.35 (d, *J* = 16.3 Hz, 1H), 3.60 (s, 2H), 3.06 (d, *J* = 11.3 Hz, 2H), 2.98 - 2.87 (m, 1H), 2.86 - 2.75 (m, 2H), 2.33 (qd, *J* = 12.9, 5.7 Hz, 1H), 2.22 - 2.09 (m, 3H), 1.90 - 1.76 (m, 4H).

### Example 3: 3-(6-fluoro-1-oxo-5-(1-(pyridin-4-ylmethyl)piperidin-4-yl)isoindolin-2-yl)piperidine-2,6-dione (I-16).

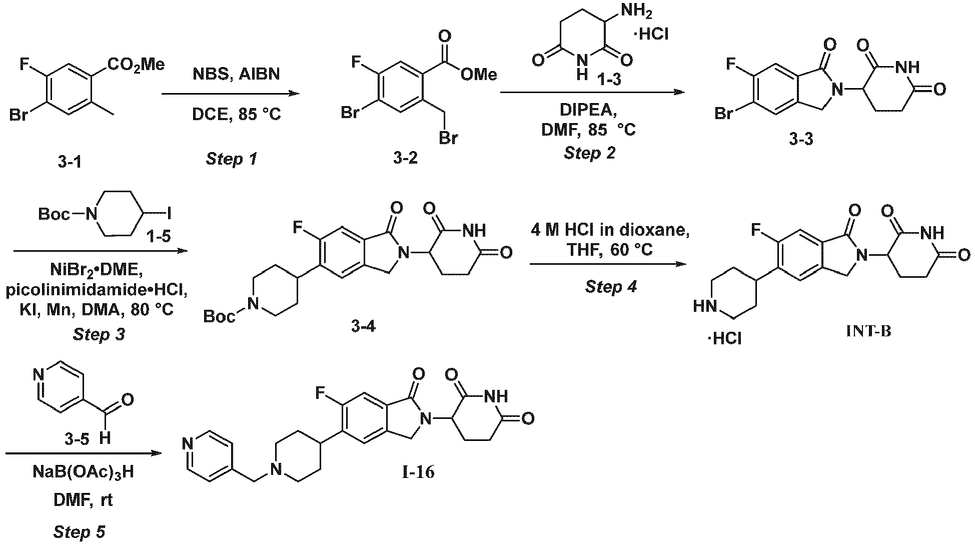

### Step 1. Methyl 4-bromo-2-(bromomethyl)-5-fluorobenzoate (3-2):

To a stirred solution of 4-bromo-5-fluoro-2-methylbenzoate (**3-1**, 2700 mg, 10.93 mmol) in DCE (25 mL) under an atmosphere of nitrogen was added NBS (2140 mg, 12.02 mmol) followed by AIBN (90 mg, 0.55 mmol), and the resulting mixture was stirred vigorously at 85 °C for 8 h. The reaction mixture was quenched with sat. aq. Na₂S₂O₃ and then extracted with DCM (x3). The combined organic extracts were concentrated to dryness. The crude product was purified via silica gel chromatography eluting with 0 to 50% EtOAc in heptane to afford **3-2** (3.37 g, 9.30 mmol, 85% yield) as a colorless oil. ¹H NMR (400 MHz, Chloroform-d) δ 7.73 (d, *J* = 9.0 Hz, 1H), 7.69 (d, *J* = 6.5 Hz, 1H), 4.89 (s, 2H), 3.95 (s, 3H).

### Step 2. 3-(5-bromo-6-fluoro-1-oxoisoindolin-2-yl)piperidine-2,6-dione (3-3):

To a solution of **3-2** (3.37 g, 9.30 mmol) in DMF (20 mL) was added 3-aminopiperidine-2,6-dione HCl salt (**1-3**, 2.30 g, 14.0 mmol), followed by DIPEA (8.10 mL, 46.5 mmol), and the resulting mixture was stirred at 85 °C for 2 days. Excess DIPEA was removed by concentrating the mixture to a constant volume at 100 mbar, 40 °C. The reaction mixture was then poured into conical flask containing H₂O (80 mL). The precipitate that formed was filtered and washed with H₂O (x2) and Et₂O (x2). The obtained solid was dried in the vacuum oven for 5 hours to afford **3-3** (2.22 g, 6.51 mmol, 70% yield) as a dark grey solid. MS [M+H]⁺ = 341.1 and 343.1 (Br isotopes). ¹H NMR (400 MHz, DMSO-*d₆*) δ 11.01 (s, 1H), 8.05 (d, *J* = 6.0 Hz, 1H), 7.71 (d, *J* = 7.7 Hz, 1H), 5.12 (dd, *J* = 13.3, 5.1 Hz, 1H), 4.46 (d, *J* = 17.5 Hz, 1H), 4.33 (d, *J* = 17.5 Hz, 1H), 2.97 - 2.83 (m, 1H), 2.65 - 2.56 (m, 1H), 2.39 (qd, *J* = 13.2, 4.5 Hz, 1H), 2.09 - 1.94 (m, 1H).

### Step 3. tert-butyl 4-(2-(2,6-dioxopiperidin-3-yl)-6-fluoro-1-oxoisoindolin-5-yl)piperidine-1-carboxylate (3-4):

To a stirred suspension of NiBr₂•(DME) (18 mg, 0.059 mmol), picolinamide HCl salt (9.2 mg, 0.059 mmol), KI (584 mg, 3.52 mmol) and manganese powder (322 mg, 5.86 mmol) in DMA (1 mL) under an atmosphere of nitrogen was added **3-3** (400 mg, 1.17 mmol) and tert-butyl 4-iodopiperidine-1-carboxylate (**1-5**, 547 mg, 1.76 mmol) dissolved in DMA (4 mL). The resulting mixture was stirred vigorously at 80 °C for 7.5 h under an atmosphere of nitrogen. The reaction was filtered and washed with minimal amount of MeCN. The obtained filtrate was concentrated to a constant volume. The obtained dark brown solution was diluted with H₂O (40 mL) which caused formation of a brown precipitate. The solid was filtered, washed with H₂O (x2) and then heptane (x3) to afford crude **3-4** (390 mg) as a brown solid. The crude product was used in the next step without further purification. MS [M-H]⁻ = 444.5.

### Step 4. 3-(6-fluoro-1-oxo-5-(piperidin-4-yl)isoindolin-2-yl)piperidine-2,6-dione HCl salt (INT-B):

To a solution of crude **3-4** (390 mg) in THF (4 mL) was added 4 M hydrogen chloride in dioxane (2.0 mL, 8.0 mmol) and the resulting mixture was stirred for 1.5 hours a 60 °C. Formation of precipitate was observed. The reaction mixture was diluted with Et₂O (4 mL) and filtered. The precipitate was washed with Et₂O (x4) and then dried on a high vacuum to afford **INT-B** (301 mg, 0.631 mmol, 54% yield over 2 steps) as a grey solid. The obtained product was used in the next step without further purification. MS [M+H]⁺= 346.2.

### Step 5. 3-(6-fluoro-1-oxo-5-(1-(pyridin-4-ylmethyl)piperidin-4-yl)isoindolin-2-yl)piperidine-2,6-dione (I-16)

To a solution of **INT-B** (100 mg, 0.21 mmol) in DMF (1 mL) was added NaB(OAc)₃H (89 mg, 0.42 mmol) and 4-pyridinecarboxaldehyde (**3-5**, 0.030 mL, 0.31 mmol). The resulting mixture was stirred overnight at room temperature. The reaction mixture was concentrated to dryness. The crude product was purified via silica gel chromatography eluting with 0 to 100% EtOAc:EtOH:Et₃N (v/v/v = 75:25:1) in DCM to afford **I-16** (35.7 mg, 0.082 mmol, 39% yield) as a pale yellow solid. MS [M+H]⁺ = 437.3. ¹H NMR (400 MHz, DMSO-*d₆*) δ 11.00 (s, 1H), 8.51 (d, *J* = 5.0 Hz, 2H), 7.63 (d, *J* = 6.3 Hz, 1H), 7.46 (d, *J* = 9.1 Hz, 1H), 7.35 (d, *J* = 5.0 Hz, 2H), 5.11 (dd, *J* = 13.4, 5.1 Hz, 1H), 4.42 (d, *J* = 17.1 Hz, 1H), 4.29 (d, *J* = 17.1 Hz, 1H), 3.55 (s, 2H), 2.97 - 2.82 (m, 4H), 2.59 (d, *J* = 17.2 Hz, 1H), 2.45 - 2.30 (m, 1H), 2.22 - 2.07 (m, 2H), 2.03 - 1.92 (m, 1H), 1.76 (s, 4H).

### Example 4: 3-(2-(1-benzylpiperidin-4-yl)-5-oxo-5,7-dihydro-6H-pyrrolo[3,4-b]pyridin-6-yl)piperidine-2,6-dione (I-1)

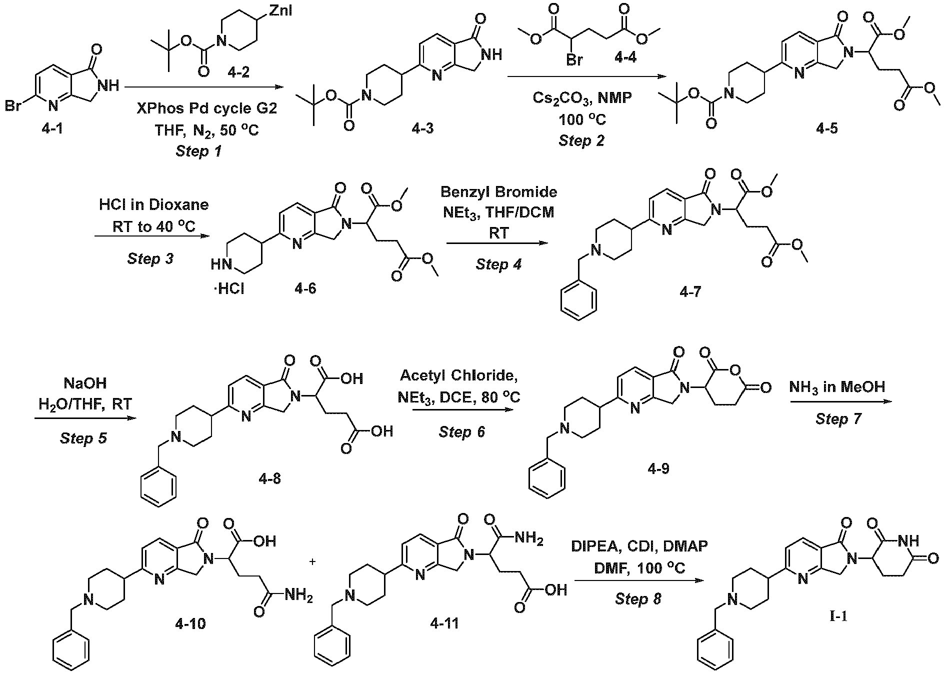

### Step 1. tert-butyl 4-(5-oxo-6,7-dihydro-5H-pyrrolo[3,4-b]pyridin-2-yl)piperidine-1-carboxylate (4-3):

A suspension of 2-bromo-6,7-dihydro-pyrrolo[3,4-b]pyridin-5-one (**4-1**, 0.100 g, 0.469 mmol) and XPhos Pd cycle G2 (0.055 g, 0.070 mmol) in THF (2.3 mL) was placed under an atmosphere of nitrogen by evacuating and backfilling the flask with nitrogen three times. 0.5 M 1-(tert-butoxycarbonyl)piperidin-4-yl)zinc(II) iodide (**4-2**) in THF (2.8 mL, 1.4 mmol) was added and the resulting mixture was stirred at 50 °C for 4 h. The reaction mixture was cooled to room temperature, quenched with saturated ammonium chloride, and extracted with ethyl acetate (x4). The combined organic extracts were passed through a phase separator and concentrated to dryness. The crude material was purified by silica gel chromatography eluting with 0-100% ethyl acetate in heptane then 0-10% methanol in dichloromethane to afford **4-3** (85.2 mg, 0.268 mmol, 57% yield) as a yellow solid. MS [M-*t*Bu+H]⁺= 262.2.

### Step 2. Dimethyl 2-(2-(1-(tert-butoxycarbonyl)piperidin-4-yl)-5-oxo-5H-pyrrolo[3,4-b]pyridin-6(7H)-yl)pentanedioate (4-5)

To a solution of dimethyl 2-bromopentanedioate (**4-4**, 0.128 g, 0.537 mmol) in NMP (2.7 mL) was added tert-butyl 4-(5-oxo-6,7-dihydro-5H-pyrrolo[3,4-b]pyridin-2-yl)piperidine-1-carboxylate (**4-3**, 0.0852 g, 0.268 mmol) and Cs₂CO₃ (0.175 g, 0.537 mmol) and the resulting mixture was stirred at 100 °C for 16 h. The reaction mixture was cooled to room temperature, quenched with sat. aq. ammonium chloride, and extracted with ethyl acetate (x3). The combined organic extracts were washed with 1:1 saturated brine and water (x3), passed through a phase separator, and concentrated onto Celite^{®}. The crude product was purified by silica gel chromatography eluting with 0-100% ethyl acetate in heptane to afford **4-5** (52 mg, 0.11 mmol, 41 % yield) as a yellow oil. MS [M+H]⁺ = 476.4. ¹H NMR (400 MHz, Chloroform-*d*) δ 8.07 (d, *J* = 7.9 Hz, 1H), 7.28 (d, *J* = 8.0 Hz, 1H), 5.14 (dd, *J* = 10.7, 4.8 Hz, 1H), 4.63 (d, *J* = 17.2 Hz, 1H), 4.40 (d, *J* = 17.2 Hz, 1H), 3.76 (s, 3H), 3.64 (s, 3H), 3.02 - 2.77 (m, 4H), 2.59 - 2.32 (m, 4H), 2.26 - 2.16 (m, 1H), 1.94 (d, *J =* 13.5 Hz, 2H), 1.79 (qd, *J* = 12.5, 4.3 Hz, 2H), 1.49 (s, 9H).

### Step 3. Dimethyl 2-(5-oxo-2-(piperidin-4-yl)-5H-pyrrolo[3,4-b]pyridin-6(7H)-yl)pentanedioate hydrochloride (4-6)

To a solution of dimethyl 2-(2-(1-(tert-butoxycarbonyl)piperidin-4-yl)-5-oxo-5H-pyrrolo[3,4-b]pyridin-6(7H)-yl)pentanedioate (**4-5,** 0.052 g, 0.11 mmol) in dioxane (1 mL) was added 4M HCl in dioxane (0.10 mL, 3.3 mmol). The resulting mixture was stirred at room temperature for 2 h, stirred at 40 °C for 2 h, and then stirred at room temperature for 16 h. The reaction mixture was concentrated to dryness to afford product **4-6** as a yellow solid, which was carried onto the next step without purification. MS [M+H]⁺ = 376.4.

### Step 4. Dimethyl 2-(2-(1-benzylpiperidin-4-yl)-5-oxo-SH-pyrrolo[3,4-b]pyridin-6(7H)-yl)pentanedioate (4-7)

To a solution of dimethyl 2-(5-oxo-2-(piperidin-4-yl)-5H-pyrrolo[3,4-b]pyridin-6(7H)-yl)pentanedioate (**4-6**, 40.9 mg, 0.109 mmol) in THF (1 mL) and DCM (1 mL) was added Et₃N (0.076 ml, 0.55 mmol). The resulting mixture was stirred at room temperature for 15 minutes and then benzyl bromide (0.016 mL, 0.13 mmol) was added. The reaction mixture was stirred at room temperature for 2 h, and then diluted with water and extracted with DCM (x3). The combined organic extracts were passed through a phase separator and concentrated to dryness. The crude material was purified by silica gel chromatography (eluting with 0-100% EtOAc in heptane) to afford product **4-7** (36.3 mg, 0.078 mmol, 71.5 % yield) as a yellow oil, which was carried onto the next step without purification. MS [M+H]⁺ = 466.5.

### Step 5. 2-(2-(1-benzylpiperidin-4-yl)-5-oxo-5H-pyrrolo[3,4-b]pyridin-6(7H)-yl)pentanedioic acid (4-8):

To a solution of dimethyl 2-(2-(1-benzylpiperidin-4-yl)-5-oxo-SH-pyrrolo[3,4-b]pyridin-6(7H)-yl)pentanedioate (**4-7,** 0.036 g, 0.078 mmol) in THF (1 mL) was added 5M aq. NaOH (0.031 mL, 0.16 mmol) and the resulting mixture was stirred at room temperature for 1 h. Methanol was added (0.2 mL) and stirring was continued at room temperature for 30 minutes. The reaction mixture was quenched with 4M HCl in dioxane (0.041 mL, 0.16 mmol) and concentrated to dryness to afford product **4-8,** as a yellow solid, which was carried onto the next step without purification. MS [M+H]⁺ = 438.2.

### Step 6. 3-(2-(1-benzylpiperidin-4-yl)-5-oxo-SH-pyrrolo[3,4-b]pyridin-6(7H)-yl)dihydro-2H-pyran-2,6(3H)-dione (4-9):

2-(2-(1-benzylpiperidin-4-yl)-5-oxo-5H-pyrrolo[3,4-b]pyridin-6(7H)-yl)pentanedioic acid (**4-8**, 0.034 g, 0.078 mmol) was treated with acetyl chloride (1.0 mL, 14 mmol), DCE (1 mL), and triethylamine (0.023 mL, 0.16 mmol). The resulting mixture was stirred for 30 minutes at room temperature, and then stirred at 80 °C for 30 minutes. The reaction mixture was cooled to room temperature and concentrated to dryness to afford product **4-9** as an orange solid, which was carried onto the next step without purification. MS [M+H]⁺ = 420.4.

### Step 7. 5-amino-4-(2-(1-benzylpiperidin-4-yl)-5-oxo-5H-pyrrolo[3,4-b]pyridin-6(7H)-yl)-5-oxopentanoic acid and 5-amino-2-(2-(1-benzylpiperidin-4-yl)-5-oxo-SH-pyrrolo[3,4-b]pyridin-6(7H)-yl)-5-oxopentanoic acid (4-10 and 4-11)

A solution of 3-(2-(1-benzylpiperidin-4-yl)-5-oxo-5H-pyrrolo[3,4-b]pyridin-6(7H)-yl)dihydro-2H-pyran-2,6(3H)-dione (**4-9**, 0.033 g, 0.078 mmol) in 7MNH₃ in MeOH (1.50 mL, 10.5 mmol) was stirred at room temperature for 16 h. The resulting mixture was concentrated to dryness and then redissolved in DCM and sonicated for 10 minutes. The reaction mixture was concentrated to afford a mixture of regioisomers **4-10** and **4-11** as a light brown oil. MS [M+H]⁺= 437.4.

### Step 8. 3-(2-(1-benzylpiperidin-4-yl)-5-oxo-5,7-dihydro-6H-pyrrolo[3,4-b]pyridin-6-yl)piperidine-2,6-dione (I-1)

To a solution of 5-amino-4-(2-(1-benzylpiperidin-4-yl)-5-oxo-5H-pyrrolo[3,4-b]pyridin-6(7H)-yl)-5-oxopentanoic acid and 5-amino-2-(2-(1-benzylpiperidin-4-yl)-5-oxo-5H-pyrrolo[3,4-b]pyridin-6(7H)-yl)-5-oxopentanoic acid (**4-10** and **4-11,** 34 mg, 0.078 mmol) in DMF (1 mL) was added DMAP (0.95 mg, 7.80 µmol), CDI (37.9 mg, 0.234 mmol), and DIPEA (0.041 ml, 0.234 mmol) and the reaction mixture was stirred at room temperature for 4 h. An additional amount of CDI (14 mg, 0.086 mmol) and DIPEA (0.20 mL, 1.15 mmol) were added and stirring was continued at 100 °C for 16 h. CDI (0.014 g, 0.086 mmol) and DIPEA (0.082 mL, 0.47 mmol) were again added and the reaction was stirring was continued at 80 °C for 4 h. Additional CDI (0.014 g, 0.086 mmol) and DIPEA (0.082 mL, 0.47 mmol) were added and the resulting mixture was stirred at 100 °C for 16 h. The reaction mixture was cooled to room temperature, filtered, and diluted with MeCN (3 mL). The crude material was purified by mass triggered acidic reverse phase HPLC (eluting with 5-20% MeCN in H₂O with 0.1% formic acid as modifier) to afford **I-1,** (3.9 mg, 9.3 µmol, 12 % yield) as a beige solid. MS [M+H]⁺= 419.4. ¹H NMR (400 MHz, Acetonitrile-*d₃*) δ 8.07 (s, 1H), 7.90 (d, *J* = 8.0 Hz, 1H), 7.33 - 7.18 (m, 6H), 5.03 (dd, *J* = 13.4, 5.2 Hz, 1H), 4.29 (d, *J* = 17.2 Hz, 1H), 4.21 (d, *J* = 17.2 Hz, 1H), 3.57 (s, 2H), 2.98 (d, *J* = 11.5 Hz, 2H), 2.84 - 2.74 (m, 2H), 2.71 (dd, *J* = 13.2, 5.2 Hz, 1H), 2.68 - 2.60 (m, 2H), 2.33 (qd, *J* = 13.2, 4.9 Hz, 3H), 2.23 - 2.15 (m, 2H), 2.09 - 1.99 (m, 1H).

### Example 5: 3-(5-(1-benzylpiperidin-4-yl)-4-methyl-1-oxoisoindolin-2-yl)piperidine-2,6-dione (I-5)

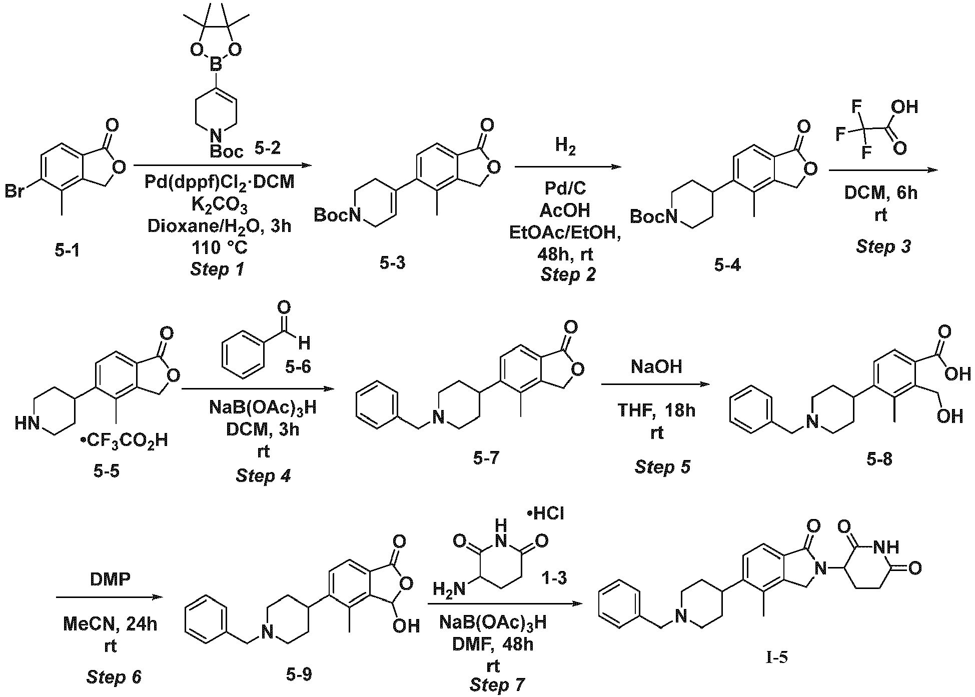

### Step 1. tert-butyl 4-(4-methyl-1-oxo-1,3-dihydroisobenzofuran-5-yl)-3,6-dihydropyridine-1(2H)-carboxylate (5-3)

To a microwave vial with a stir bar containing 5-bromo-4-methylisobenzofuran-1(3H)-one (**5-1**, 311.7 mg, 1.373 mmol), was added tert-butyl 4-(4,4,5,5-tetramethyl-1,3,2-dioxaborolan-2-yl)-3,6-dihydropyridine-1(2H)-carboxylate (**5-2**, 466.9 mg, 1.510 mmol), potassium carbonate (474 mg, 3.43 mmol), and Pd(dppf)Cl₂·DCM (57 mg, 0.069 mmol) and then placed under an atmosphere of nitrogen. Dioxane (3 mL) and Water (0.33 mL) were then added and the reaction was sparged with nitrogen gas for 5 minutes. The reaction mixture was then placed in a microwave reactor and heated at 110 °C for 3 hours. The resulting solution was filtered through Celite^{®} and the pad was washed with ethyl acetate. The solution was then diluted with ethyl acetate (140 mL) and washed with water (30 mL), saturated sodium bicarbonate solution (30 mL), and brine (20 mL). The organic extract was then dried over magnesium sulfate, filtered, and concentrated to afford a crude material. The crude product was diluted with dichloromethane and purified by column chromatography (ISCO, 40 g SiO₂, eluting with Hexane/Ethyl acetate, 0-70% over 16 minutes) to afford **5-3** as a white solid (395 mg, 1.16 mmol, 85% yield). MS [M+H]⁺ = 330.4. ¹H NMR (400 MHz, Methylene Chloride-*d₂*) δ 7.66 (d, *J* = 7.8 Hz, 1H), 7.28 (d, *J* = 7.8 Hz, 1H), 5.63 (tt, *J* = 3.3, 1.6 Hz, 1H), 5.23 (s, 2H), 4.05 (q, *J* = 2.8 Hz, 2H), 3.63 (t, *J* = 5.6 Hz, 2H), 2.35 (ttd, *J* = 5.6, 2.7, 1.9 Hz, 2H), 2.24 (s, 3H), 1.48 (s, 9H).

### Step 2. tert-butyl 4-(4-methyl-1-oxo-1,3-dihydroisobenzofuran-5-yl)piperidine-1-carboxylate (5-4)

In a reaction vial tert-butyl 4-(4-methyl-1-oxo-1,3-dihydroisobenzofuran-5-yl)-3,6-dihydropyridine-1(2H)-carboxylate (**5-3**, 395.3 mg, 1.200 mmol) was dissolved in EtOAc (6 mL) and EtOH (2 mL) containing a drop of acetic acid. Nitrogen gas was then bubbled through the solution for 10 minutes with a 16 gauge metal needle. Palladium on carbon (128.3 mg, 0.121 mmol) was then quickly added to the reaction vial and resealed. The solution was bubbled with nitrogen gas again for 10 minutes. Hydrogen gas was bubbled through the solution for 10 minutes and the reaction vial was then placed under an atmosphere of hydrogen gas using a balloon. The reaction mixture was allowed to stir vigorously overnight. The balloon was replaced with a nitrogen line and nitrogen gas was bubbled through the reaction for 5 minutes. The solution was then opened to air and nitrogen was bubbled through the solution for an additional 10 minutes. The reaction mixture was filtered through Celite^{®}, the pad washed with ethyl acetate, and the filtrate concentrated to afford **5-4** as a white solid (391.4 mg, 1.075 mmol, 90% yield). MS [M-*t*Bu+H]⁺ = 276.3. ¹H NMR (400 MHz, Methylene Chloride-*d₂*) δ 7.72 (d, *J* = 8.0 Hz, 1H), 7.43 (d, *J* = 8.0 Hz, 1H), 5.31 - 5.23 (m, 2H), 4.40 - 4.22 (m, 2H), 3.05 (tt, *J* = 11.9, 3.6 Hz, 1H), 2.87 (ddd, *J* = 13.3, 12.1, 2.8 Hz, 2H), 2.34 (s, 3H), 1.87 - 1.63 (m, 4H), 1.50 (s, 9H).

### Step 3. 4-methyl-5-(piperidin-4-yl)isobenzofuran-1(3H)-one trifluoroacetate (5-5)

To a reaction vial with a stir bar containing tert-butyl 4-(4-methyl-1-oxo-1,3-dihydroisobenzofuran-5-yl)piperidine-1-carboxylate (**5-4**, 391 mg, 1.18 mmol) dissolved in DCM (6 mL) was added TFA (0.5 mL, 6.5 mmol) and the resulting solution was allowed to stir overnight at room temperature. The reaction mixture was concentrated and azeotroped with methanol and dichloromethane to afford the **5-5** as an off-white solid (473.8 mg, 1.372 mmol, quantitative). MS [M+H]⁺ = 232.3. ¹H NMR (400 MHz, DMSO-*d₆*) δ 7.71 (d, *J* = 7.9 Hz, 1H), 7.40 (d, *J* = 8.0 Hz, 1H), 5.39 (s, 2H), 3.49 - 3.35 (m, 2H), 3.25 (tt, *J* = 10.0, 4.7 Hz, 1H), 3.16 - 3.01 (m, 2H), 2.30 (s, 3H), 1.94 - 1.71 (m, 4H).

### Step 4. 5-(1-benzylpiperidin-4-yl)-4-methylisobenzofuran-1(3H)-one (5-7)

To a reaction vial with a stir bar containing 4-methyl-5-(piperidin-4-yl)isobenzofuran-1(3H)-one (**5-5,** 408 mg, 1.18 mmol) and benzaldehyde (**5-6**, 0.24 mL, 2.4 mmol) dissolved in DCM (5 mL) was added in one portion sodium triacetoxyborohydride (626 mg, 2.95 mmol) and the resulting mixture was allowed to stir at room temperature open to air until the starting materials were consumed. The reaction solution was diluted with ethyl acetate (120 mL) and washed with 1:1 water/saturated sodium bicarbonate solution (30 mL) and brine (20 mL). The organic solution was then dried over magnesium sulfate, filtered, and concentrated. The crude product was diluted with dichloromethane and purified by column chromatography (ISCO, 40 g SiO₂, eluting with Heptane/Ethyl acetate with 0.1% triethylamine modifier, 0-100% over 16 minutes) to afford the desired product **5-7** as a white solid (203.2 mg, 0.601 mmol, 51 % yield). MS [M+H]⁺ = 322.3.

### Step 5. 4-(1-benzylpiperidin-4-yl)-2-(hydroxymethyl)-3-methylbenzoic acid (5-8)

To a reaction vial with a stir bar containing 5-(1-benzylpiperidin-4-yl)-4-methylisobenzofuran-1(3H)-one (**5-7,** 203.2 mg, 0.632 mmol) dissolved in THF (1 mL) was added sodium hydroxide (2 mL, 2 mmol) and the resulting mixture was allowed to stir at room temperature overnight. The solution was concentrated under reduced pressure and the crude material was diluted with water and acetonitrile and purified by mass-directed reversed phase column chromatography (Xbridge C18 OBD 5 um 30 x 50 mm column eluting with Water/Acetonitrile with 10mM NH₄OH 75mL/min 1.5mL injection and a gradient of 10-30% MeCN over a 3.5 min period). The desired peaks were collected and concentrated by under reduced pressure to afford the desired product **5-8** as a white solid (187.4 mg, 0.497 mmol, 79% yield). MS [M+H]⁺ = 340.4. ¹H NMR (400 MHz, DMSO-*d₆*) δ 7.40 - 7.18 (m, 6H), 7.01 (d, *J* = 8.0 Hz, 1H), 4.41 (s, 2H), 3.50 (s, 2H), 2.91 (d, *J* = 11.2 Hz, 2H), 2.75 - 2.64 (m, 1H), 2.24 (s, 3H), 2.07 (t, *J* = 12.8 Hz, 2H), 1.63 (dd, *J* = 8.1, 3.1 Hz, 4H).

### Step 6. 4-(1-benzylpiperidin-4-yl)-2-formyl-3-methylbenzoic acid (5-9)

A reaction vial with a stir bar containing 4-(1-benzylpiperidin-4-yl)-2-(hydroxymethyl)-3-methylbenzoic acid (**5-8**, 187.4 mg, 0.552 mmol) dissolved in MeCN (3 mL) was placed under an atmosphere of nitrogen and cooled to 0°C. DMP (351.4 mg, 0.828 mmol) was then added to the solution and the reaction mixture was allowed to slowly warm to room temperature and stir overnight. The solution was filtered through Celite^{®} and the pad washed with acetonitile. The filtrate was then concentrated. The crude material was diluted with water and acetonitrile and purified by mass-directed reversed phase column chromatography using (Xbridge C18 OBD 5um 30 x 50mm column eluting with Water/Acetonitrile with 10mM NH₄OH 75 mL/min 1.5mL injection and a gradient of 10-30% MeCN over a 3.5 min period). The desired peaks were collected and concentrated under reduced pressure to afford the desired product **5-9** as a pale yellow solid (13.3 mg, 0.038 mmol, 6.9% yield). MS [M+H]⁺ = 338.3. ¹H NMR (400 MHz, DMSO-*d₆*) δ 7.98 (d, *J* = 8.5 Hz, 1H), 7.66 - 7.47 (m, 2H), 7.42 - 7.17 (m, 5H), 6.66 (d, *J* = 8.4 Hz, 1H), 3.52 (s, 2H), 2.90 (d, *J* = 36.3 Hz, 2H), 2.36 (s, 3H), 2.18 - 2.08 (m, 2H), 1.84 - 1.59 (m, 4H).

### Step 7. 3-(5-(1-benzylpiperidin-4-yl)-4-methyl-1-oxoisoindolin-2-yl)piperidine-2,6-dione (I-5)

To a reaction vial with a stir bar containing 4-(1-benzylpiperidin-4-yl)-2-formyl-3-methylbenzoic acid (**5-9**, 13.1 mg, 0.039 mmol) and 3-aminopiperidine-2,6-dione hydrochloride (**1-3**, 13.4 mg, 0.081 mmol) dissolved in DMF (0.6 mL) was added sodium triacetoxyborohydride (20.6 mg, 0.097 mmol) and the resulting mixture was allowed to stir at room temperature overnight and open to air. The reaction solution was then diluted with ethyl acetate and filtered through Celite^{®}. The filtrate was washed multiple times with ethyl acetate and the combined organic extracts were then concentrated. The crude product was diluted with dichloromethane and purified by column chromatography (ISCO, 12 g SiO₂ Gold, eluting with Dichloromethane/(3:1) Ethyl acetate:Ethanol with 0.1% triethylamine modifier, 5-100% over 17 minutes) to afford the desired product **I-5** as a white solid (7.8 mg, 0.018 mmol, 45% yield). MS [M+H]⁺ = 432.4. ¹H NMR (400 MHz, DMSO-*d₆*) δ 10.97 (s, 1H), 7.52 (d, *J* = 7.9 Hz, 1H), 7.41 (d, *J* = 8.0 Hz, 1H), 7.34 (d, *J* = 4.4 Hz, 4H), 7.28-7.23 (m, 1H), 5.11 (dd, *J* = 13.3, 5.1 Hz, 1H), 4.50 - 4.14 (m, 2H), 3.58-3.49 (m, 2H), 3.03 - 2.74 (m, 3H), 2.70 - 2.52 (m, 3H), 2.48 - 2.36 (m, 1H), 2.26 (s, 3H), 2.19-2.05 (m, 1H), 2.04 - 1.94 (m, 1H), 1.75 - 1.64 (m, 4H).

### Example 6: 3-(6-(1-benzylpiperidin-4-yl)-3-oxo-1,3-dihydro-2H-pyrrolo[3,4-c]pyridin-2-yl)piperidine-2,6-dione (I-2)

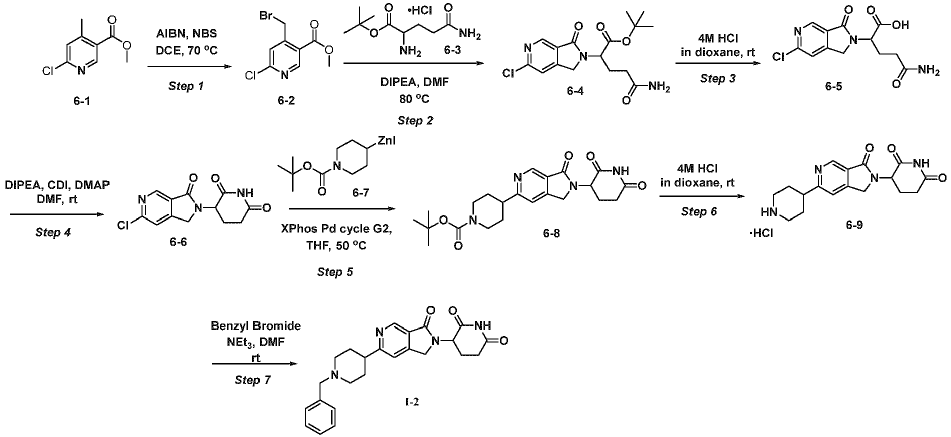

### Step 1: Methyl 4-(bromomethyl)-6-chloronicotinate (6-2)

To a solution of methyl 6-chloro-4-methylnicotinate (**6-1**, 0.50 g, 2.7 mmol) in dichloroethane (6.7 mL) was added NBS (0.527 g, 2.96 mmol) and AIBN (0.088 g, 0.539 mmol). A findenser was placed on top of the reaction mixture and it was stirred at 70 °C overnight. The reaction mixture was quenched with saturated sodium thiosulfate and extracted with dichloromethane (x3). The combined organic layers were passed through a phase separator and concentrated onto Celite^{®}. The Celite^{®} residue was purified by silica gel chromatography using 0-50% ethyl acetate in heptane to afford crude product **6-2** (494 mg, 1.87 mmol, 69 % yield) as a white solid; MS [M+H]⁺ = 263.8

### Step 2: tert-butyl 5-amino-2-(6-chloro-3-oxo-1H-pyrrolo[3,4-c]pyridin-2(3H)-yl)-5-oxopentanoate, Methyl 6-chloro-4-methylnicotinate (6-4)

Methyl 4-(bromomethyl)-6-chloronicotinate (**6-2**, 0.494 g, 1.868 mmol) and tert-butyl 2,5-diamino-5-oxopentanoate hydrochloride (**6-3**, 0.669 g, 2.80 mmol) were dissolved in DMF (2.3 mL) and DIPEA (1.63 mL, 9.34 mmol) was added. The resulting mixture was stirred at 80 °C for 12 h. The reaction was cooled to rt, quenched with water and extracted three times with dichloromethane. The combined organic extracts were passed through a phase separator and concentrated onto Celite^{®}. The crude product on the Celite^{®} residue was purified by silica gel chromatography eluting with 0-50% ethyl acetate in heptane and 0-10% methanol in dichloromethane to afford product **6-4** (444 mg, 1.23 mmol, 67 % yield) as a yellow oil. MS [M-tBu+H]⁺ = 298.1; ¹H NMR (400 MHz, Chloroform-d) δ 8.86 (d, *J* = 0.9 Hz, 1H), 7.50 (d, *J* = 0.9 Hz, 1H), 5.66 (s, 1H), 5.27 (s, 1H), 5.07 - 4.95 (m, 1H), 4.79 (d, *J* = 18.4 Hz, 1H), 4.52 (d, *J* = 18.4 Hz, 1H), 2.45 - 2.17 (m, 4H), 1.48 (s, 9H).

### Step 3: 5-amino-2-(6-chloro-3-oxo-1H-pyrrolo[3,4-c]pyridin-2(3H)-yl)-5-oxopentanoic acid hydrochloride (6-5)

To a solution of tert-butyl 5-amino-2-(6-chloro-3-oxo-1H-pyrrolo[3,4-c]pyridin-2(3H)-yl)-5-oxopentanoate (**6-4**, 0.153 g, 0.432 mmol) in dioxane (4.3 mL) was added 4M HCl in dioxane (0.43 mL, 1.7 mmol). The resulting mixture was stirred at room temperature for 72 h. The reaction mixture was then concentrated to dryness to afford product **6-5** as an orange solid, which was carried onto the next step without purification. MS [M+H]⁺ = 298.1.

### Step 4: 3-(6-chloro-3-oxo-1H-pyrrolo[3,4-c]pyridin-2(3H)-yl)piperidine-2,6-dione (6-6)

To a solution of 5-amino-2-(6-chloro-3-oxo-1H-pyrrolo[3,4-c]pyridin-2(3H)-yl)-5-oxopentanoic acid (6-5, 129 mg, 0.432 mmol) in DMF (4.3 mL) was added CDI (700 mg, 4.32 mmol) and DIPEA (1.5 mL, 8.6 mmol) and the resulting mixture was stirred at r.t. for 48 hrs. The reaction mixture was filtered through a syringe filter and concentrated to dryness. The crude material was purified by silica gel chromatography eluting with ethyl acetate and 10% triethylamine in ethyl acetate to afford product 6-6 (123 mg, 0.440 mmol, quantitative) as an orange oil. MS [M+H]⁺ = 280.1.

### Step 5: tert-butyl 4-(2-(2,6-dioxopiperidin-3-yl)-3-oxo-2,3-dihydro-1H-pyrrolo[3,4-c]pyridin-6-yl)piperidine-1-carboxylate (6-8)

3-(6-chloro-3-oxo-1H-pyrrolo[3,4-c]pyridin-2(3H)-yl)piperidine-2,6-dione (**6-6,** 0.073 g, 0.26 mmol) and XPhos Pd cycle G2 (0.031 g, 0.039 mmol) were suspended in THF (1.3 mL) and the resulting mixture was evacuated and backfilled with nitrogen three times. 0.5M (1-(tert-butoxycarbonyl)piperidin-4-yl)zinc(II) iodide (**6-7**) in THF (1.3 mL, 0.65 mmol) was added. The reaction mixture was stirred at 50 °C for 1 h. The reaction mixture was then cooled to room temperature, quenched with saturated ammonium chloride, and extracted four times with ethyl acetate. The combined organic extracts were passed through a phase separator and concentrated onto Celite^{®}. The crude product on the Celite^{®} residue was purified with silica gel chromatography eluting with 0-100% ethyl acetate in heptane and then 0-10% methanol in DCM to afford **6-8** (31.2 mg, 0.073 mmol, 28 % yield) as a yellow solid. MS [M+H]⁺ = 429.3

### Step 6: 3-(3-oxo-6-(piperidin-4-yl)-1H-pyrrolo[3,4-c]pyridin-2(3H)-yl)piperidine-2,6-dione hydrochloride (6-9)

To a suspension of tert-butyl 4-(2-(2,6-dioxopiperidin-3-yl)-3-oxo-2,3-dihydro-1H-pyrrolo[3,4-c]pyridin-6-yl)piperidine-1-carboxylate (**6-8,** 0.0312 g, 0.073 mmol) in dioxane (0.73 mL) was added 4M HCl in dioxane (0.1 mL, 0.4 mmol) and the resulting mixture was stirred at rt for 6 h. The reaction mixture was concentrated to dryness to afford product **6-9** as an orange oil, which was carried onto the next step without purification. MS [M+H]⁺ = 329.0.

### Step 7: 3-(6-(1-benzylpiperidin-4-yl)-3-oxo-1,3-dihydro-2H-pyrrolo[3,4-c]pyridin-2-yl)piperidine-2,6-dione (I-2)

To a suspension of 3-(3-oxo-6-(piperidin-4-yl)-1H-pyrrolo[3,4-c]pyridin-2(3H)-yl)piperidine-2,6-dione hydrochloride (**6-9,** 24 mg, 0.073 mmol) in DMF (0.73 mL) was added triethylamine (51 µL, 0.37 mmol) and the resulting mixture was stirred at rt for 15 minutes. Benzyl bromide (10 µL, 0.088 mmol) was then added and the mixture was stirred at rt for 2 h. The reaction mixture was then concentrated and redissolved in DMF. Crude material was purified by mass triggered reverse phase HPLC (eluting with 5-20% MeCN in water with 0.1% formic acid as modifier) to afford **I-2** (6.31 mg, 0.013 mmol, 17 % yield) as a cream solid. MS [M+H]⁺ = 419.3. ¹H NMR (400 MHz, DMSO-*d₆*) δ 11.00 (s, 1H), 8.86 (d, *J* = 1.0 Hz, 1H), 7.59 (d, *J* = 1.1 Hz, 1H), 7.35 - 7.21 (m, 5H), 5.12 (dd, *J* = 13.3, 5.1 Hz, 1H), 4.51 (d, *J* = 18.3 Hz, 1H), 4.36 (d, *J* = 18.3 Hz, 1H), 3.51 (s, 2H), 2.96 - 2.90 (m, 2H), 2.88 - 2.77 (m, 2H), 2.63 - 2.54 (m, 1H), 2.45 - 2.36 (m, 1H), 2.09 (td, *J* = 11.2, 3.4 Hz, 2H), 2.04 - 1.95 (m, 1H), 1.86 - 1.77 (m, 4H).

### Example 7: 3-(4-fluoro-5-(1-(((lr,4r)-4-methoxycyclohexyl)methyl)piperidin-4-yl)-1-oxoisoindolin-2-yl)piperidine-2,6-dione HCOOH salt (I-18)

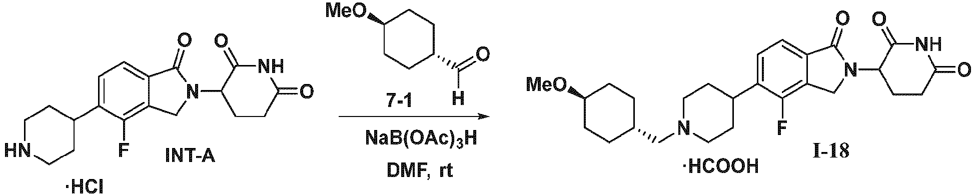

Aldehyde **7-1** was made according to the procedure described in WO2019/038717.

(1r,4r)-4-methoxycyclohexane-1-carbaldehyde **7-1** (67 mg, 0.47 mmol) and **INT-A** (90 mg, 0.24 mmol) were dissolved in DMF (1.6 mL) and stirred at room temperature for 15 minutes. NaBH(OAc)₃ (100 mg, 0.471 mmol) was added and the reaction mixture was stirred for 2 h at room temperature. The reaction was quenched with 50% saturated aqueous sodium bicarbonate. The aqueous layer was extracted three times with 4:1 dichloromethane:isopropanol. The organic layers were combined, passed through a phase separator and concentrated onto Celite^{®}. The crude material was purified by silica gel chromatography eluting with 0-100% EtOAc:EtOH:TEA (v/v/v = 3:1:0.04) in heptane. Fractions containing product were concentrated and further purified by RP HPLC (5-20% ACN in water with 0.1% formic acid as modifier; Waters XBridge C18 OBD 30 x 50 mm). Fractions containing desired product were concentrated to afford the HCOOH salt of **I-18** (8.8 mg, 0.014 mmol, 6% yield) as a white solid. MS [M+H] ⁺ = 472.3. ¹H NMR (400 MHz, CDCl₃) δ 8.13 (s, 1H), 7.71 (d, *J* = 7.8 Hz, 1H), 7.46 (t, *J* = 7.1 Hz, 1H), 5.24 (dd, *J* = 13.1, 5.1 Hz, 1H), 4.55 (d, *J* = 16.1 Hz, 1H), 4.40 (d, *J* = 16.1 Hz, 1H), 3.78 (d, *J* = 11.2 Hz, 2H), 3.37 (s, 3H), 3.24 - 3.08 (m, 2H), 3.00 - 2.71 (m, 6H), 2.49 - 2.35 (m, 3H), 2.30 - 2.21 (m, 1H), 2.18 - 2.11 (m, 2H), 2.05 - 1.93 (m, 4H), 1.85 - 1.77 (m, 1H), 1.29 - 1.10 (m, 4H).

### Example 8: 3-(4-chloro-5-(1-(((1r,4r)-4-methoxycyclohexyl)methyl)piperidin-4-yl)-1-oxoisoindolin-2-yl)piperidine-2,6-dione (I-17)

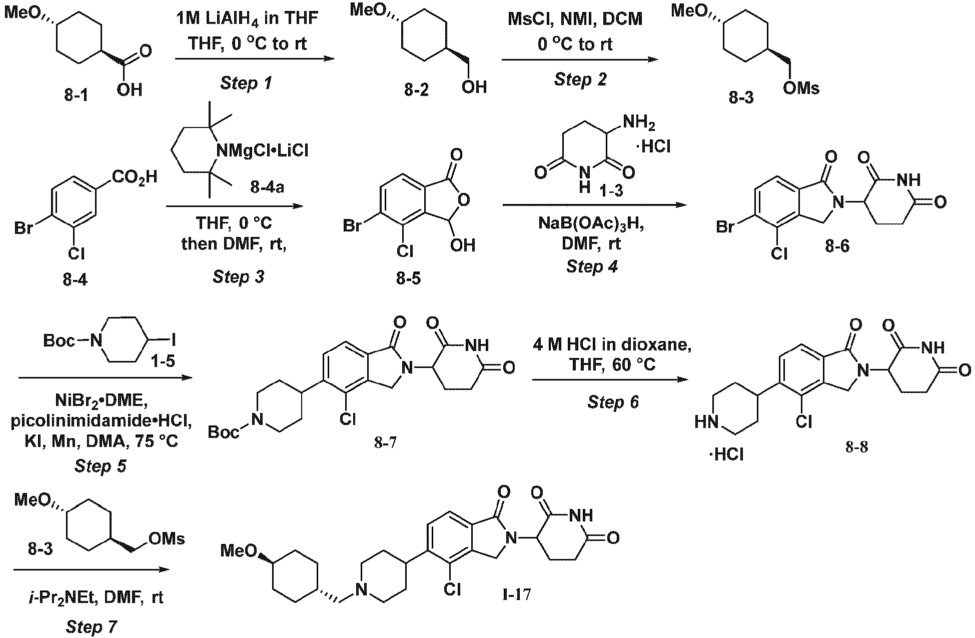

### Step 1. ((1R,4R)-4-Methoxycyclohexyl)methanol (8-2)

To a solution of *trans-*4-methoxycyclohexane-1-carboxylic acid (**8-1,** 1.00 g, 6.32 mmol) in dry THF (10 mL), under an atmosphere of nitrogen and cooled using an ice bath, was added lithium aluminum hydride 1M in THF (9.5 mL, 9.5 mmol) dropwise. The resulting mixture was stirred using an ice bath for 2 h, then allowed to warm to room temperature and stirred for 16 h. A solution of saturated aqueous potassium sodium tartrate (Rochelle's Salt) (150 mL) was then added with stirring. The reaction mixture was extracted with DCM (x4) and the combined organic phases were passed through a phase separating column and concentrated to dryness to afford **8-2** (903 mg, 6.26 mmol, 99% yield) as a colorless oil. The product was carried onto the next step without purification. ¹H NMR (400 MHz, DMSO-*d₆*) δ 4.36 (s, 1H), 3.21 (s, 3H), 3.19 (d, *J* = 6.3 Hz, 2H), 3.02 (tt, *J =* 10.7, 4.1 Hz, 1H), 2.07-1.91 (m, 2H), 1.80-1.66 (m, 2H), 1.36-1.21 (m, 1H), 1.11-0.96 (m, 2H), 0.87 (tdd, *J* = 13.2, 11.6, 3.1 Hz, 2H).

### Step 2. ((1r,4r)-4-methoxycyclohexyl)methyl methanesulfonate (8-3)

To a solution of **8-2** (220 mg, 1.53 mmol), DIPEA (0.53 mL, 3.1 mmol), 1-methyl-1H-imidazole (0.24 mL, 3.0 mmol) in DCM (3 mL) was added methanesulfonyl chloride (0.18 mL, 2.3 mmol) dropwise under a stream of nitrogen. The reaction mixture was stirred at rt overnight. The reaction mixture was diluted with DCM (30 mL total). Organics were washed with 1M aq. HCl (x3), followed by a sat. aq. solution of NaHCOs (x2) and brine (x1). Organics were passed through a phase separating column, solvent collected and evaporated off to afford **8-3** (329 mg, 1.48 mmol, 97% yield) as a colorless oil.
¹H NMR (400MHz, DMSO-*d₆*): δ 4.01 (d, *J* = 6.4 Hz, 2H), 3.22 (s, 3H), 3.15 (s, 3H), 3.10 - 2.99 (m, 1H), 2.05 - 1.95 (m, 2H), 1.81 - 1.70 (m, 2H), 1.69-1.57 (m, 1H), 1.16 - 0.94 (m, 4H).

### Step 3. 5-bromo-4-chloro-3-hydroxyisobenzofuran-1(3H)-one (8-5):

To a solution of 4-bromo-3-chlorobenzoic acid (**8-4,** 1000 mg, 4.25 mmol) in THF (15 mL) under an atmosphereof nitrogen was added TMPMgCl•LiCl (**8-4a,** 1M in THF/PhMe, 9.3 mL, 9.3 mmol) dropwise at 0 °C. The reaction mixture was stirred for 1.5 h at 0 °C. DMF (0.50 mL, 6.4 mmol) was then added dropwise and the reaction mixture was allowed to reach rt and stirred for additional 2 h. The reaction mixture was quenched with 1 M aq. HCl (20 mL) at 0 °C and extracted with DCM (x3). The combined organic phases were concentrated to dryness. The crude product was purified by silica gel chromatography eluting with 0 to 40% EtOAc in heptane to afford **8-5** (611 mg, 2.32 mmol, 55 % yield) as a white solid. MS [M+H]⁺ = 263.0. ¹H NMR (400 MHz, Methylene Chloride-*d₂*) δ 7.89 (d, *J* = 8.0 Hz, 1H), 7.62 (d, *J* = 8.0 Hz, 1H), 6.64 (s, 1H), 4.05 (s, 1H).

### Step 4. 3-(5-bromo-4-chloro-1-oxoisoindolin-2-yl)piperidine-2,6-dione (8-6):

To a solution of **8-5** (611 mg, 2.32 mmol) and 3-aminopiperidine-2,6-dione HCl (**1-3,** 573 mg, 3.48 mmol) in DMF (5 mL) was added NaBH(OAc)₃ (1229 mg, 5.80 mmol) and the reaction mixture was stirred overnight at rt. The reaction mixture was poured into a conical flask containing cold H₂O (30 mL). The reaction mixture was filtered and the solid was washed with minimal amount of cold H₂O and then Et₂O to afford **8-6** (362 mg, 0.998 mmol, 43% yield) as a grey solid. MS [M+H]⁺ = 357.1. ¹H NMR (400 MHz, DMSO-*d₆*) δ 11.02 (s, 1H), 7.95 (d, *J* = 8.0 Hz, 1H), 7.65 (d, *J* = 8.1 Hz, 1H), 5.14 (dd, *J* = 13.3, 5.1 Hz, 1H), 4.54 (d, *J* = 17.9 Hz, 1H), 4.37 (d, *J* = 17.9 Hz, 1H), 3.02 - 2.84 (m, 1H), 2.66 - 2.55 (m, 1H), 2.48 - 2.37 (m, 1H), 2.06 - 1.97 (m, 1H).

### Step 5. tert-butyl 4-(4-chloro-2-(2,6-dioxopiperidin-3-yl)-1-oxoisoindolin-5-yl)piperidine-1-carboxylate (8-7):

To a stirred suspension of NiBr₂• (DME) (16 mg, 0.051 mmol), picolinimidamide HCl salt (8.0 mg, 0.051 mmol), KI (504 mg, 3.04 mmol) and manganese powder (278 mg, 5.06 mmol) under an atmosphere of nitrogen in DMA (1 mL) was added **8-6** (362 mg, 1.01 mmol) and tert-butyl 4-iodopiperidine-1-carboxylate (**1-5,** 473 mg, 1.52 mmol), dissolved in DMA (3 mL). The resulting mixture was then stirred vigorously at 75 °C for 24 hours under an atmosphere of nitrogen. The reaction mixture was filtered and filter was washed with minimal amount of MeCN. The obtained filtrate was concentrated (100 mbar, 40 °C) to a constant volume. Cold H₂O (20 mL) was added and formed brown precipitate was filtered, washed with H₂O, heptane, and dried in the vacuum oven. Obtained crude product **8-7** (359 mg) was used in the next step without further purification. MS [M+H]⁺ = 462.3.

### Step 6. 3-(4-chloro-1-oxo-5-(piperidin-4-yl)isoindolin-2-yl)piperidine-2,6-dione HCl salt (8-8):

To a stirred solution of crude **8-7** (359 mg) in THF (3 mL) was added 4 M hydrogen chloride in dioxane (1.5 mL, 6.0 mmol) and the reaction mixture was stirred for 24 hours a 60 °C. The reaction mixture was diluted with Et₂O and filtered. The precipitate was washed with Et₂O (x4) and then dried on a high vac to afford crude **8-8** (103 mg) as a white solid. Obtained crude product was used in the next step without further purification. MS [M+H]⁺ = 362.3.

### Step 7. 3-(4-chloro-5-(1-(((1r,4r)-4-methoxycyclohexyl)methyl)piperidin-4-yl)-1-oxoisoindolin-2-yl)piperidine-2,6-dione (I-17):

To a solution of crude **8-8** (103 mg), DIPEA (0.17 mL, 0.95 mmol) in DMF (1 mL) was added **8-3** (54.5 mg, 0.245 mmol) in one portion and the reaction mixture was stirred overnight at at room temperature. The reaction mixture was concentrated to dryness. Crude product was purified by silica gel chromatography eluting with 0 to 100% EtOAc:EtOH:Et₃N (v/v/v = 75:25: 1) in DCM to afford **1-17** (3.2 mg, 6.5 µmol, 0.6% yield over 3 steps) as a white powder. MS [M+H]⁺ = 488.4. ¹H NMR (400 MHz, DMSO-*d₆*) δ 11.00 (s, 1H), 7.69 (d, *J* = 7.9 Hz, 1H), 7.58 (d, *J* = 7.9 Hz, 1H), 5.12 (dd, *J* = 13.3, 5.1 Hz, 1H), 4.47 (d, *J* = 17.6 Hz, 1H), 4.30 (d, *J* = 17.6 Hz, 1H), 3.23 (s, 3H), 3.08 - 2.84 (m, 5H), 2.68 - 2.56 (m, 1H), 2.23 - 1.89 (m, 6H), 1.84 - 1.63 (m, 6H), 1.48 (s, 1H), 1.09 (q, *J* = 12.1 Hz, 2H), 0.94 - 0.78 (m, 2H). Missing protons are overlapping with residual DMSO or H₂O solvent peaks.

### Example 9: 3-(5-(1-benzyl-1,2,3,6-tetrahydropyridin-4-yl)-4-methoxy-1-oxoisoindolin-2-yl)piperidine-2,6-dione (I-20)

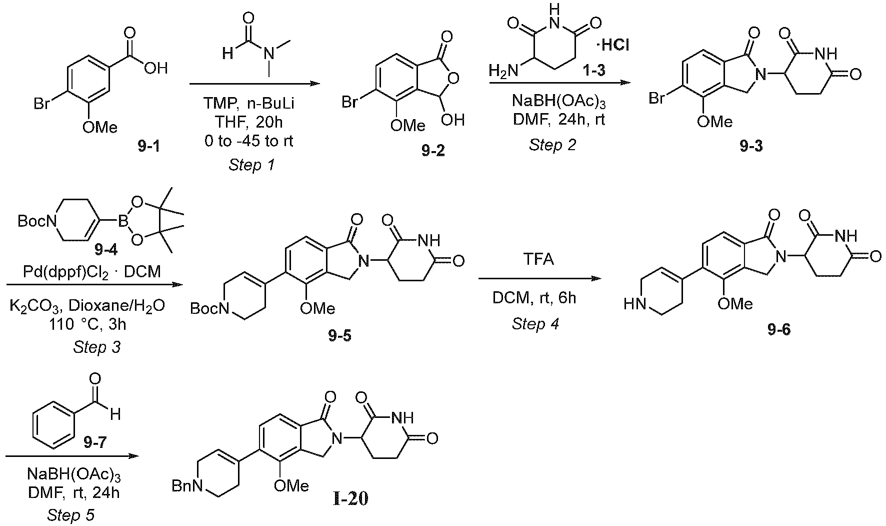

### Step 1: 5-bromo-3-hydroxy-4-methoxyisobenzofuran-1(3H)-one (9-2)

In a 200 mL round bottomed flask with stir bar, 2,2,6,6-tetramethylpiperidine (TMP, 4.00 mL, 23.7 mmol) was dissolved in THF (18 mL) and placed under nitrogen atmosphere. The solution was cooled to 0°C and n-butyllithium in hexanes (9 mL, 22.5 mmol) was added dropwise. The solution was then allowed to stir at 0°C for 30 minutes. Separately, 4-bromo-3-methoxybenzoic acid (**9-1,** 2.16 g, 9.33 mmol) was dissolved in THF (6 mL). The lithium 2,2,6,6-tetramethylpiperidine solution was then cooled to -45°C (using a dry ice/acetonitrile bath) and the benzoic acid solution was added dropwise. The resulting mixture was stirred at -45°C for 4 hours. DMF (1.10 mL, 14.2 mmol) was then added and the reaction mixture was allowed to warm to room temperature and stirred overnight. The solution was then cooled to 0°C, quenched with 3 M HCl (20 mL), and extracted with dichloromethane (3 × 60 mL). The organic phase was washed with 0.1 M HCl (2 × 20 mL) and brine (20 mL), dried over magnesium sulfate, filtered, and concentrated to afford a crude product as an orange solid. The crude product was diluted with dichloromethane and purified by column chromatography (ISCO, 80 g SiO₂, eluting with Heptane/Ethyl acetate, 0-80% over 20 minutes) to afford the product contaminated with impurities. The product was repurified by column chromatography (ISCO, 40 g SiO₂, eluting with Heptane/Ethyl acetate, 0-60% over 15 minutes) to afford the desired product **9-2** as a light orange solid (54.2 mg, 0.084 mmol, 0.90% yield): MS [M+H]⁺ = 259.0. ¹H NMR (400 MHz, DMSO-*d₆*) δ 8.33 (d, *J* = 8.5 Hz, 1H), 7.90 (d, *J* = 7.9 Hz, 1H), 7.42 (d, *J* = 7.9 Hz, 1H), 6.95 (d, *J* = 8.1 Hz, 1H), 4.09 (s, 3H).

### Step 2: 3-(5-bromo-4-methoxy-1-oxoisoindolin-2-yl)piperidine-2,6-dione (9-3)

In a reaction vial with stir bar, 5-bromo-3-hydroxy-4-methoxyisobenzofuran-1(3H)-one (**9-2**, 54.2 mg, 0.209 mmol) and 3-aminopiperidine-2,6-dione hydrochloride (**1-3,** 54.7 mg, 0.332 mmol) were dissolved in DMF (1 mL). Sodium triacetoxyborohydride (111 mg, 0.523 mmol) was added to the solution and the resulting mixture was allowed to stir at room temperature open to the air overnight. The reaction mixture was diluted with ethyl acetate and filtered through Celite^{®}. The filtrate was washed multiple times with ethyl acetate and the organic phase was then concentrated. The crude material was diluted with water and acetonitrile and purified by mass-directed reverse phase column (eluting with 15-40% ACN in water with 0.1% formic acid as modifier; Waters XBridge C18 OBD 30 x 50 mm). The desired peaks were collected and concentrated by vacuum to afford the desired product 9-3 as a white solid (16.2 mg, 0.046 mmol, 21.9 % yield): ¹H NMR (400 MHz, DMSO-*d₆*) δ 11.02 (s, 1H), 7.77 (d, *J* = 8.0 Hz, 1H), 7.36 (d, *J* = 7.9 Hz, 1H), 5.12 (dd, *J* = 13.3, 5.1 Hz, 1H), 4.87 - 4.42 (m, 2H), 4.00 (s, 3H), 3.00 - 2.85 (m, 1H), 2.61 (d, *J* = 17.7 Hz, 1H), 2.46 - 2.42 (m, 1H), 2.02 (ddd, *J* = 10.4, 5.4, 3.1 Hz, 1H)

### Step 3: tert-butyl 4-(2-(2,6-dioxopiperidin-3-yl)-4-methoxy-1-oxoisoindolin-5-yl)-3,6-dihydropyridine-1(2H)-carboxylate (9-5)

A microwave vial with stir bar was charged with 3-(5-bromo-4-methoxy-1-oxoisoindolin-2-yl)piperidine-2,6-dione (**9-3,** 16 mg, 0.046 mmol), tert-butyl 4-(4,4,5,5-tetramethyl-1,3,2-dioxaborolan-2-yl)-3,6-dihydropyridine-1(2H)-carboxylate (**9-4,** 17.0 mg, 0.055 mmol), potassium carbonate (16.5 mg, 0.119 mmol), and Pd(dppf)Cl₂ · DCM (3.75 mg, 4.59 µmol) and then placed under an atmosphere of nitrogen. Dioxane (0.45 mL) and water (0.05 mL) were then added and the reaction mixture was sparged with nitrogen gas for 5 minutes. The resulting mixture was then placed in a microwave reactor and heated at 110 °C for 2 hours. The reaction mixture was filtered through Celite^{®} and washed with ethyl acetate. The filtrate was then diluted with ethyl acetate (150 mL) and the organic phase was washed with water (30 mL), saturated sodium bicarbonate solution (30 mL), and brine (20 mL). The organic phase was then dried over magnesium sulfate, filtered, and concentrated to afford the crude product **9-5,** which was taken onto the next step without purification.

### Step 4: 3-(4-methoxy-1-oxo-5-(1,2,3,6-tetrahydropyridin-4-yl)isoindolin-2-yl)piperidine-2,6-dione (9-6)

In a reaction vial with stir bar, tert-butyl 4-(2-(2,6-dioxopiperidin-3-yl)-4-methoxy-1-oxoisoindolin-5-yl)-3,6-dihydropyridine-1(2H)-carboxylate (**9-5,** 20.1 mg, 0.044 mmol) was dissolved in DCM (1 mL). TFA (0.03 mL, 0.4 mmol) was added and the resulting mixture was allowed to stir overnight at room temperature. Incomplete conversion to the desired product observed after 16 hours. Additional TFA (0.03 mL) was added and stirring was continued overnight at room temperature. The reaction mixture was concentrated to afford the crude product, which was then azeotroped with methanol and dichloromethane to afford the crude product **9-6** as a brown, viscous liquid. The product was taken forward to the next step onto the next step without purification. MS [M+H]⁺ = 356.3.

### Step 5: 3-(5-(1-benzyl-1,2,3,6-tetrahydropyridin-4-yl)-4-methoxy-1-oxoisoindolin-2-yl)piperidine-2,6-dione (I-20)

In a reaction vial with stir bar, 3-(4-methoxy-1-oxo-5-(1,2,3,6-tetrahydropyridin-4-yl)isoindolin-2-yl)piperidine-2,6-dione (**9-6,** 17.7 mg, 0.044 mmol) and benzaldehyde (0.02 mL, 0.2 mmol) were dissolved in DMF (0.5 mL). Sodium triacetoxyborohydride (65.0 mg, 0.307 mmol) was added in one portion and the resulting mixture was allowed to stir at room temperature open to air until complete consumption of starting material was observed. The reaction mixture was then filtered through Celite^{®} and washed with ethyl acetate. The filtrate was then concentrated under reduced pressure. The crude product was dissolved with acetonitrile and purified by reverse-phase column chromatography (eluting with 10-30% ACN in water with 0.1% formic acid as modifier; Waters XBridge C18 OBD 30 x 50 mm). The desired peaks were collected and concentrated under reduced pressure. The obtained product was diluted with dichloromethane and purified by column chromatography (ISCO, 4 g SiO₂, eluting with dichloromethane/Isopropanol, 0-100% over 12 minutes) to afford **I-20** as a white solid (2.9 mg, 6.4 µmol, 14% yield): MS [M+H]⁺ = 446.4. ¹H NMR (400 MHz, DMSO-*d₆*) δ 10.99 (s, 1H), 7.45 - 7.19 (m, 7H), 5.93 - 5.81 (m, 1H), 5.10 (dd, *J* = 13.3, 5.1 Hz, 1H), 4.68 - 4.35 (m, 2H), 3.84 (s, 3H), 3.61 (s, 2H), 3.08 (q, *J* = 2.9 Hz, 2H), 2.92 (ddd, *J* = 18.0, 13.5, 5.3 Hz, 1H), 2.64 (d, *J* = 5.6 Hz, 2H), 1.99 (dd, *J* = 9.5, 4.4 Hz, 1H). Missing protons are overlapping with DMSO solvent peak.

### Biological Assays and Data

The activity of a compound according to the present invention can be assessed by the following in vitro methods.

### Example 10: Prolabel Quantification of IKZF1, IKZF2 or GSPT1 protein levels in 293GT cells

The Prolabel system from DiscoverX was used to develop high-throughput and quantitative assays to measure changes in IKZF1, IKZF2 and GSPT1 protein levels in response to compounds. The prolabel tag was derived from the alpha fragment of beta galactosidase and has the following protein sequence: mssnslavvlqrrdwenpgvtqlnrlaahppfaswrnseeartdrpsqqlrslnge. The complementary fragment of betagalactosidase (from DiscoverX), is added to the prolabel tag to form an active beta galactosidase enzyme whose activity can be precisely measured. In this way, the levels of a fusion protein with the prolabel tag can be quantified in cell lysates.

Lentiviral vectors, based on the Invitrogen pLenti6.2/V5 DEST backbone, were constructed that placed the prolabel tag upstream of IKZF1, IKZF2 or GSPT1 and expressed the fusion protein from a CMV promoter.

To ensure moderate and consistent expression of the prolabel fusion proteins across all cells in the population, stable cell lines were constructed from cells expressing a single copy of the construct. Lentivirus packaged with the constructs was made using the Virapower kit from Invitrogen. Strongly adherent 293GT cell, GripTite 293 MSR cells from Thermo Fisher Scientific (Catalog number: R79507), were infected with the virus at low multiplicity of infection and selected by 5 µg/mL blasticidin for 2 weeks.

The levels of prolabel tagged fusion proteins in compound treated cell lines were measured as follows:
Day 1, Cells were diluted to 1.0 × 10⁶ cells/ml in normal growth medium. 17.5 µL of cells were plated in each well of a solid white 384 well plate. Plates were incubated overnight in a 37 °C tissue culture incubator.
Day 2, Serial dilutions of compounds were made in 384 well plates from 10 mM stocks. 15 µL of DMSO was added to each well of a 384 well plate. In the first column, 15µL of stock compound was added. The solution was mixed and 15 µL was transferred to the next column. This was repeated until 20 two-fold dilutions were prepared. 2.5 µL of diluted compounds were transferred into 60 µL of cell culture medium in another 384 well plate, and mixed well. 2.5 µL of this mixture was added to the plated cells. The final DMSO concentration was 0.5% and the highest concentration of compound was 50 µM. Plates were incubated overnight (e.g., about 14 h, 18 h, or 24 h) in a 37 °C tissue culture incubator.
Day 3, Plates were removed from the incubator and allowed to equilibrate at rt for 30 minutes. Prolabel substrate (DiscoverX PathHunter Prolabel Detection Kit, User manual: 93-0180) was added as described by the manufacturers protocols. Plates were incubated at rt for three hours and luminescence was read using an Envision reader (Perkin Elmer) Data was analyzed and visualized using the Spotfire software package.

Table 14 shows Helios (IKZF2) and Ikaros (IKZF1) degradation activity of compounds of the invention in Pro-label assays in 293GT cells, (% degradation is at 10 µM). Pomalidomide was tested as the control.

**TABLE 14:**

| **Cmpd No.** | **Compound Structure** | **IKZF1 Degradation [AC50 uM (%)]** | **IKZF2 Degradation [AC50 uM (%)]** |
|---|---|---|---|
| **I-1** | 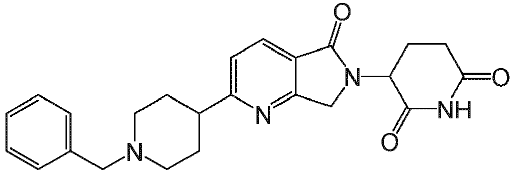 | >30 | 0.26 (33%) |
| **I-2** | 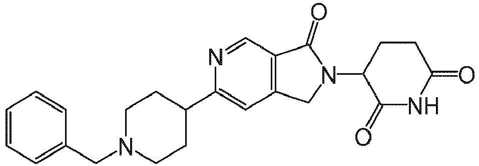 | >30 | >30 |
| **I-3** | 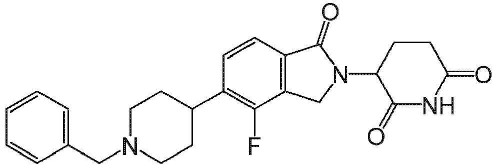 | >30 | 0.006 (72%) |
| **I-5** | 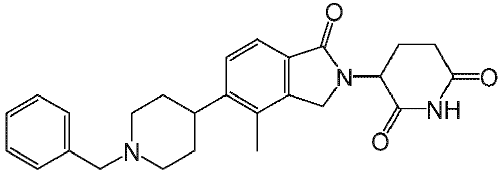 | >30 | 0.11 (59%) |
| **I-16** | 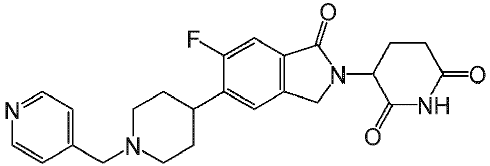 | 7.6 (22%) | 0.56 (42%) |
| **I-17** | 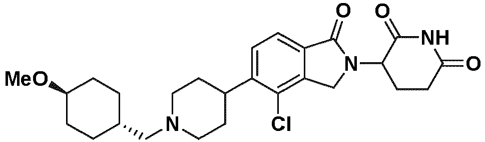 | 0.13 (40%) | 0.075 (30%) |
| **I-20** | 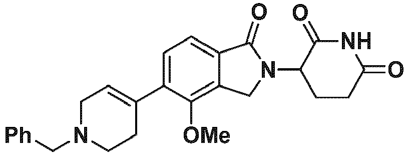 | 0.066 (40%) | 0.20 (42%) |
| **Control** | Pomalidomide | 0.05 (80% degradation at 10 µM) | >50 |

### Example 8: Quantification of in vitro Suppressive Potency of Primary Human Regulatory T cells Expanded in the Presence of Compounds

### Materials and methods

### Treg cell sorting:

Human buffy coats are obtained from BioreclamationIVT, in the USA. CD4+ T cells are isolated from said buffy coats using the RosetteSep Human CD4+ T cell enrichment Cocktail (Stemcell technologies, USA) and gradient centrifugation over Ficoll Paque Plus (GE HealthCare LifeSciences, USA) as per manufacturer's recommendations. Cells are resuspended in RPMI medium supplemented with 1% penicillin-Streptomycin solution, 10% Fetal Bovine Serum, HEPES (10 mM), MEM NEAA (100 nM), sodium pyruvate (1 mM) (all supplements from Thermo Fisher Scientific, USA), thereafter referred to as complete RPMI (cRPMI), and rested overnight at 37 °C, 5% CO₂ in the presence of 2U/mL rhIL-2 (Proleukin, Novartis). Cells are collected and resuspended in autoMACS Running Buffer supplemented with BSA (Miltenyi Biotec, USA) and labelled using CD4-FITC antibody (clone RPA-T4), CD25-APC antibody (clone M-A251) (Biolegend) and CD25 Microbeads (Miltenyi Biotec, USA). CD25-enriched cells are then isolated using the autoMACS Pro Separator. A highly purified population of Treg cells is then obtained by further sorting CD4+ CD25Hi cells using a Sony SH800 cell sorter. The resulting Treg cell population is routinely above 90% pure according to FOXP3 expression.

### Treg cell expansion:

Purified Treg cells are plated in cRPMI in 96-well, round-bottom plates at a density of 25000-50000 cells per well and activated in the presence of 500 U/mL rhIL2, and Treg expander Dynabeads (Thermo Fisher Scientific, USA) according to manufacturer's recommendations, in the presence or absence of 100 µM rapamycin (Thermo Fisher Scientific, USA). The compounds of the present invention are then added at a final concentration of 10 µM and DMSO was added as a vehicle control. Cells are incubated at 37 °C, 5% CO₂ for a total of 12-14 days. The compound and rhIL2 are replenished every 48h during the entirety of the culture.

### Phenotypic analysis of expanded Treg cells:

Cell are collected and counted and the fold expansion is calculated as (number of cells recovered)/(number of cells plated). A fraction of the cells is fixed and permeabilized using the eBioscience Foxp3 staining Buffer kit (eBioscience, Thermo Fisher Scientific, USA) and stained with Helios-PECyanine7 antibody (Clone 22F6). To determine IL2-expression, expanded Treg cells are further incubated in the presence of the eBioscience Cell Stimulation Cocktail with Protein inhibitors (Thermo Fisher Scientific) for 4 hours, followed by fixation and staining with IL2-BV711 antibody (clone MQ1-17H12) (Biolegend, USA). Cells are acquired on an LSRFortessa (Becton Dickinson, USA) and analysis was performed using the FlowJo software (TreeStar, USA).

### Functional analysis of expanded Treg cells:

Primary human PBMCs are obtained from freshly prepared buffy coats (BioReclamationIVT) using gradient centrifugation over Ficoll Paque Plus as per manufacturer's recommendations. Cells are then labelled with CFSE (5(6)-Carboxyfluorescein diacetate N-succinimidyl ester, Sigma-Aldrich, USA) and plated in triplicates cRPMI in round bottom 96-well plates, alone or with expanded Treg cells at a 1:2 PBMC:Treg ratio. The compounds of the present invention are then added at a final concentration of 10 µM and DMSO is added as a vehicle control. Cells are activated using soluble anti-CD3 antibody (clone OKT3) (eBioscience, ThermoFisher Scientific, USA) at a final concentration of 100 ng/ml. Cells are incubated at 37 °C, 5% CO₂ for a total of 4-5 days. At the end of the culture, cells are stained using the Live/dead Blue viability stain (Thermo Fisher Scientific, USA) as per manufacturer's instructions, followed by staining with CD4-BUV737 (Clone SK3) (BDBiosciences, USA) and CD8-BV711 (clone RPA-T8) (Biolegend, USA). Cells are acquired on an LSRFortessa (Becton Dickinson, USA) and analysis is performed using the FlowJo software (TreeStar, USA). Proliferation is assessed in each population as the proportion of cells having diluted CFSE. Suppression is assessed for each condition in comparison to the responders plated alone.

## Claims

1. A compound of Formula (I): 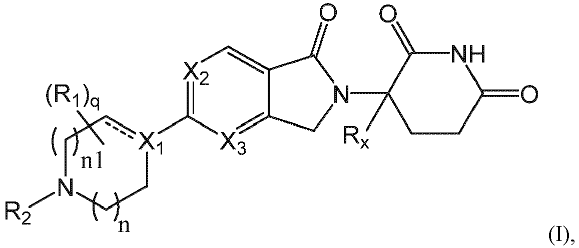 wherein:
X₁ is CR₃;
-̅ -̅ -̅ -̅ -̅ -̅ is optionally a double bond when X₁ is CR₃ and R₃ is absent;
X₂ is N and X₃ is CR₁₄; or X₂ is CR₁₃ and X₃ is N; or X₂ is CR₁₅ and X₃ is CR₁₄; or X₂ is CR₁₃ and X₃ is CR₁₆;
each R₁ is independently D, (C₁-C₆)alkyl, (C₁-C₆)haloalkyl, (C₁-C₆)hydroxyalkyl, CN, or halogen, or
two R₁ together with the carbon atoms to which they are attached form (C₃-C₇)cycloalkyl or a 4- to 6-membered heterocycloalkyl ring comprising 1 to 3 heteroatoms selected from O, N, and S, or
two R₁, when on adjacent atoms, together with the atoms to which they are attached form a (C₆-C₁₀)aryl ring or a 5- or 6-membered heteroaryl ring comprising 1 to 3 heteroatoms selected from O, N, and S;
R₂ is (C₁-C₆)alkyl, (C₆-C₁₀)aryl, 5- or 6-membered heteroaryl comprising 1 to 3 heteroatoms selected from O, N, and S, (C₃-C₈)cycloalkyl, or 5- to 7-membered heterocycloalkyl comprising 1 to 3 heteroatoms selected from O, N, and S, wherein the alkyl is optionally substituted with one or more R₄; and the aryl, heteroaryl, cycloalkyl, and heterocycloalkyl are optionally substituted with one or more R₅, or
R₁ and R₂, when on adjacent atoms, together with the atoms to which they are attached form a 5- or 6-membered heterocycloalkyl ring;
R₃ is H or D, or R₃ is absent when -̅-̅-̅-̅-̅-̅ is a double bond;
each R₄ is independently selected from -C(O)OR₆, -C(O)NR₆R_{6'}, -NR₆C(O)R_{6'}, halogen, -OH, -NH₂, CN, (C₆-C₁₀)aryl, 5- or 6-membered heteroaryl comprising 1 to 4 heteroatoms selected from O, N, and S, (C₃-C₈)cycloalkyl, and 4- to 7-membered heterocycloalkyl ring comprising 1 to 3 heteroatoms selected from O, N, and S, wherein the aryl, heteroaryl, cycloalkyl, and heterocycloalkyl groups are optionally substituted with one or more R₇;
each R₅ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, (C1-C₆)hydroxyalkyl, halogen, -OH, -NH₂, CN, (C₃-C₇)cycloalkyl, 5- to 7-membered heterocycloalkyl comprising 1 to 3 heteroatoms selected from O, N, and S, (C₆-C₁₀)aryl, and 5- or 6-membered heteroaryl comprising 1 to 3 heteroatoms selected from O, N, and S, or
two R₅, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more Rio, or
two R₅, when on adjacent atoms, together with the atoms to which they are attached form a (C₅-C₇)cycloalkyl ring or a 5- to 7-membered heterocycloalkyl ring comprising 1 to 3 heteroatoms selected from O, N, and S optionally substituted with one or more R₁₀;
R₆ and R_{6'} are each independently H, (C₁-C₆)alkyl, or (C₆-C₁₀)aryl;
each R₇ is independently selected from (C₁-C₆)alkyl, (C₂-C₆)alkenyl, (C₂-C₆)alkynyl, (C₁-C₆)alkoxy, (C₁-C₆)haloalkyl, (C₁-C₆)haloalkoxy, -C(O)R₈, -(CH₂)₀₋₃C(O)OR₈, -C(O)NR₈R₉, -NR₈C(O)R₉, - NR₈C(O)OR₉, -S(O)ₚNR₈R₉, -S(O)ₚR₁₂, (C₁-C₆)hydroxyalkyl, halogen, -OH, -O(CH₂)₁₋₃CN, -NH₂, CN, -O(CH₂)₀₋₃(C₆-C₁₀)aryl, adamantyl, -O(CH₂)₀₋₃-5- or 6-membered heteroaryl comprising 1 to 3 heteroatoms selected from O, N, and S, (C₆-C₁₀)aryl, monocyclic or bicyclic 5- to 10-membered heteroaryl comprising 1 to 3 heteroatoms selected from O, N, and S, (C₃-C₇)cycloalkyl, and 5- to 7-membered heterocycloalkyl comprising 1 to 3 heteroatoms selected from O, N, and S, wherein the alkyl is optionally substituted with one or more R₁₁, and the aryl, heteroaryl, and heterocycloalkyl are optionally substituted with one or more substituents each independently selected from halogen, (C₁-C₆)alkyl, (C₁-C₆)haloalkyl, and (C₁-C₆)alkoxy, or
two R₇ together with the carbon atom to which they are attached form a =(O), or
two R₇, when on adjacent atoms, together with the atoms to which they are attached form a (C₆-C₁₀)aryl ring or a 5- or 6-membered heteroaryl ring comprising 1 to 3 heteroatoms selected from O, N, and S, optionally substituted with one or more Rio, or
two R₇ together with the atoms to which they are attached form a (C₅-C₇) cycloalkyl ring or a 5- to 7-membered heterocycloalkyl ring comprising 1 to 3 heteroatoms selected from O, N, and S, optionally substituted with one or more R₁₀;
R₈ and R₉ are each independently H or (C₁-C₆)alkyl;
each R₁₀ is independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN, or
two R₁₀ together with the carbon atom to which they are attached form a =(O);
each R₁₁ is independently selected from CN, (C₁-C₆)alkoxy, (C₆-C₁₀)aryl, and 5- to 7-membered heterocycloalkyl comprising 1 to 3 heteroatoms selected from O, N, and S, wherein the aryl and heterocycloalkyl are optionally substituted with one or more substituents each independently selected from (C₁-C₆)alkyl, (C₁-C₆)alkoxy, (C₁-C₆)haloalkyl, (C₁-C₆)haloalkoxy, (C₁-C₆)hydroxyalkyl, halogen, -OH, -NH₂, and CN;
R₁₂ is (C₁-C₆)alkyl, (C₁-C₆)haloalkyl, (C₆-C₁₀)aryl, or 5- to 7-membered heterocycloalkyl comprising 1 to 3 heteroatoms selected from O, N, and S;
R₁₃ is H, halogen, -OH, or -NH₂;
R₁₄ is H, (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN;
R₁₅ is halogen, -OH, or -NH₂;
R₁₆ is (C₁-C₃)alkyl, (C₁-C₃)alkoxy, (C₁-C₃)haloalkyl, (C₁-C₃)haloalkoxy, (C₁-C₃)hydroxyalkyl, halogen, -OH, -NH₂, -NO₂, or CN;
Rₓ is H or D;
p is 0, 1, or 2;
n is 0, 1, or 2;
n1 is 1 or 2, wherein n + n1 ≤ 3; and
q is 0, 1, 2, 3, or 4;
or a pharmaceutically acceptable salt, hydrate, solvate, stereoisomer, or tautomer thereof.

2. The compound according to claim 1, wherein Rₓ is H.

3. The compound according to claim 1 or 2, wherein X₂ is N and X₃ is CR₁₄ or wherein X₂ is CR₁₃ and X₃ is N or wherein X₂ is CR₁₅, and X₃ is CR₁₄ or wherein X₂ is CR₁₃, and X₃ is CR₁₆.

4. The compound according to claim 1, having a Formula (Ia), Formula (Ib), Formula (Ic), or Formula (Id): 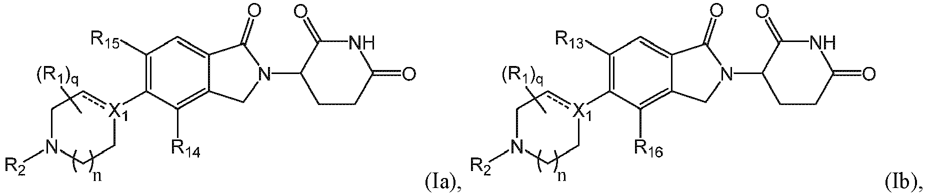 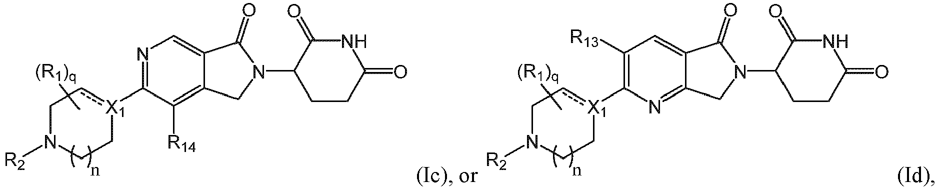 or a pharmaceutically acceptable salt, hydrate, solvate, stereoisomer, or tautomer thereof.

5. The compound according to any one of claims 1-4, wherein -̅-̅-̅-̅-̅-̅ is a double bond, X₁ is CR₃, and R₃ is absent or wherein -̅ -̅ -̅ -̅ -̅ -̅ is a single bond, X₁ is C R₃, and R₃ is H.

6. The compound of claim 1, having a Formula (Ie), Formula (If), Formula (Ig), or Formula (Ih): 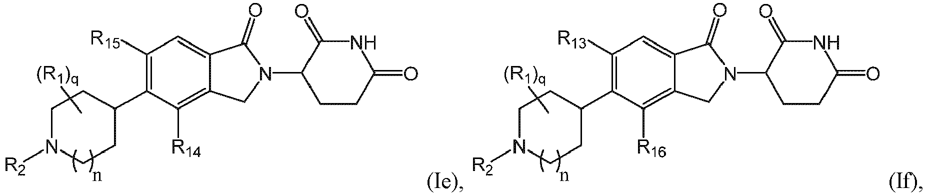 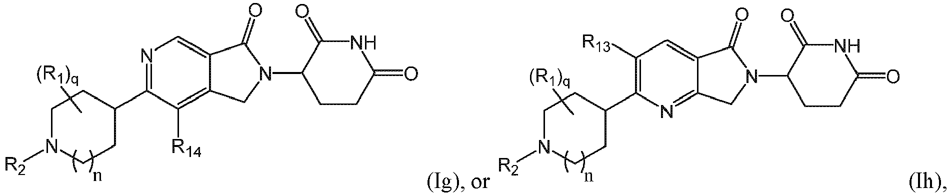 or a pharmaceutically acceptable salt, hydrate, solvate, stereoisomer, or tautomer thereof.

7. The compound according to any one of claims 1-6, wherein n is 0, 1, or 2.

8. The compound of claim 1, having a Formula (Ij), Formula (Ik), or Formula (II): 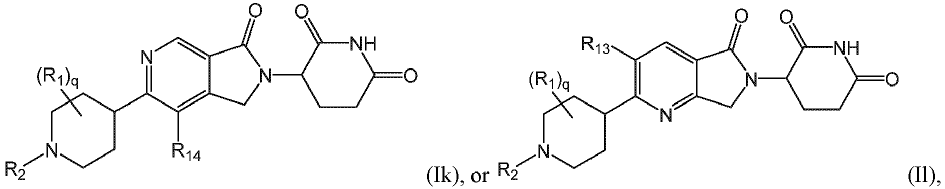 or a pharmaceutically acceptable salt, hydrate, solvate, stereoisomer, or tautomer thereof.

9. The compound according to any one of claims 1-8, wherein R₂ is (C₆-C₁₀)aryl, (C₃-C₈)cycloalkyl, or 5- to 7-membered heterocycloalkyl comprising 1 to 3 heteroatoms selected from O, N, and S, wherein the aryl, heteroaryl, cycloalkyl, and heterocycloalkyl are optionally substituted with one to three R₅.

10. The compound according to any one of claims 1-8, wherein R₂ is (C₁-C₆)alkyl optionally substituted with one to three R₄ or wherein R₂ is (C₁-C₆)alkyl substituted with one to three R₄.

11. The compound according to claim 1 selected from:
3-(2-(1-benzylpiperidin-4-yl)-5-oxo-5,7-dihydro-6H-pyrrolo [3,4-b]pyridin-6-yl)piperidine-2,6-dione 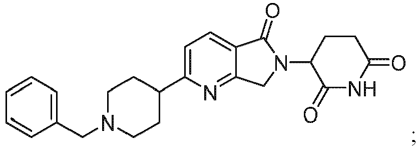
3-(6-(1-benzylpiperidin-4-yl)-3-oxo-1,3-dihydro-2H-pyrrolo[3,4-c]pyridin-2-yl)piperidine-2,6-dione 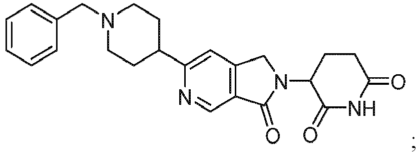
3-(5-(1-benzylpiperidin-4-yl)-4-fluoro-1-oxoisoindofin-2-yl)piperidine-2,6-dione 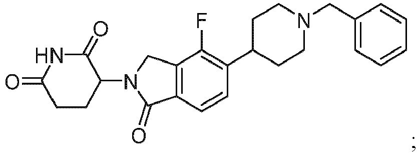
3-(5-(1-benzylpiperidin-4-yl)-6-fluoro-1-oxoisoindofin-2-yl)piperidine-2,6-dione 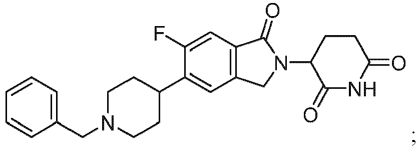
3-(5-(1-benzylpiperidin-4-yl)-4-methyl-1-oxoisoindolin-2-yl)piperidine-2,6-dione 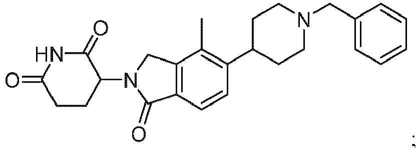
3-(4-amino-5-(1-benzylpiperidin-4-yl)-1-oxoisoindolin-2-yl)piperidine-2,6-dione 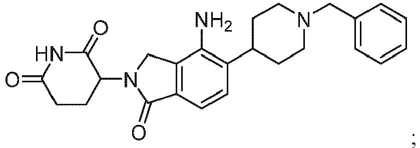
3-(6-amino-5-(1-benzylpiperidin-4-yl)-1-oxoisoindolin-2-yl)piperidine-2,6-dione 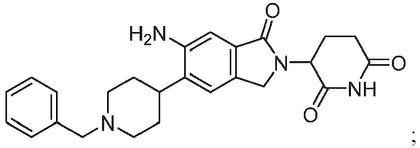
3-(5-(1-benzylpiperidin-4-yl)-4-chloro-1-oxoisoindolin-2-yl)piperidine-2,6-dione 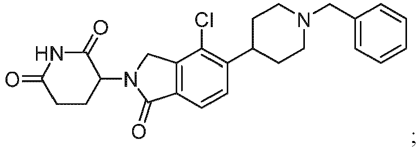
3-(5-(1-benzylpiperidin-4-yl)-6-chloro-1-oxoisoindolin-2-yl)piperidine-2,6-dione 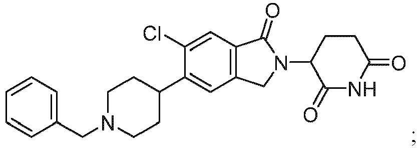
3-(5-(1-benzylpiperidin-4-yl)-4-hydroxy-1-oxoisoindolin-2-yl)piperidine-2,6-dione 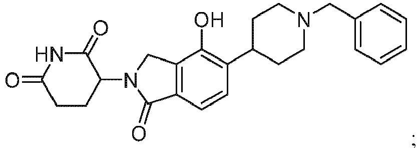
3-(5-(1-benzylpiperidin-4-yl)-6-hydroxy-1-oxoisoindolin-2-yl)piperidine-2,6-dione 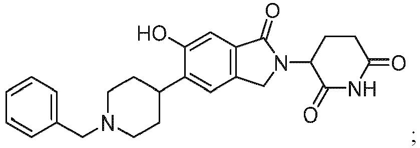
3-(5-(1-benzylpiperidin-4-yl)-4-methoxy-1-oxoisoindofin-2-yl)piperidine-2,6-dione 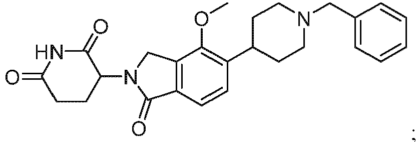
5-(1-benzylpiperidin-4-yl)-2-(2,6-dioxopiperidin-3-yl)-1-oxoisoindoline-4-carbonitrile 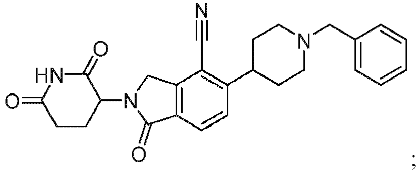
3-(5-(1-benzylpiperidin-4-yl)-1-oxo-4-(trifluoromethyl)isoindolin-2-yl)piperidine-2,6-dione 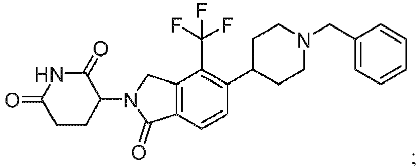
3-(5-(1-benzylpiperidin-4-yl)-4-nitro-1-oxoisoindolin-2-yl)piperidine-2,6-dione 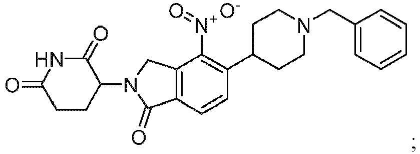
3-(6-fluoro-1-oxo-5-(1-(pyridin-4-ylmethyl)piperidin-4-yl)isoindolin-2-yl)piperidine-2,6-dione 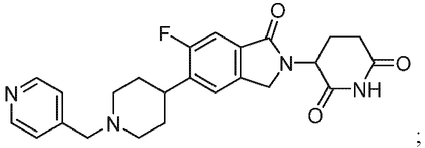
3-(4-chloro-5-(1-(((1r,4r)-4-methoxycyclohexyl)methyl)piperidin-4-yl)-1-oxoisoindolin-2-yl)piperidine-2,6-dione 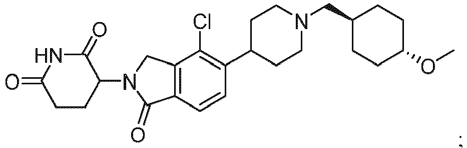
3-(4-fluoro-5-(1-(((1r,4r)-4-methoxycyclohexyl)methyl)piperidin-4-yl)-1-oxoisoindolin-2-yl)piperidine-2,6-dione 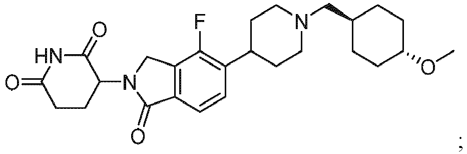
3-(4-hydroxy-5-(1-(((1r,4r)-4-methoxycyclohexyl)methyl)piperidin-4-yl)-1-oxoisoindolin-2-yl)piperidine-2,6-dione 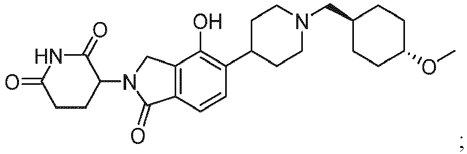 and
3-(5-(1-benzyl-1,2,3,6-tetrahydropyridin-4-yl)-4-methoxy-1-oxoisoindolin-2-yl)piperidine-2,6-dione 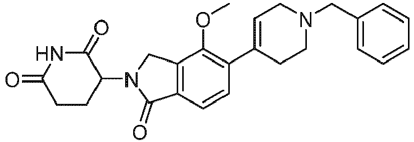 or a pharmaceutically acceptable salt, hydrate, solvate, stereoisomer, or tautomer thereof.

12. A pharmaceutical composition comprising a therapeutically effective amount of a compound according to any one of the claims 1-11, or a pharmaceutically acceptable salt, hydrate, solvate, stereoisomer, or tautomer thereof, and a pharmaceutically acceptable carrier or excipient, optionally further comprising at least one additional pharmaceutical agent.

13. A compound according to any one of claims 1-11, or a pharmaceutically acceptable salt, hydrate, solvate, stereoisomer, or tautomer thereof, for use in the treatment of a disease or disorder that is affected by the reduction of IKZF2 protein levels.

14. The compound, or a pharmaceutically acceptable salt, hydrate, solvate, stereoisomer, or tautomer thereof, for use according to claim 13, wherein the disease or disorder is selected from non-small cell lung cancer (NSCLC), melanoma, triple-negative breast cancer (TNBC), nasopharyngeal cancer (NPC), microsatellite stable colorectal cancer (mssCRC), thymoma, carcinoid, acute myelogenous leukemia, and gastrointestinal stromal tumor (GIST).

15. A pharmaceutical combination comprising a compound according to any one of claims 1-11, or a pharmaceutically acceptable salt, hydrate, solvate, stereoisomer, or tautomer thereof, and one or more additional therapeutic agent(s).

## Patentansprüche

1. Verbindung der Formel (I): 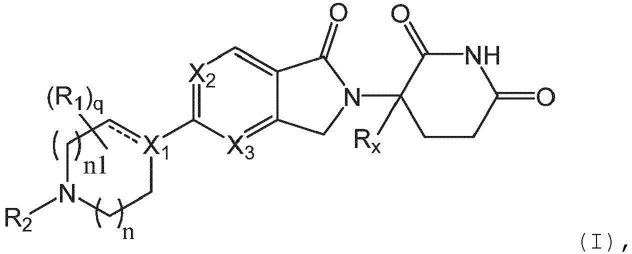 wobei:
X₁ für CR₃ steht;
-̅-̅-̅-̅-̅-̅ gegebenenfalls eine Doppelbindung ist, wenn X₁ für CR₃ steht und R₃ fehlt;
X₂ für N steht und X₃ für CR₁₄ steht oder X₂ für CR₁₃ steht und X₃ für N steht oder X₂ für CR₁₅ steht und X₃ für CR₁₄ steht oder X₂ für CR₁₃ steht und X₃ für CR₁₆ steht;
R₁ jeweils unabhängig für D, (C₁-C₆)Alkyl, (C₁-C₆)-Halogenalkyl, (C₁-C₆)Hydroxyalkyl, CN oder Halogen steht oder
zwei R₁ zusammen mit den Kohlenstoffatomen, an die sie gebunden sind, (C₃-C₇)Cycloalkyl oder einen 4- bis 6-gliedrigen Heterocycloalkylring mit 1 bis 3 Heteroatomen, die aus O, N und S ausgewählt sind, bilden oder
zwei R₁, wenn sie sich an benachbarten Atomen befinden, zusammen mit den Atomen, an die sie gebunden sind, einen (C₆-C₁₀)Arylring oder einen 5- oder 6-gliedrigen Heteroarylring mit 1 bis 3 Heteroatomen, die aus O, N und S ausgewählt sind, bilden;
R₂ für (C₁-C₆)Alkyl, (C₆-C₁₀)Aryl, 5- oder 6-gliedriges Heteroaryl mit 1 bis 3 Heteroatomen, die aus O, N und S ausgewählt sind, (C₃-C₈)Cycloalkyl oder 5- bis 7-gliedriges Heterocycloalkyl mit 1 bis 3 Heteroatomen, die aus O, N und S ausgewählt sind, steht, wobei das Alkyl gegebenenfalls durch ein oder mehrere R₄ substituiert ist und das Aryl, Heteroaryl, Cycloalkyl und Heterocycloalkyl gegebenenfalls durch ein oder mehrere R₅ substituiert sind, oder
R₁ und R₂, wenn sie sich an benachbarten Atomen befinden, zusammen mit den Atomen, an die sie gebunden sind, einen 5- oder 6-gliedrigen Heterocycloalkylring bilden;
R₃ für H oder D steht oder R₃ fehlt, wenn -̅-̅-̅-̅-̅-̅ eine Doppelbindung ist;
R₄ jeweils unabhängig aus -C(O)OR₆, -C(O)NR₆R_{6'}, - NR₆C(O)R_{6'}, Halogen, -OH, -NH₂, CN, (C₆-C₁₀)Aryl, 5- oder 6-gliedrigem Heteroaryl mit 1 bis 4 Heteroatomen, die aus O, N und S ausgewählt sind, (C₃-C₈)Cycloalkyl und einem 4- bis 7-gliedrigen Heterocycloalkylring mit 1 bis 3 Heteroatomen, die aus O, N und S ausgewählt sind, ausgewählt ist, wobei die Aryl-, Heteroaryl-, Cycloalkyl- und Heterocycloalkylgruppen gegebenenfalls durch ein oder mehrere R₇ substituiert sind;
R₅ jeweils unabhängig aus (C₁-C₆)Alkyl, (C₂-C₆)Alkenyl, (C₂-C₆)Alkinyl, (C₁-C₆)Alkoxy, (C₁-C₆)Halogenalkyl, (C₁-C₆)Halogenalkoxy, (C₁-C₆)Hydroxyalkyl, Halogen, -OH, -NH₂, CN, (C₃-C₇)Cycloalkyl, 5- bis 7-gliedrigem Heterocycloalkyl mit 1 bis 3 Heteroatomen, die aus O, N und S ausgewählt sind, (C₆-C₁₀)Aryl und 5- oder 6-gliedrigem Heteroaryl mit 1 bis 3 Heteroatomen, die aus O, N und S ausgewählt sind, ausgewählt ist oder
zwei R₅, wenn sie sich an benachbarten Atomen befinden, zusammen mit den Atomen, an die sie gebunden sind, einen (C₆-C₁₀)Arylring oder einen 5- oder 6-gliedrigen Heteroarylring mit 1 bis 3 Heteroatomen, die aus O, N und S ausgewählt sind, der gegebenenfalls durch ein oder mehrere R₁₀ substituiert ist, bilden oder
zwei R₅, wenn sie sich an benachbarten Atomen befinden, zusammen mit den Atomen, an die sie gebunden sind, einen (C₅-C₇)Cycloalkylring oder einen 5- bis 7-gliedrigen Heterocycloalkylring mit 1 bis 3 Heteroatomen, die aus O, N und S ausgewählt sind, der gegebenenfalls durch ein oder mehrere R₁₀ substituiert ist, bilden;
R₆ und R_{6'} jeweils unabhängig für H, (C₁-C₆)Alkyl oder (C₆-C₁₀)Aryl stehen;
R₇ jeweils unabhängig aus (C₁-C₆)Alkyl, (C₂-C₆)Alkenyl, (C₂-C₆)Alkinyl, (C₂-C₆)Alkoxy, (C₁-C₆)Halogenalkyl, (C₁-C₆)Halogenalkoxy, -C(O)R₈, -(CH₂)₀₋₃C(O)OR₈, -C(O)NR₈R₉, - NR₈C(O)R₉, -NR₈C(O)OR₉, -S(O)ₚNR₈R₉, -S(O)ₚR₁₂, (C₁-C₆)Hydroxyalkyl, Halogen, -OH, -O(CH₂)₁₋₃CN, -NH₂, CN, - O(CH₂)₀₋₃(C₆-C₁₀)Aryl, Adamantyl, -O(CH₂)₀₋₃-5- oder 6-gliedrigem Heteroaryl mit 1 bis 3 Heteroatomen, die aus O, N und S ausgewählt sind, (C₆-C₁₀)Aryl, monocyclischem oder bicyclischem 5- bis 10-gliedrigem Heteroaryl mit 1 bis 3 Heteroatomen, die aus O, N und S ausgewählt sind, (C₃-C₇)Cycloalkyl und 5- bis 7-gliedrigem Heterocycloalkyl mit 1 bis 3 Heteroatomen, die aus O, N und S ausgewählt sind, ausgewählt ist, wobei das Alkyl gegebenenfalls durch ein oder mehrere R₁₁ substituiert ist und das Aryl, Heteroaryl und Heterocycloalkyl gegebenenfalls durch einen oder mehrere Substituenten, die jeweils unabhängig aus Halogen (C₁-C₆)Alkyl, (C₁-C₆)Halogenalkyl und (C₁-C₆)Alkoxy ausgewählt sind, substituiert sind, oder
zwei R₇ zusammen mit dem Kohlenstoffatom, an das sie gebunden sind, ein =(O) bilden oder
zwei R₇, wenn sie sich an benachbarten Atomen befinden, zusammen mit den Atomen, an die sie gebunden sind, einen (C₆-C₁₀)Arylring oder einen 5- oder 6-gliedrigen Heteroarylring mit 1 bis 3 Heteroatomen, die aus O, N und S ausgewählt sind, der gegebenenfalls durch ein oder mehrere R₁₀ substituiert ist, bilden oder
zwei R₇ zusammen mit den Atomen, an die sie gebunden sind, einen (C₅-C₇)Cycloalkylring oder einen 5- bis 7-gliedrigen Heterocycloalkylring mit 1 bis 3 Heteroatomen, die aus O, N und S ausgewählt sind, der gegebenenfalls durch ein oder mehrere R₁₀ substituiert ist, bilden;
R₈ und R₉ jeweils unabhängig für H oder (C₁-C₆)Alkyl stehen;
R₁₀ jeweils unabhängig aus (C₁-C₆)Alkyl, (C₁-C₆)Alkoxy, (C₁-C₆)Halogenalkyl, (C₁-C₆)Halogenalkoxy, (C₁-C₆)Hydroxyalkyl, Halogen, -OH, -NH₂ und CN ausgewählt ist oder
zwei R₁₀ zusammen mit dem Kohlenstoffatom, an das sie gebunden sind, ein =(O) bilden;
R₁₁ jeweils unabhängig aus CN, (C₁-C₆)Alkoxy, (C₆-C₁₀)Aryl und 5- bis 7-gliedrigem Heterocycloalkyl mit 1 bis 3 Heteroatomen, die aus O, N und S ausgewählt sind, ausgewählt ist, wobei das Aryl und Heterocycloalkyl gegebenenfalls durch einen oder mehrere Substituenten, die jeweils unabhängig aus (C₁-C₆)Alkyl, (C₁-C₆)Alkoxy, (C₁-C₆)Halogenalkyl, (C₁-C₆)Halogenalkoxy, (C₁-C₆)Hydroxyalkyl, Halogen, -OH, -NH₂ und CN ausgewählt sind, substituiert sind;
R₁₂ für (C₁-C₆)Alkyl, (C₁-C₆)Halogenalkyl, (C₆-C₁₀)Aryl oder 5- bis 7-gliedriges Heterocycloalkyl mit 1 bis 3 Heteroatomen, die aus O, N und S ausgewählt sind, steht;
R₁₃ für H, Halogen, -OH oder -NH₂ steht;
R₁₄ für H, (C₁-C₃)Alkyl, (C₁-C₃)Alkoxy, (C₁-C₃)Halogenalkyl, (C₁-C₃)Halogenalkoxy, (C₁-C₃)Hydroxyalkyl, Halogen, -OH, -NH₂, -NO₂ oder CN steht;
R₁₅ für Halogen, -OH oder -NH₂ steht;
R₁₆ für (C₁-C₃)Alkyl, (C₁-C₃)Alkoxy, (C₁-C₃)Halogenalkyl, (C₁-C₃)Halogenalkoxy, (C₁-C₃)Hydroxyalkyl, Halogen, -OH, -NH₂, -NO₂ oder CN steht;
Rₓ für H oder D steht;
p für 0, 1 oder 2 steht;
n für 0, 1 oder 2 steht;
n1 für 1 oder 2 steht, wobei n + n1 ≤ 3; und
q für 0, 1, 2, 3 oder 4 steht;
oder ein pharmazeutisch unbedenkliches Salz, Hydrat, Solvat, Stereoisomer oder Tautomer davon.

2. Verbindung nach Anspruch 1, wobei Rₓ für H steht.

3. Verbindung nach Anspruch 1 oder 2, wobei X₂ für N steht und X₃ für CR₁₄ steht oder wobei X₂ für CR₁₃ steht und X₃ für N steht oder wobei X₂ für CR₁₅ steht und X₃ für CR₁₄ steht oder wobei X₂ für CR₁₃ steht und X₃ für CR₁₆ steht.

4. Verbindung nach Anspruch 1 mit einer Formel (Ia), Formel (Ib), Formel (Ic) oder Formel (Id): 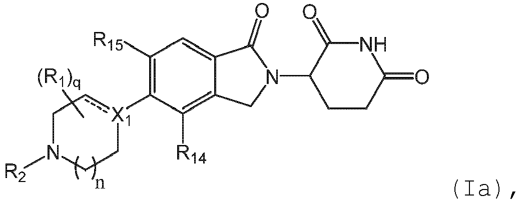 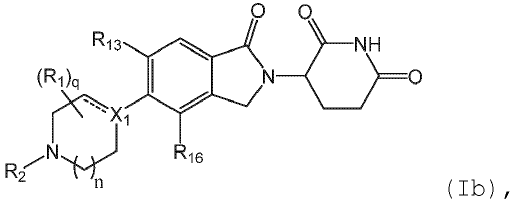 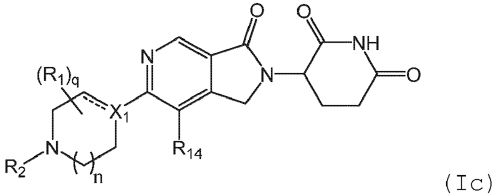 oder 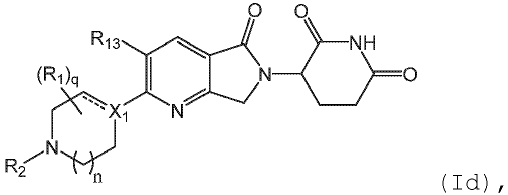 oder ein pharmazeutisch unbedenkliches Salz, Hydrat, Solvat, Stereoisomer oder Tautomer davon.

5. Verbindung nach einem der Ansprüche 1-4, wobeieine Doppelbindung ist, X₁ für CR₃ steht und R₃ fehlt oder wobei -̅ -̅ -̅ -̅ -̅ -̅ eine Einfachbindung ist, X₁ für CR₃ steht und R₃ für H steht.

6. Verbindung nach Anspruch 1 mit einer Formel (Ie), Formel (If), Formel (Ig) oder Formel (Ih): 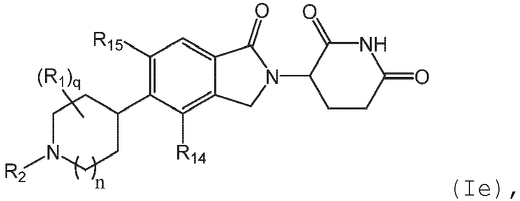 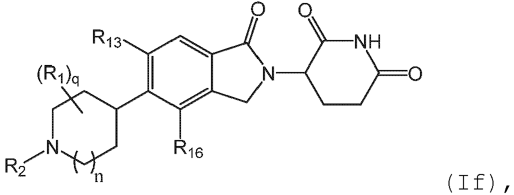 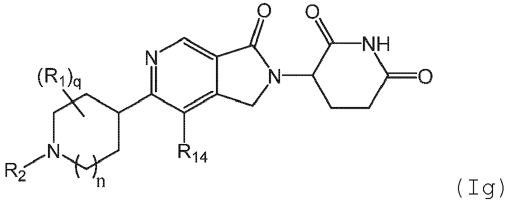 oder 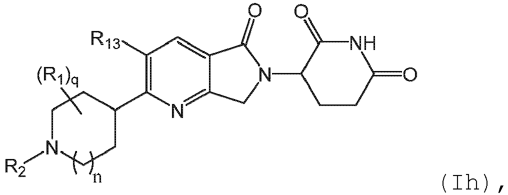 oder ein pharmazeutisch unbedenkliches Salz, Hydrat, Solvat, Stereoisomer oder Tautomer davon.

7. Verbindung nach einem der Ansprüche 1-6, wobei n für 0, 1 oder 2 steht.

8. Verbindung nach Anspruch 1 mit einer Formel (Ii), Formel (Ij), Formel (Ik) oder Formel (II): 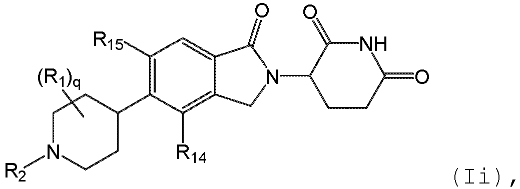 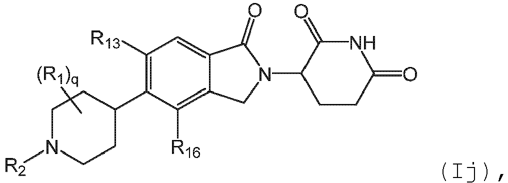 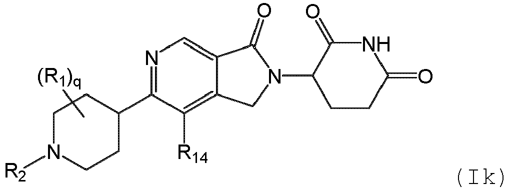 oder 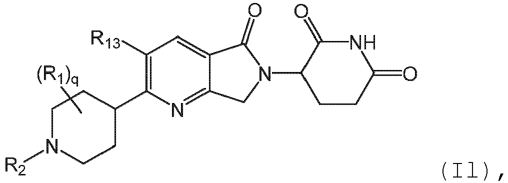 oder ein pharmazeutisch unbedenkliches Salz, Hydrat, Solvat, Stereoisomer oder Tautomer davon.

9. Verbindung nach einem der Ansprüche 1-8, wobei R₂ für (C₆-C₁₀)Aryl, (C₃-C₈)Cycloalkyl oder 5- bis 7-gliedriges Heterocycloalkyl mit 1 bis 3 Heteroatomen, die aus O, N und S ausgewählt sind, steht, wobei das Aryl, Heteroaryl, Cycloalkyl und Heterocycloalkyl gegebenenfalls durch ein bis drei R₅ substituiert sind.

10. Verbindung nach einem der Ansprüche 1-8, wobei R₂ für (C₁-C₆)Alkyl, das gegebenenfalls durch ein bis drei R₄ substituiert ist, steht oder wobei R₂ für (C₁-C₆)Alkyl, das durch ein bis drei R₄ substituiert ist, steht.

11. Verbindung nach Anspruch 1, ausgewählt aus:
3-(2-(1-Benzylpiperidin-4-yl)-5-oxo-5,7-dihydro-6H-pyrrolo[3,4-b]pyridin-6-yl)piperidin-2,6-dion 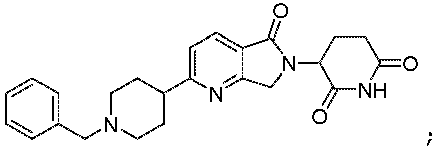
3-(6-(1-Benzylpiperidin-4-yl)-3-oxo-1,3-dihydro-2H-pyrrolo[3,4-c]pyridin-2-yl)piperidin-2,6-dion 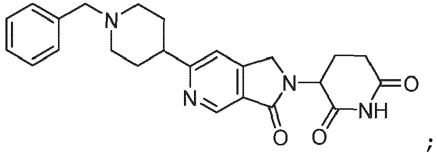
3-(5-(1-Benzylpiperidin-4-yl)-4-fluor-1-oxoisoindolin-2-yl)piperidin-2,6-dion 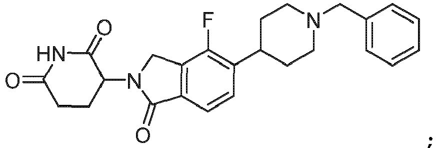
3-(5-(1-Benzylpiperidin-4-yl)-6-fluor-1-oxoisoindolin-2-yl)piperidin-2,6-dion 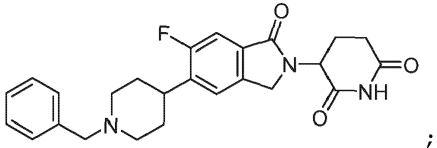
3-(5-(1-Benzylpiperidin-4-yl)-4-methyl-1-oxoisoindolin-2-yl)piperidin-2,6-dion 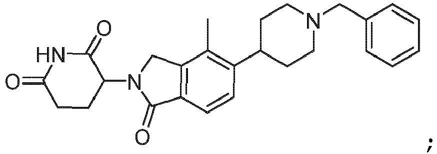
3-(4-Amino-5-(1-benzylpiperidin-4-yl)-1-oxoisoindolin-2-yl)piperidin-2,6-dion 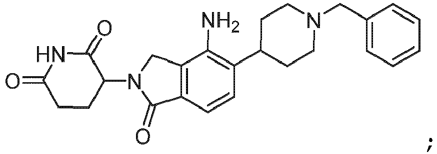
3-(6-Amino-5-(1-benzylpiperidin-4-yl)-1-oxoisoindolin-2-yl)piperidin-2,6-dion 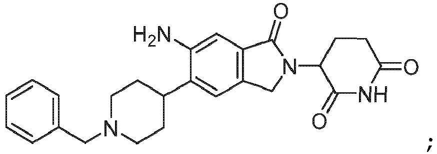
3-(5-(1-Benzylpiperidin-4-yl)-4-chlor-1-oxoisoindolin-2-yl)piperidin-2,6-dion 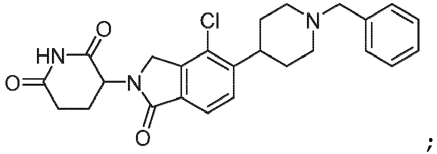
3-(5-(1-Benzylpiperidin-4-yl)-6-chlor-1-oxoisoindolin-2-yl)piperidin-2,6-dion 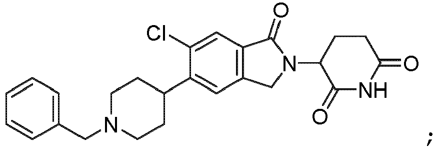
3-(5-(1-Benzylpiperidin-4-yl)-4-hydroxy-1-oxoisoindolin-2-yl)piperidin-2,6-dion 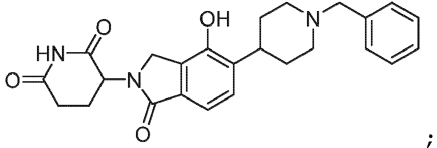
3-(5-(1-Benzylpiperidin-4-yl)-6-hydroxy-1-oxoisoindolin-2-yl)piperidin-2,6-dion 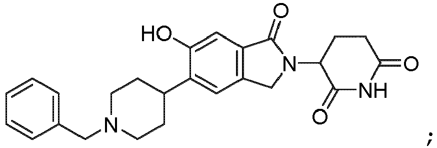
3-(5-(1-Benzylpiperidin-4-yl)-4-methoxy-1-oxoisoindolin-2-yl)piperidin-2,6-dion 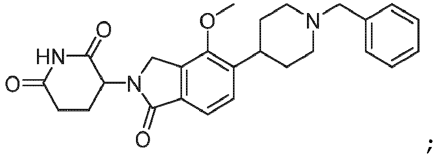
5-(1-Benzylpiperidin-4-yl)-2-(2,6-dioxopiperidin-3-yl)-1-oxoisoindolin-4-carbonitril 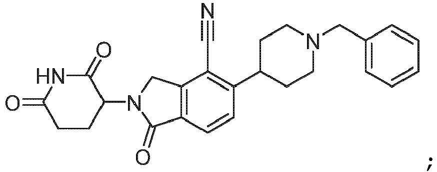
3-(5-(1-Benzylpiperidin-4-yl)-1-oxo-4-(trifluormethyl)isoindolin-2-yl)piperidin-2,6-dion 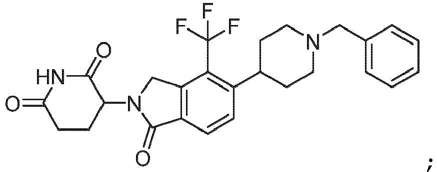
3-(5-(1-Benzylpiperidin-4-yl)-4-nitro-1-oxoisoindolin-2-yl)piperidin-2,6-dion 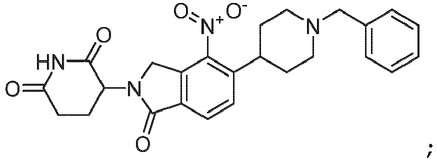
3-(6-Fluor-1-oxo-5-(1-(pyridin-4-ylmethyl)piperidin-4-yl)isoindolin-2-yl)piperidin-2,6-dion 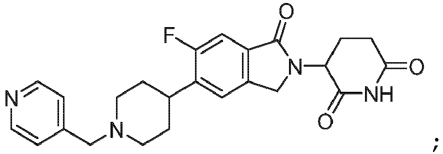
3-(4-Chlor-5-(1-(((1r,4r)-4-methoxycyclohexyl)methyl)piperidin-4-yl)-1-oxoisoindolin-2-yl)piperidin-2,6-dion 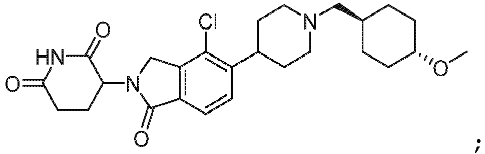
3-(4-Fluor-5-(1-(((1r,4r)-4-methoxycyclohexyl)methyl)piperidin-4-yl)-1-oxoisoindolin-2-yl)piperidin-2,6-dion 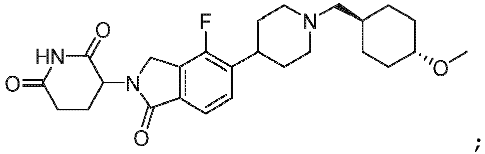
3-(4-Hydroxy-5-(1-(((1r,4r)-4-methoxycyclohexyl)methyl)piperidin-4-yl)-1-oxoisoindolin-2-yl)piperidin-2,6-dion 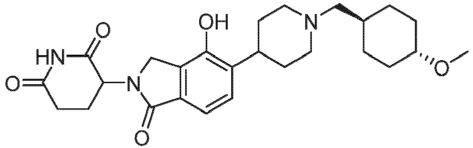 und
3-(5-(1-Benzyl-1,2,3,6-tetrahydropyridin-4-yl)-4-methoxy-1-oxoisoindolin-2-yl)piperidin-2,6-dion 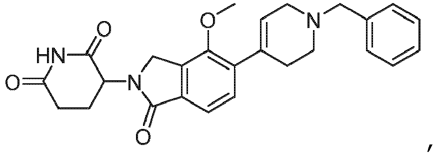 oder ein pharmazeutisch unbedenkliches Salz, Hydrat, Solvat, Stereoisomer oder Tautomer davon.

12. Pharmazeutische Zusammensetzung, umfassend eine therapeutisch wirksame Menge einer Verbindung nach einem der Ansprüche 1-11 oder eines pharmazeutisch unbedenklichen Salzes, Hydrats, Solvats, Stereoisomers oder Tautomers davon und einen pharmazeutisch unbedenklichen Träger oder Hilfsstoff und gegebenenfalls ferner umfassend mindestens ein zusätzliches pharmazeutisches Mittel.

13. Verbindung nach einem der Ansprüche 1-11 oder ein pharmazeutisch unbedenkliches Salz, Hydrat, Solvat, Stereoisomer oder Tautomer davon zur Verwendung bei der Behandlung einer Erkrankung oder Störung, die durch die Verringerung von IKZF2-Proteinniveaus beeinflusst wird.

14. Verbindung oder ein pharmazeutisch unbedenkliches Salz, Hydrat, Solvat, Stereoisomer oder Tautomer davon, zur Verwendung nach Anspruch 13, wobei die Erkrankung oder Störung aus nichtkleinzelligem Lungenkrebs (NSCLC), Melanom, dreifach-negativem Brustkrebs (TNBC), Nasopharyngealkrebs (NPC), mikrosatellitenstabilem Kolorektalkrebs (mssCRC), Thymom, Karzinoid, akuter myeloischer Leukämie und gastrointestinalem Stromatumor (GIST) ausgewählt ist.

15. Pharmazeutische Kombination, umfassend eine Verbindung nach einem der Ansprüche 1-11 oder ein pharmazeutisch unbedenkliches Salz, Hydrat, Solvat, Stereoisomer oder Tautomer davon und ein oder mehrere zusätzliche therapeutische Mittel.

## Revendications

1. Composé de formule (I) : 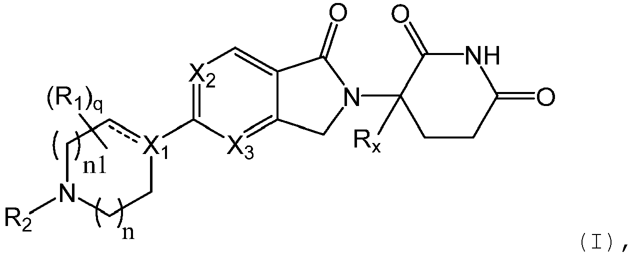
X₁ étant CR₃ ;
-̅-̅-̅-̅-̅-̅ étant éventuellement une double liaison lorsque X₁ est CR₃ et R₃ est absent ;
X₂ étant N et X₃ étant CR₁₄ ; ou X₂ étant CR₁₃ et X₃ étant N ; ou X₂ étant CR₁₅ et X₃ étant CR₁₄ ; ou X₂ étant CR₁₃ et X₃ étant CR₁₆ ;
chaque R₁ étant indépendamment D, (C₁-C₆) alkyle, (C₁-C₆)halogénoalkyle, (C₁-C₆)hydroxyalkyle, CN, ou halogène, ou
deux R₁ conjointement avec les atomes de carbone auxquels ils sont fixés formant (C₃-C₇)cycloalkyle ou un cycle hétérocycloalkyle à 4 à 6 chaînons comprenant 1 à 3 hétéroatomes choisis parmi O, N, et S, ou
deux R₁, lorsqu'ils sont sur des atomes adjacents, conjointement avec les atomes auxquels ils sont fixés formant un cycle (C₆-C₁₀)aryle ou un cycle hétéroaryle à 5 ou 6 chaînons comprenant 1 à 3 hétéroatomes choisis parmi O, N, et S ;
R₂ étant (C₁-C₆)alkyle, (C₆-C₁₀)aryle, hétéroaryle à 5 ou 6 chaînons comprenant 1 à 3 hétéroatomes choisis parmi O, N, et S, (C₃-C₈)cycloalkyle, ou hétérocycloalkyle à 5 à 7 chaînons comprenant 1 à 3 hétéroatomes choisis parmi O, N, et S, l'alkyle étant éventuellement substitué par un ou plusieurs R₄ ; et l'aryle, l'hétéroaryle, le cycloalkyle, et l'hétérocycloalkyle étant éventuellement substitués par un ou plusieurs R₅, ou
R₁ et R₂, lorsqu'ils sont sur des atomes adjacents, conjointement avec les atomes auxquels ils sont fixés formant un cycle hétérocycloalkyle à 5 ou 6 chaînons ;
R₃ étant H ou D, ou R₃ étant absent lorsque -̅-̅-̅-̅-̅-̅ est une double liaison ;
chaque R₄ étant indépendamment choisi parmi -C(O)OR₆, - C(O)NR₆R_{6'}, -NR₆C(O)R_{6'}, halogène, -OH, -NH₂, CN, (C₆-C₁₀)aryle, hétéroaryle à 5 ou 6 chaînons comprenant 1 à 4 hétéroatomes choisis parmi O, N, et S, (C₃-C₈)cycloalkyle, et un cycle hétérocycloalkyle à 4 à 7 chaînons comprenant 1 à 3 hétéroatomes choisis parmi O, N, et S, les groupes aryle, hétéroaryle, cycloalkyle, et hétérocycloalkyle étant éventuellement substitués par un ou plusieurs R₇ ;
chaque R₃ étant indépendamment choisi parmi (C₁-C₆)alkyle, (C₂-C₆) alcényle, (C₂-C₆) alcynyle, (C₁-C₆)alcoxy, (C₁-C₆)halogénoalkyle, (C₁-C₆)halogénoalcoxy, (C₁-C₆)hydroxyalkyle, halogène, -OH, -NH₂, CN, (C₃-C₇)cycloalkyle, hétérocycloalkyle à 5 à 7 chaînons comprenant 1 à 3 hétéroatomes choisis parmi O, N, et S, (C₆-C₁₀)aryle, et hétéroaryle à 5 ou 6 chaînons comprenant 1 à 3 hétéroatomes choisis parmi O, N, et S, ou
deux R₅, lorsqu'ils sont sur des atomes adjacents, conjointement avec les atomes auxquels ils sont fixés formant un cycle (C₆-C₁₀)aryle ou un cycle hétéroaryle à 5 ou 6 chaînons comprenant 1 à 3 hétéroatomes choisis parmi O, N, et S, éventuellement substitué par un ou plusieurs R₁₀, ou
deux R₅, lorsqu'ils sont sur des atomes adjacents, conjointement avec les atomes auxquels ils sont fixés formant un cycle (C₅-C₇) cycloalkyle ou un cycle hétérocycloalkyle à 5 à 7 chaînons comprenant 1 à 3 hétéroatomes choisis parmi O, N, et S éventuellement substitué par un ou plusieurs R₁₀ ;
R₆ et R_{6'} étant chacun indépendamment H, (C₁-C₆)alkyle, ou (C₆-C₁₀)aryle ;
chaque R₇ étant indépendamment choisi parmi (C₁-C₆)alkyle, (C₂-C₆) alcényle, (C₂-C₆) alcynyle, (C₁-C₆)alcoxy, (C₁-C₆)halogénoalkyle, (C₁-C₆)halogénoalcoxy, -C(O)R₈, - (CH₂)₀₋₃C(O)OR₈, -C(O)NR₈R₉, -NR₈C(O)R₉, -NR₈C(O)OR₉, - S(O)ₚNR₈R₉, -S(O)ₚR₁₂, (C₁-C₆)hydroxyalkyle, halogène, -OH, -O(CH₂)₁₋₃CN, -NH₂, CN, -O(CH₂)₀₋₃(C₆-C₁₀)aryle, adamantyle, -O(CH₂)₀₋₃-hétéroaryle à 5 ou 6 chaînons comprenant 1 à 3 hétéroatomes choisis parmi O, N, et S, (C₆-C₁₀)aryle, hétéroaryle à 5 à 10 chaînons monocyclique ou bicyclique comprenant 1 à 3 hétéroatomes choisis parmi O, N, et S, (C₃-C₇)cycloalkyle, et hétérocycloalkyle à 5 à 7 chaînons comprenant 1 à 3 hétéroatomes choisis parmi O, N, et S, l'alkyle étant éventuellement substitué par un ou plusieurs R₁₁, et l'aryle, l'hétéroaryle, et l'hétérocycloalkyle étant éventuellement substitués par un ou plusieurs substituants chacun indépendamment choisis parmi halogène, (C₁-C₆)alkyle, (C₁-C₆)halogénoalkyle, et (C₁-C₆)alcoxy, ou
deux R₇ conjointement avec l'atome de carbone auquel ils sont fixés formant un =(O), ou
deux R₇, lorsqu'ils sont sur des atomes adjacents, conjointement avec les atomes auxquels ils sont fixés formant un cycle (C₆-C₁₀)aryle ou un cycle hétéroaryle à 5 ou 6 chaînons comprenant 1 à 3 hétéroatomes choisis parmi O, N, et S, éventuellement substitué par un ou plusieurs R₁₀, ou
deux R₇ conjointement avec les atomes auxquels ils sont fixés formant un cycle (C₅-C₇) cycloalkyle ou un cycle hétérocycloalkyle à 5 à 7 chaînons comprenant 1 à 3 hétéroatomes choisis parmi O, N, et S, éventuellement substitué par un ou plusieurs R₁₀ ;
R₈ et R₉ étant chacun indépendamment H ou (C₁-C₆)alkyle ; chaque R₁₀ étant indépendamment choisi parmi (C₁-C₆)alkyle, (C₁-C₆)alcoxy, (C₁-C₆)halogénoalkyle, (C₁-C₆)halogénoalcoxy, (C₁-C₆)hydroxyalkyle, halogène, -OH, - NH₂, et CN, ou
deux R₁₀ conjointement avec l'atome de carbone auquel ils sont fixés formant un =(O) ;
chaque R₁₁ étant indépendamment choisi parmi CN, (C₁-C₆)alcoxy, (C₆-C₁₀)aryle, et hétérocycloalkyle à 5 à 7 chaînons comprenant 1 à 3 hétéroatomes choisis parmi O, N, et S, l'aryle et l'hétérocycloalkyle étant éventuellement substitués par un ou plusieurs substituants chacun indépendamment choisis parmi (C₁-C₆)alkyle, (C₁-C₆)alcoxy, (C₁-C₆)halogénoalkyle, (C₁-C₆)halogénoalcoxy, (C₁-C₆)hydroxyalkyle, halogène, -OH, - NH₂, et CN ;
R₁₂ étant (C₁-C₆)alkyle, (C₁-C₆)halogénoalkyle, (C₆-C₁₀)aryle, ou hétérocycloalkyle à 5 à 7 chaînons comprenant 1 à 3 hétéroatomes choisis parmi O, N, et S ;
R₁₃ étant H, halogène, -OH, ou -NH₂ ;
R₁₄ étant H, (C₁-C₃)alkyle, (C₁-C₃)alcoxy, (C₁-C₃)halogénoalkyle, (C₁-C₃)halogénoalcoxy, (C₁-C₃)hydroxyalkyle, halogène, -OH, -NH₂, -NO₂, ou CN ;
R₁₅ étant halogène, -OH, ou -NH₂ ;
R₁₆ étant (C₁-C₃)alkyle, (C₁-C₃)alcoxy, (C₁-C₃)halogénoalkyle, (C₁-C₃)halogénoalcoxy, (C₁-C₃)hydroxyalkyle, halogène, -OH, -NH₂, -NO₂, ou CN ;
Rₓ étant H ou D ;
p étant 0, 1, ou 2 ;
n étant 0, 1, ou 2 ;
n1 étant 1 ou 2, n + n1 ≤ 3 ; et
q étant 0, 1, 2, 3, ou 4 ;
ou sel, hydrate, solvate, stéréoisomère ou forme tautomère pharmaceutiquement acceptable correspondant(e).

2. Composé selon la revendication 1, Rₓ étant H.

3. Composé selon la revendication 1 ou 2, X₂ étant N et X₃ étant CR₁₄ ou X₂ étant CR₁₃ et X₃ étant N ou X₂ étant CR₁₅, et X₃ étant CR₁₄ ou X₂ étant CR₁₃, et X₃ étant CR₁₆.

4. Composé selon la revendication 1, ayant une formule (Ia), une formule (Ib), une formule (Ic), ou une formule (Id) : 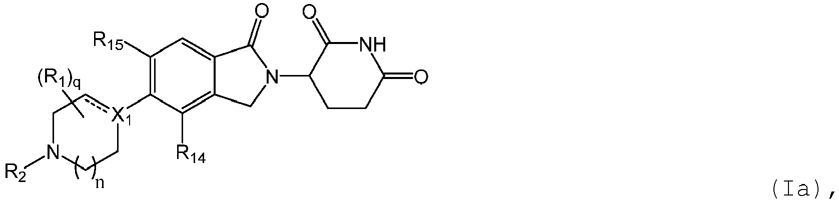 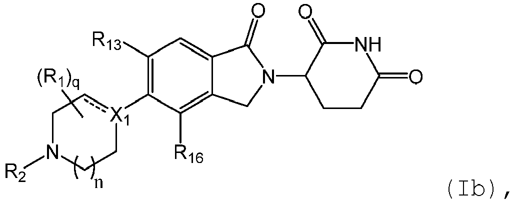 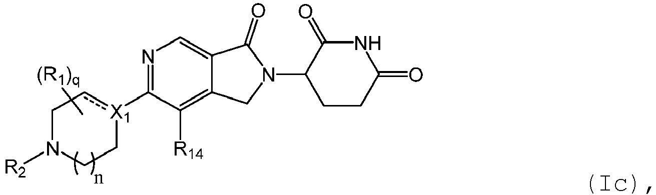 ou 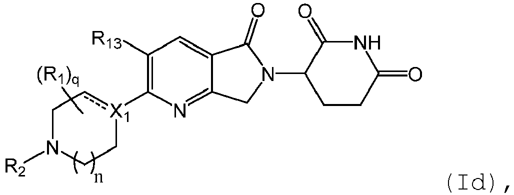 ou sel, hydrate, solvate, stéréoisomère ou forme tautomère pharmaceutiquement acceptable correspondant(e).

5. Composé selon l'une quelconque des revendications 1-4, -̅-̅-̅-̅-̅-̅ étant une double liaison, X₁ étant CR₃, et R₃ étant absent ou -̅-̅-̅-̅-̅-̅ étant une simple liaison, X₁ étant C R₃, et R₃ étant H.

6. Composé selon la revendication 1, ayant une formule (Ie), une formule (If), une formule (Ig), ou une formule (Ih) : 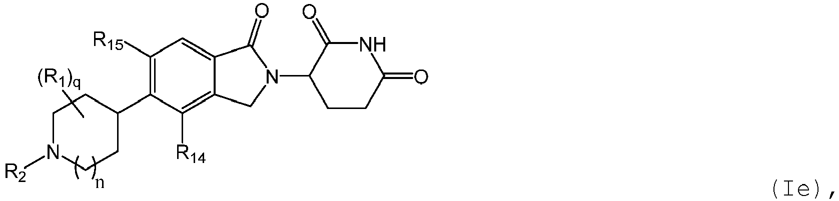 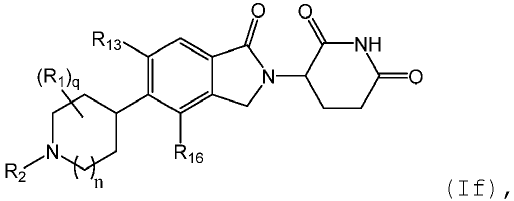 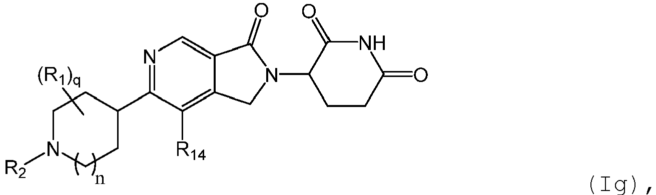 ou 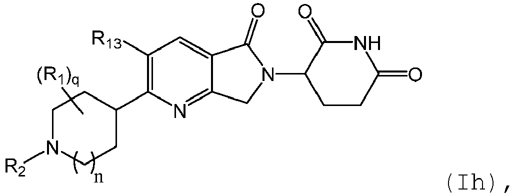 ou sel, hydrate, solvate, stéréoisomère ou forme tautomère pharmaceutiquement acceptable correspondant(e).

7. Composé selon l'une quelconque des revendications 1-6, n étant 0, 1, ou 2.

8. Composé selon la revendication 1, ayant une formule (Ii), une formule (Ij), une formule (Ik), ou une formule (Il) : 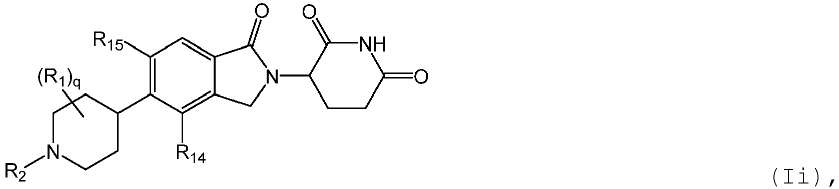 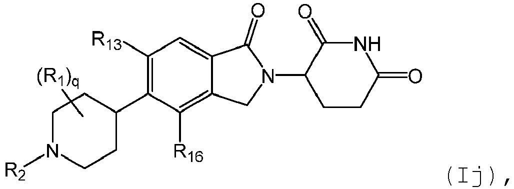 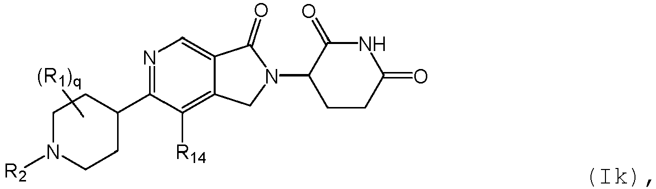 ou 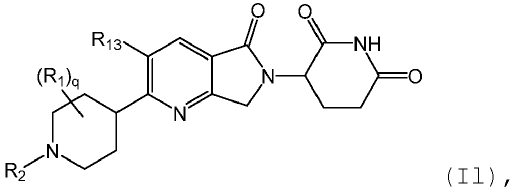 ou sel, hydrate, solvate, stéréoisomère ou forme tautomère pharmaceutiquement acceptable correspondant(e).

9. Composé selon l'une quelconque des revendications 1-8, R₂ étant (C₆-C₁₀)aryle, (C₃-C₈)cycloalkyle, ou hétérocycloalkyle à 5 à 7 chaînons comprenant 1 à 3 hétéroatomes choisis parmi O, N, et S, l'aryle, l'hétéroaryle, le cycloalkyle, et l'hétérocycloalkyle étant éventuellement substitués par un à trois R₅.

10. Composé selon l'une quelconque des revendications 1-8, R₂ étant (C₂-C₆)alkyle éventuellement substitué par un à trois R₄ ou R₂ étant (C₁-C₆)alkyle substitué par un à trois R₄.

11. Composé selon la revendication 1 choisi parmi :
3-(2-(1-benzylpipéridin-4-yl)-5-oxo-5,7-dihydro-6H-pyrrolo[3,4-b]pyridin-6-yl)pipéridine-2,6-dione 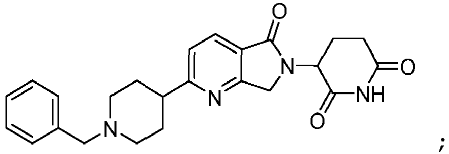
3-(6-(1-benzylpipéridin-4-yl)-3-oxo-1,3-dihydro-2H-pyrrolo[3,4-c]pyridin-2-yl)pipéridine-2,6-dione 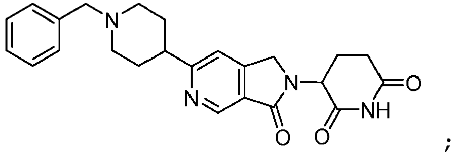
3-(5-(1-benzylpipéridin-4-yl)-4-fluoro-1-oxoisoindolin-2-yl)pipéridine-2,6-dione 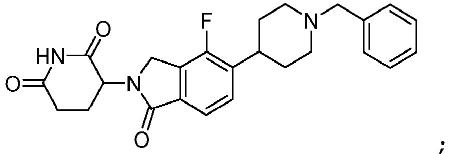
3-(5-(1-benzylpipéridin-4-yl)-6-fluoro-1-oxoisoindolin-2-yl)pipéridine-2,6-dione 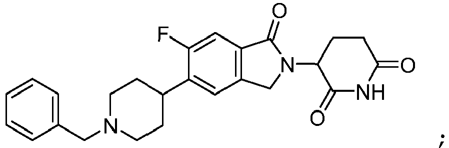
3-(5-(1-benzylpipéridin-4-yl)-4-méthyl-1-oxoisoindolin-2-yl)pipéridine-2,6-dione 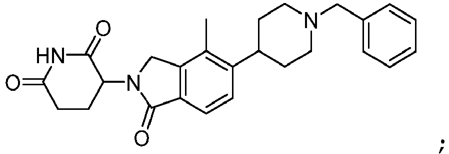
3-(4-amino-5-(1-benzylpipéridin-4-yl)-1-oxoisoindolin-2-yl)pipéridine-2,6-dione 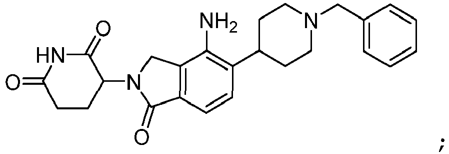
3-(6-amino-5-(1-benzylpipéridin-4-yl)-1-oxoisoindolin-2-yl)pipéridine-2,6-dione 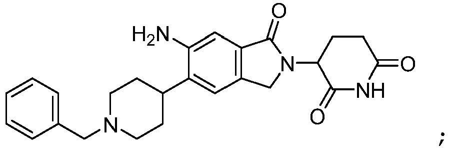
3-(5-(1-benzylpipéridin-4-yl)-4-chloro-1-oxoisoindolin-2-yl)pipéridine-2,6-dione 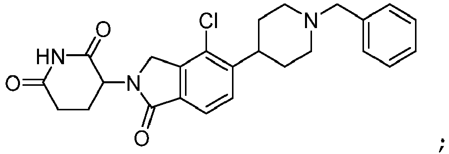
3-(5-(1-benzylpipéridin-4-yl)-6-chloro-1-oxoisoindolin-2-yl)pipéridine-2,6-dione 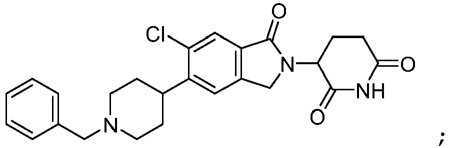
3-(5-(1-benzylpipéridin-4-yl)-4-hydroxy-1-oxoisoindolin-2-yl)pipéridine-2,6-dione 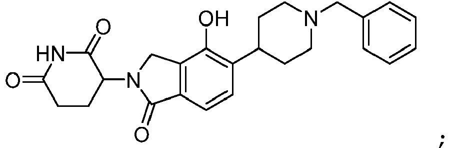
3-(5-(1-benzylpipéridin-4-yl)-6-hydroxy-1-oxoisoindolin-2-yl)pipéridine-2,6-dione 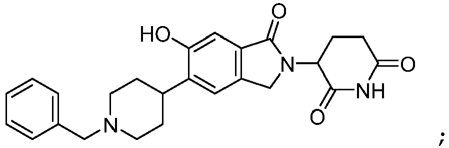
3-(5-(1-benzylpipéridin-4-yl)-4-méthoxy-1-oxoisoindolin-2-yl)pipéridine-2,6-dione 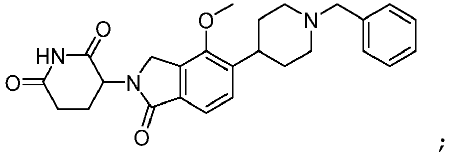
5-(1-benzylpipéridin-4-yl)-2-(2,6-dioxopipéridin-3-yl)-1-oxoisoindoline-4-carbonitrile 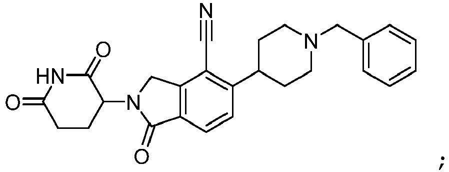
3-(5-(1-benzylpipéridin-4-yl)-1-oxo-4-(trifluorométhyl)isoindolin-2-yl)pipéridine-2,6-dione 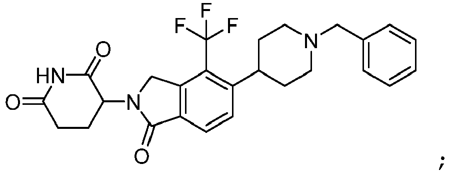
3-(5-(1-benzylpipéridin-4-yl)-4-nitro-1-oxoisoindolin-2-yl)pipéridine-2,6-dione 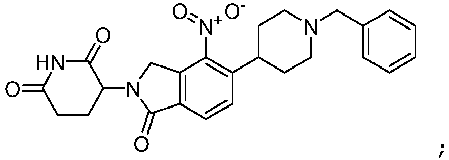
3-(6-fluoro-1-oxo-5-(1-(pyridin-4-ylméthyl)pipéridin-4-yl)isoindolin-2-yl)pipéridine-2,6-dione 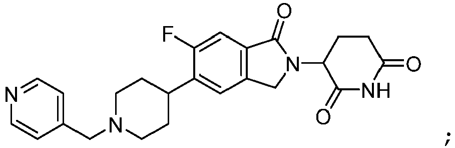
3-(4-chloro-5-(1-(((1r,4r)-4-méthoxycyclohexyl)méthyl)pipéridin-4-yl)-1-oxoisoindolin-2-yl)pipéridine-2,6-dione 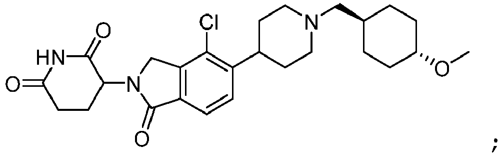
3-(4-fluoro-5-(1-(((1r,4r)-4-méthoxycyclohexyl)méthyl)pipéridin-4-yl)-1-oxoisoindolin-2-yl)pipéridine-2,6-dione 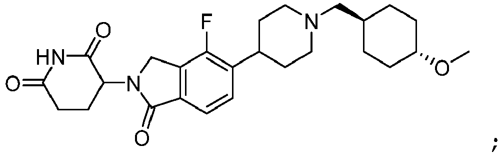
3-(4-hydroxy-5-(1-(((1r,4r)-4-méthoxycyclohexyl)méthyl)pipéridin-4-yl)-1-oxoisoindolin-2-yl)pipéridine-2,6-dione 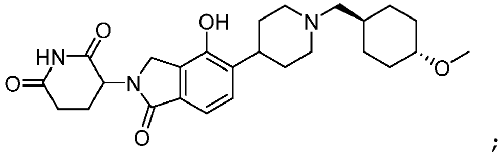 et
3-(5-(1-benzyl-1,2,3,6-tetrahydropyridin-4-yl)-4-méthoxy-1-oxoisoindolin-2-yl)pipéridine-2,6-dione 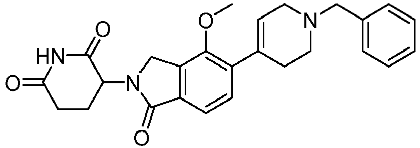 ou sel, hydrate, solvate, stéréoisomère ou forme tautomère pharmaceutiquement acceptable correspondant(e).

12. Composition pharmaceutique comprenant une quantité thérapeutiquement efficace d'un composé selon l'une quelconque des revendications 1 à 11, ou d'un sel, d'un hydrate, d'un solvate, d'un stéréoisomère ou d'une forme tautomère pharmaceutiquement acceptable correspondant(e), et un support ou excipient pharmaceutiquement acceptable, éventuellement comprenant en outre au moins un agent pharmaceutique supplémentaire.

13. Composé selon l'une quelconque des revendications 1 à 11, ou sel, hydrate, solvate, stéréoisomère ou forme tautomère pharmaceutiquement acceptable correspondant(e), pour une utilisation dans le traitement d'une maladie ou d'un trouble qui est affecté par la réduction de niveaux de protéine IKZF2.

14. Composé, ou sel, hydrate, solvate, stéréoisomère ou forme tautomère pharmaceutiquement acceptable correspondant(e), pour une utilisation selon la revendication 13, la maladie ou le trouble étant choisi(e) parmi un cancer du poumon non à petites cellules (NSCLC), un mélanome, un cancer du sein triple négatif (TNBC), un cancer du nasopharynx (NPC), un cancer colorectal stable microsatellite (mssCRC), un thymome, une leucémie myélogène aiguë, un carcinoïde, et une tumeur stromale gastro-intestinale (GIST).

15. Combinaison pharmaceutique comprenant un composé selon l'une quelconque des revendications 1 à 11, ou un sel, un hydrate, un solvate, un stéréoisomère ou une forme tautomère pharmaceutiquement acceptable correspondant(e), et un ou plusieurs agents thérapeutiques supplémentaires.
